# EUROPEAN PATENT APPLICATION

(11) **EP 4 488 290 A1**
(43) Date of publication of application: **08.01.2025**
(21) Application number: 23763762.4
(22) Date of filing: 03.03.2023
(51) Int. Cl.: C07K 16/00, C07K 7/08, A61K 47/68

(54) **METHOD FOR INCREASING PRODUCTION YIELD OF ANTIBODY-DRUG CONJUGATE BY USING THIOL-REACTIVE ADDITIVE**

(30) Priority: 04.03.2022 KR 20220028159
(71) Applicant: ABTIS Co., Ltd., Gyeonggi-do 16648 (KR); Research & Business Foundation Sungkyunkwan University, Gyeonggi-do 16419 (KR)
(72) Inventor: CHUNG, Sang Jeon, Suwon-si, Gyeonggi-do 16420 (KR); OH, Yeong Soo, Gwacheon-si Gyeonggi-do 13833 (KR); KIM, Juhwan, Suwon-si Gyeonggi-do 16489 (KR); LEE, Sunmin, Hwaseong-si Gyeonggi-do 18337 (KR); LEE, Taejin, Suwon-si Gyeonggi-do 16627 (KR); LEE, Younggeun, Icheon-si Gyeonggi-do 17309 (KR); SEO, Jinwoo, Suwon-si Gyeonggi-do 16408 (KR); LEE, Geonmin, Hwaseong-si Gyeonggi-do 18264 (KR); SON, Jinyoung, Suwon-si Gyeonggi-do 16329 (KR)
(74) Representative: Schön, Christoph
(86) International application number: PCT/KR2023/002986
(87) International publication number: WO 2023/167564

(57) **Abstract**

The present application relates to a method of increasing the production yield of an antibody comprising a functional group using a thiol-reactive additive.

## Description

The present application relates to a method of preparing an antibody including a functional group or an antibody-drug conjugate. More particularly, the present application relates to a method of increasing a production yield of an antibody comprising a functional group or an antibody-drug conjugate using a thiol-reactive additive.

### [Background Art]

Antibodies are biomolecules that have the function of recognizing specific molecules and are used for various industrial purposes. For example, antibodies can be used to detect or search for (screen) a specific material, confirm a path through which a specific material moves in the body or cells, and induce an immune response to a specific material, and therefore can be used for a therapeutic use.

There have been attempts to improve antibodies to expand their functions. Typically, attempts have been made to label foreign materials to complement or expand the function of antibodies. For example, antibodies may be labeled with a fluorescence material to be used in a fluorescence assay, or antibodies may be labeled with an agent for treating a specific condition to maximize the therapeutic efficacy of the antibodies. These attempts and technologies are collectively referred to as antibody labeling in the present application. The present application relates to a novel method of labeling antibodies.

Initially, antibody labeling was accomplished by randomly attaching foreign materials to antibodies, but this method had many problems. The antibodies prepared thereby had a problem of reduced uniformity. These antibodies have different numbers of materials attached thereto at different binding sites, and therefore exhibit non-uniform effects. These problems are major obstacles to the development of antibody-drug conjugate (ADC) technology that requires high safety and reproducibility.

In addition, there is a problem of significantly degrading the effect of recognizing an antibody. An antibody is composed of an Fab domain with an antigen-binding domain, which recognizes an antigen, and an Fc domain involved in crystallization of an antibody. Random labeling greatly reduced the antibody recognition effect by binding a foreign material to an antibody-binding domain of an antibody or a site adjacent thereto.

As a result, there was a demand for a technology of uniformly labeling antibodies in a site-specific manner in the corresponding technical field. Although some technologies were developed, most of them lacked technical and economic effects, such as genetically manipulating or modifying antibodies.

Afterward, the technology of site-specifically conjugating an antibody with a functional material such as a drug without the genetic modification of the antibody was developed. That is, the technology of site-specifically attaching a drug to an antibody through a chemical reaction without additional manipulation of the antibody was developed.

Particularly, the documents [PCT International Application No. PCT/KR2020/003282, Publication No. WO2020/184944] disclose a method of transferring a drug or a functional group such as a bio-orthogonal functional group site-specifically to an antibody using a compound comprising a thioester group and an Fc-binding peptide.

### [Disclosure]

### [Technical Problem]

As described above, a method of site-specifically transferring a drug or a functional group such as a bio-orthogonal functional group to an antibody by using a compound comprising a thioester group and an Fc-binding peptide has been developed. However, according to the present application, it is found that this method has the problem of low yield of antibodies comprising a functional group, which is a desired product, when the compound comprising a thioester group and an Fc-binding peptide is used.

Therefore, the present application is directed to providing a method of increasing a yield of an antibody comprising a functional group, which is a desired product, using a thiol-reactive additive upon the preparation of an antibody comprising a functional group using a compound comprising a thioester group and an Fc-binding peptide.

### [Technical Solution]

One aspect of the present application provides a method of preparing an antibody that includes a functional group.

Another aspect of the present application provides a method of increasing a production yield of an antibody that includes a functional group using a thiol-reactive additive.

Still another aspect of the present application provides a method of increasing a yield of an antibody that includes a functional group, prepared by a reaction of the antibody and a compound for transferring the functional group, using a thiol-reactive additive, the method including:
(a) contacting an antibody with a compound for transferring a functional group in a reaction composition,
   wherein the compound for transferring a functional group includes a thioester group, a functional group (FG), and an Fc-binding peptide (FcBP),
   wherein the thioester group of the compound for transferring a functional group reacts with a nucleophile of the antibody,
   as a result of the reaction between the thioester group and the nucleophile of the antibody,
   an antibody comprising the functional group, and a by-product comprising a thiol group and an Fc-binding peptide derived from the compound for transferring the functional group are produced, wherein the thiol group of the by-product is produced by the reaction of the thioester group of the compound for transferring the functional group with the nucleophile of the antibody,
   wherein the by-product has a reaction inhibitory effect of inhibiting a reaction of an antibody, which has not yet reacted, with a compound for transferring a functional group, which has not yet reacted, in the reaction composition; and
(b) contacting the by-product with the thiol-reactive additive in the reaction composition to remove the reaction inhibitory effect of the by-product,
   wherein the thiol-reactive additive has reactivity with a thiol group,
   wherein the thiol-reactive additive reacts with the thiol group of the by-product,
   as a result of the reaction between the thiol-reactive additive and the thiol group, the thiol group of the by-product is capped.

In a specific embodiment, the reaction inhibitory effect of the by-product may be removed by capping the thiol group of the by-product.

In a specific embodiment, the thiol-reactive additive may be a compound having little or no reactivity with the antibody.

In a specific embodiment, the thiol-reactive additive may be a small molecule compound.

In a specific embodiment, the molecular weight of the thiol-reactive additive may be 50 g/mol or more and 1000 g/mol or less.

In a specific embodiment, the thiol-reactive additive may be a compound that undergoes a thiol-disulfide exchange reaction.

In a specific embodiment, the thiol-reactive additive may include a disulfide group.

In a specific embodiment, the thiol-reactive additive may be diphenyl disulfide or a derivative thereof.

In a specific embodiment, the thiol-reactive additive may be diphenyl disulfide, dipyridyldisulfide, 5,5'-dithiobis-(2-nitrobenzoic acid) (DTNB), 2,2'-dithiodibenzoic acid, 4-aminophenyl disulfide, 2-aminophenyl disulfide, bis(4-chlorophenyl disulfide), bis(2-nitrophenyl disulfide), p-tolyl disulfide, bis(4-nitrophenyl) disulfide, bis(2-nitrophenyl) disulfide, bis(4-methoxyphenyl) disulfide, or bis(2-methoxyphenyl) disulfide, or derivatives thereof.

In a specific embodiment, the thiol-reactive additive may be 5,5'-dithiobis-(2-nitrobenzoic acid) (DTNB) or 2,2'-dipyridyldisulfide.

In a specific embodiment, the thiol-reactive additive may be a compound having the structure of Formula 6 or a derivative thereof:

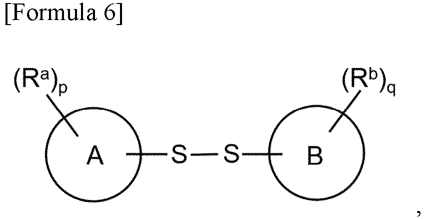

Here,
A is a 5- or 6-membered aromatic ring, wherein A optionally includes one or more heteroatoms in the ring, and the heteroatoms are each independently selected from N, O, and S,
p is an integer of 0 to 4,
each R^{a} is independently selected from -R, -NO₂, -CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂,
B is a 5- or 6-membered aromatic ring, wherein B optionally includes one or more heteroatoms in the ring, and the heteroatoms are each independently selected from N, O, and S,
q is an integer of 0 to 4,
each R^{b} is independently selected from -R, -NO₂, -CR₃, -NR₂, -OR, -SR, -SO₂R, - OSO₂R, -PO₂R₂, -OPOR₂, -C(=O)R, -C(=O)CR₃, -C(=O)OR, and -C(=O)NR₂, and
each R is independently selected from H, halogen, C₁₋₆ alkyl, C₃₋₁₀ cycloalkyl, C₃₋₁₀ heterocycloalkyl, aryl, heteroaryl, -OH, and -NH₂.

Here, in a specific embodiment, at least one of R^{a} may be an electron withdrawing group or a hydrophilic group.

Here, in a specific embodiment, at least one of R^{b} may be an electron withdrawing group or a hydrophilic group.

In a specific embodiment, the thiol-reactive additive may be maleimide or a derivative thereof.

In a specific embodiment, the thiol-reactive additive may be maleimide, N-methylmaleimide, N-ethylmaleimide, N-phenylmaleimide, N-benzylmaleimide, 3-maleimidopropionic acid, N-tert-butylmaleimide, N-cyclohexylmaleimide, or derivatives thereof.

In a specific embodiment, the thiol-reactive additive may be tert-butylmaleimide.

In a specific embodiment, the thiol-reactive additive may be a compound having the structure of Formula 9 or a derivative thereof: 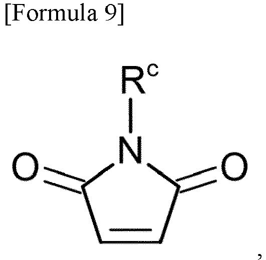 wherein R^{c} is selected from -R, =O, =S, -NO₂, -CR₃, -CH₂CR₃, -NR₂, -OR, -SR, - C(=O)R, -C(=O)CR₃, -C(=O)OR, and -C(=O)NR₂, in which each R is independently selected from H, halogen, C₁₋₆ alkyl, C₃₋₁₀ cycloalkyl, C₃₋₁₀ heterocycloalkyl, aryl, heteroaryl, -OH, - NH₂, and -SH.

In a specific embodiment, the thiol-reactive additive may be a compound having any one of the structures of Formulas 10 to 31.

In a specific embodiment, the compound for transferring the functional group may have the structure of Formula 1:

[Formula 1] FU-RU-PU,

wherein
FU is a functional unit including a functional group (FG),
RU is a reactive unit including a thioester group, and
PU is a peptide unit (PU) including an Fc-binding peptide (FcBP).

In a specific embodiment, the Fc-binding peptide may have a length of 8 to 20 amino acid residues.

In a specific embodiment, the Fc-binding peptide may include any one amino acid residue selected from SEQ ID NOs: 2 to 80, or an amino acid residue having at least 80% residue identity thereto.

In a specific embodiment, the functional group may include one or more bio-orthogonal functional groups.

Here, in a specific embodiment, the bio-orthogonal functional group may each independently be a group that participates in any one bio-orthogonal reaction selected from Staudinger ligation, copper-catalyzed azide-alkyne cycloaddition (CuAAC), copper-free azide-alkyne cycloaddition, tetrazine ligation, tetrazole ligation, oxime ligation, and isocyanide click reaction.

Here, in a specific embodiment, the bio-orthogonal functional group is independently any one selected from azide, terminal alkyne, cycloalkyne, tetrazine, norbornene, cycloalkene, tetrazole, oxime, and isocyanide groups.

In a specific embodiment, the functional group may include one or more active moieties.

Here, in a specific embodiment, each active moiety may independently be any one selected from a drug, an imaging moiety, a ligand for a radioactive isotope, an affinity material, a stabilization material, a vitamin, and a hydrophilic moiety.

In a specific embodiment, the reaction inhibitory effect of the by-product may be exhibited because the Fc-binding peptide included in the by-product guides the by-product to a reaction site on the antibody, where the reaction between the compound for transferring the functional group and the antibody occurs, and the thiol group included in the by-product interacts with the antibody at the reaction site.

In a specific embodiment, the yield of the antibody comprising a functional group in the method may be 1.5 times or more compared to that when a thiol-reactive additive is not used.

In a specific embodiment, the yield of the antibody comprising a functional group may be measured based on an antibody comprising two functional groups.

In a specific embodiment, in the above-described method, (a) the contacting of the antibody with the compound for transferring a functional group, and (b) the contacting of the by-product with the thiol-reactive additive may be performed by mixing the thiol-reactive additive, the antibody, and the compound for transferring the functional group.

Here, in a specific embodiment, in the mixing of the thiol-reactive additive, the antibody, and the compound for transferring the functional group, the thiol-reactive additive is first mixed with the antibody, and then mixed with the compound for transferring a functional group.

Here, in a specific embodiment, in the mixing of the thiol-reactive additive, the antibody, and the compound for transferring the functional group, the thiol reactive additive may be first mixed with the compound for transferring a functional group, and then mixed with the antibody.

Yet another embodiment of the present application provides a kit that is used to increase a production yield of an antibody comprising a functional group, which includes a thiol-reactive additive, an antibody, and a compound for transferring a functional group.

Yet another embodiment of the present application provides a composition that is used to increase a production yield of an antibody, which includes a thiol-reactive additive, an antibody, and a compound for transferring a functional group.

### [Advantageous Effects]

According to a method of some embodiments of the present application, a production yield of an antibody comprising a functional group may increase.

### [Description of Drawings]

FIG. 1 shows the sequence (GPSVFLFPPKPKDTLMI; SEQ ID NO: 1) of an Fc region comprising the K246 and K248 sites in IgG or trastuzumab and numbers in the amino acid sequence according to the EU numbering system.
FIG. 2 illustrates an example of the reaction of an antibody and a compound for transferring a functional group.
FIG. 3 is an example illustrating the reaction inhibition mechanism of a by-product.
FIG. 4 is another example illustrating the reaction inhibition mechanism of a by-product.
FIG. 5 illustrates a FAR 1 antibody comprising a functional group and a FAR 2 antibody comprising a functional group.
FIG. 6 illustrates a FAR 3 antibody comprising a functional group and a FAR 4 antibody comprising a functional group.
FIG. 7 illustrates the structure of synthesized FcBPs (L6Dap FcBP, L6Dab FcBP, L6Orn FcBP, and L6Lys FcBP).
FIG. 8 illustrates the structure of Compound 7 (FcBP-N₃).
FIG. 9 illustrates the mass spectrometry data of Compound 7 (FcBP-N₃).
FIG. 10 illustrates the structure of Compound 17 (FcBP-C6PEG4).
FIG. 11 illustrates the mass spectrometry data of Compound 17 (FcBP-C6PEG4).
FIG. 12 illustrates the structure of Compound 27 (FcBP-C6PEG3).
FIG. 13 illustrates the mass spectrometry data of Compound 27 (FcBP-C6PEG3).
FIG. 14 is a schematic diagram of an antibody comprising a functional group comprising an azide, prepared using Compound 7 (FcBP-N₃).
FIG. 15 is the analysis results of production yields of an antibody and an antibody-drug conjugate, which include a functional group, according to the presence or absence of a thiol-reactive additive in the process of preparing an antibody and an antibody-drug conjugate, including a functional group, using Compound 7 (FcBP-N₃).
FIG. 16 is a schematic diagram of an antibody comprising a functional group comprising an azide, prepared using Compound 17 (FcBP-C6PEG4).
FIG. 17 is the analysis results of production yields of an antibody and an antibody-drug conjugate, which include a functional group, according to the presence or absence of a thiol-reactive additive in the process of preparing an antibody and an antibody-drug conjugate, including a functional group, using Compound 17 (FcBP-C6PEG4).
FIG. 18 is a schematic diagram of an antibody comprising a functional group comprising an azide, prepared using Compound 27 (FcBP-C6PEG3).
FIG. 19 is the analysis results of production yields of an antibody and an antibody-drug conjugate, which include a functional group, according to the presence or absence of a thiol-reactive additive in the process of preparing an antibody and an antibody-drug conjugate, including a functional group, using Compound 27 (FcBP-C6PEG3).
FIG. 20 illustrates the structure of FcBP(L6Dap)-norbomene.
FIG. 21 illustrates the HPLC chromatogram data of FcBP(L6Dap)-norbomene.
FIG. 22 illustrates the structure of FcBP(L6Dab)-norbomene.
FIG. 23 illustrates the HPLC chromatogram data of FcBP(L6Dab)-norbomene.
FIG. 24 illustrates the structure of FcBP(L6Orn)-norbornene.
FIG. 25 illustrates the HPLC chromatogram data of FcBP(L6Orn)-norbornene.
FIG. 26 illustrates the structure of FcBP(L6Lys)-norbornene.
FIG. 27 illustrates the HPLC chromatogram data of FcBP(L6Lys)-norbornene.
FIG. 28 is the analysis result of the production yield of an antibody comprising a functional group according to the presence or absence of a thiol-reactive additive in the process of preparing an antibody and an antibody-drug conjugate, including a functional group, using FcBP-norbomene.
FIG. 29 illustrates graphs of the analysis results of the production yields of an antibody comprising a functional group according to the presence or absence of a thiol-reactive additive in the process of preparing an antibody and an antibody-drug conjugate, including a functional group, using FcBP-norbornene.
FIG. 30 illustrates the LC-MS analysis result of trastuzumab.
FIG. 31 illustrates the LC-MS analysis result of a trastuzumab/2-norbornene (T/2-N) conjugate.
FIG. 32 illustrates the HIC-HPLC monitoring result analyzed at each incubation time.
FIGS. 33 to 39 are the analysis results of the production yields of antibody-drug conjugates according to the use of thiol-reactive additives (thiol-reactive additives 1 to 12) in the process of preparing an antibody-drug conjugate using FcBP-N₃ (Compound 7). FIG. 33 is the HIC-HPLC analysis result for a control (the case that does not use a thiol-reactive additive).
FIGS. 40 to 46 are the analysis results of the production yields of antibody-drug conjugates according to the use of thiol-reactive additives (thiol-reactive additives 1 to 12) in the process of preparing an antibody-drug conjugate using FcBP-C6PEG4 (Compound 17).
FIGS. 47 to 54 are the results of analyzing the reactivity of a maleimide-based compound with an antibody.

### [Modes of the Invention]

### Definitions of terms

Unless defined otherwise, all technological and scientific terms used in the present application have meanings commonly understood by those skilled in the art of the present application.

"Halogen" or "halo" refers to the group including fluorine, chlorine, bromine, and iodine, included in the halogen group of elements in the periodic table.

The term "hetero" used herein refers to a compound or group that includes one or more hetero atoms. That is, the term "hetero" may be used together with a term used to refer to the molecule itself or a part of the molecule. For example, heteroalkylene refers to an alkylene group comprising one or more hetero atoms on the main chain. In another example, heteroaryl refers to an aryl group including one or more hetero atoms on a ring (e.g., a group substituted with a hetero atom in which one or more carbons on the ring of C₆ aryl are each independently selected). The term "hetero atom" refers to an atom other than carbon or hydrogen, which includes, for example, B, Si, N, P, O, S, F, Cl, Br, I, and Se. Preferably, the hetero atom includes a multivalent atom such as N, O, and S. For example, when one structure contains one or more hetero atoms, each of the hetero atoms may be independently selected from N, O, and S.

The term "alkyl" or "alkane" used to refer to the molecule itself or a part thereof is used to mean a fully saturated group of chain or branched hydrocarbons. Examples of chain and branched alkyl groups may include methyl, ethyl, n-propyl, iso-propyl, n-butyl, sec-butyl, tert-butyl, iso-butyl, pentyl, hexyl, heptyl, octyl, nonyl, and decyl. Alkyl groups may include cyclic structures. The term "C_{x-y}" is intended to include, for example, a residue having from x to y carbons on a chain or ring when used together with alkyl. For example, the term "C_{x-y} alkyl" may be a substituted or unsubstituted alkyl group that has a chain shape, a branched shape, or a cyclic structure, which includes from x to y carbons, and further includes a haloalkyl group such as difluoromethyl or 2,2,2-trifluoroethyl. C₀ alkyl refers to hydrogen. Examples of C₁₋₄ alkyl include methyl, ethyl, n-propyl, iso-propyl, n-butyl, sec-butyl, tert-butyl, iso-butyl, difluoromethyl, and 2,2,2-trifluoroethyl, but the present application is not limited thereto.

The term "heteroalkyl" used herein means alkyl having one or more hetero atoms. Here, each of the hetero atoms is independently selected.

The term "alkylene" used to refer to the molecule itself or a part thereof means a divalent radical derived from alkyl. The term "alkylene" may be used together with the term "substituted" or "unsubstituted" if needed. When the term "alkylene" is not used together with the term "substituted" or "unsubstituted," the term "alkylene" is intended to include both a substituted alkylene and an unsubstituted alkylene. Examples of alkylenes may include -CH₂-, -CH₂CH₂-, -CH₂CH₂CH₂-, and -CH₂CH₂CH₂CH₂-, but the present application is not limited thereto. For example, an alkylene may be used together with C₂ alkylene to mean an alkylene group having two carbon atoms on the main chain. For example, "C_{x-y} alkylene" is used herein to mean an alkylene that includes all of substituted or unsubstituted alkylenes which have from X to Y carbon atoms on the main chain. The term "alkylene" used herein may be used to encompass an alkylene group having a hetero atom and a hetero alkylene group having one or more hetero atoms on the main chain.

The term "heteroalkylene" used to refer to the molecule itself or a part thereof refers to a divalent radical derived from heteroalkyl. The term "heteroalkylene" may be used together with the term "substituted" or "unsubstituted" if needed. When the term "heteroalkylene" is not used together with the term "substituted" or "unsubstituted," the term "heteroalkylene" is intended to include all of substituted heteroalkylenes or unsubstituted heteroalkylenes. For example, heteroalkylene groups include -CH₂-CH₂-O-CH₂-CH₂-, and -CH₂-O-CH₂-CH₂-NH-CH₂-, but the present application is not limited thereto. A heteroalkylene group may include one or more hetero atoms, wherein the hetero atoms may be the same as or different from each other. For example, a heteroalkylene group may include one or more hetero atoms at sites other than the terminals of a chain or branch, wherein the hetero atoms may be the same as or different from each other. For example, a heteroalkylene group may include one or more hetero atoms at each or all terminals of a chain or branch, wherein the hetero atoms may be the same as or different from each other. For example, the term "C_{x-y} heteroalkylene" is used to include all of substituted or unsubstituted heteroalkylenes having a total of x to y carbon atoms and hetero atoms on the main chain. In one example, C₃ heteroalkylene may be used to mean substituted or unsubstituted heteroalkylene having two carbon atoms and one hetero atom on the main chain. In another example, C₅ heteroalkylene may be used to mean substituted or unsubstituted heteroalkylene having three carbon atoms and two hetero atoms on the main chain.

The term "cycloalkyl" is used to refer to a fully saturated cyclic hydrocarbon group. A "cycloalkyl" includes monocyclic and polycyclic groups. Unless defined otherwise, generally, a monocyclic cycloalkyl group has 3 to about 10 carbon atoms on the ring. Rings other than the first ring of a polycyclic cycloalkyl may be selected from saturated, unsaturated, and aromatic rings. A cycloalkyl is a bicyclic molecule in which 1, 2, 3 or more atoms are shared between two rings. The term "fused cycloalkyl" is polycyclic cycloalkyl, referring to a group in which each ring shares two adjacent atoms with another ring. The rings other than the first ring of a fused polycyclic cycloalkyl may be selected from saturated, unsaturated, and aromatic rings. A cycloalkyl may be used together with the term "substituted" or "unsubstituted," and substituted cycloalkyl refers to a group provided when one or more hydrogen atoms linked to a carbon atom on the ring are substituted with one or more independent substituents. Further, a cycloalkyl may be used with hetero, wherein the heterocycloalkyl refers to a cycloalkyl group having one or more hetero atoms on the ring. The term "cycloalkyl" may be used to encompass all of substituted or unsubstituted cycloalkyls, and substituted or unsubstituted heterocycloalkyls.

The term "cycloalkylene" is used to mean a divalent radical derived from cycloalkyl. For example, the term "cycloalkylene" may be used with the term "substituted" or "unsubstituted." For example, the term "cycloalkylene" may be used with the term "hetero." The term "cycloalkylene" may be used to encompass all of substituted or unsubstituted cycloalkylenes, and substituted or unsubstituted heterocycloalkylenes.

The term "alkene" or "alkenyl" used to refer to the molecule itself or a part thereof encompasses chain or branched non-aromatic hydrocarbons, including one or more double bonds. For example, a chain or branched alkenyl group may have 2 to about 5, 2 to 20, or 2 to 10 carbon atoms.

The term "heteroalkene" or "heteroalkenyl" refers to alkenyl having one or more hetero atoms. Here, each hetero atom is independently selected.

The term "alkenylene" used to refer to the molecule itself or a part thereof refers to a divalent radical derived from alkenyl. The term "alkenylene" may be used together with the term "substituted" or "unsubstituted." When the term "alkenylene" is not used with the term "substituted" or "unsubstituted," the term "alkenylene" is intended to encompass all of substituted alkenylene and unsubstituted alkenylene. Alkenylene may be, for example, -C=C-, -C-C-C=C-C=C-, or -C-C-C-C=C-, but the present application is not limited thereto. When "C_{x-y} alkenylene" is used in the specification, C_{x-y} alkenylene is used to refer to alkenylene encompassing all of substituted or unsubstituted alkenylene having from x to y carbon atoms on the main chain. The term "alkenylene" used herein may be used to encompass an alkenylene group not including a hetero atom and a hetero alkenylene group including one or more hetero atoms on the main chain.

The term "heteroalkenylene" used to refer to the molecule itself or a part thereof refers to a divalent radical derived from heteroalkenyl. For example, the term "heteroalkenylene" may be used to refer to an alkenylene group including one or more hetero atoms on the main chain. When "C_{x-y} heteroalkenylene" is used in the specification, C_{x-y} heteroalkenylene is used to refer to heteroalkenylene encompassing all of substituted or unsubstituted heteroalkenylene having from x to y carbon atoms and hetero atoms (e.g., the sum of the number of carbon atoms and the number of hetero atoms is from x to y) on the main chain.

The term "cycloalkene" or "cycloalkenyl" is a cyclic hydrocarbon including one or more double bonds on a ring. "Cycloalkenyl" includes monocyclic and polycyclic groups. Unless defined otherwise, generally, monocyclic cycloalkenyl has 3 to about 10 carbon atoms on the ring. The rings other than the first ring of polycyclic cycloalkenyl may be selected from saturated, unsaturated, and aromatic rings. Cycloalkenyl is a bicyclic molecule, in which 1, 2, 3 or more atoms are shared between two rings. The term "fused cycloalkenyl" is polycyclic cycloalkenyl, in which each ring shares two adjacent atoms with another ring. The rings other than the first ring of the fused polycyclic cycloalkenyl may be selected from saturated, unsaturated, and aromatic rings. The term "cycloalkenyl" may be used together with "substituted" or "unsubstituted," and substituted cycloalkenyl refers to a group provided when one or more hydrogen atoms linked to a carbon atom on the ring are substituted with one or more independent substituents. Further, the term "cycloalkenyl" may be used with the term "hetero," and here, heterocycloalkenyl refers to a cycloalkenyl group including one or more hetero atoms on the ring. The term "cycloalkenyl" may be used to encompass all of substituted or unsubstituted cycloalkenyl, and substituted or unsubstituted heterocycloalkenyl.

The term "cycloalkenylene" is used to mean a divalent radical derived from cycloalkenyl. For example, the term "cycloalkenylene" may be used with the term "substituted" or "unsubstituted." For example, the term "cycloalkenylene" may be used with the term "hetero." The term "cycloalkenylene" may be used to encompass all of substituted or unsubstituted cycloalkenylene, and substituted or unsubstituted heterocycloalkenylene.

The term "alkyne" or "alkynyl" used to refer to the molecule itself or a part thereof is a chain or branched non-aromatic hydrocarbon, and includes one or more triple bonds. For example, a chain or branched alkyl group may have 2 to about 50, 2 to 20, or 2 to 10 carbon atoms.

The term "heteroalkynyl" or "heteroalkyne" refers to alkynyl having one or more hetero atoms. Here, each hetero atom is independently selected.

The term "alkynylene" used to refer to the molecule itself or a part thereof refers to a divalent radical derived from alkynyl. The term "alkynylene" may be used with the term "substituted" or "unsubstituted" if needed. When the term "alkynylene" is not used with the term "substituted" or "unsubstituted," the term "alkynylene" is intended to encompass all of substituted alkynylene and unsubstituted alkynylene. When "C_{x-y} alkynylene" is used in the specification, the C_{x-y} alkynylene is used to refer to alkynylene including all of substituted or unsubstituted alkynylene, having from x to y carbon atoms on the main chain. The term "alkynylene" used herein may be used to encompass an alkynylene group not including a hetero atom and a heteroalkynylene group including one or more hetero atoms on the main chain.

The term "heteroalkynylene" used to refer to the molecule itself or a part thereof refers to a divalent radical derived from heteroalkynyl. For example, the term "heteroalkynylene" may be used to refer to an alkynylene group including one or more hetero atoms on the main chain. When "C_{x-y} heteroalkynylene" is used herein, the C_{x-y} heteroalkynylene is used to mean heteroalkynylene encompassing all of substituted or unsubstituted heteroalkynylene, having from x to y carbon atoms and hetero atoms (e.g., the sum of the number of carbon atoms and the number of hetero atoms is from x to y) on the main chain.

The term "cycloalkyne" or "cycloalkynyl" is a cyclic hydrocarbon including one or more triple bonds on the ring, and is also called "strained alkyne." "Cycloalkynyl" includes monocyclic and polycyclic groups. Unless defined otherwise, generally, monocyclic cycloalkynyl has 2 to about 10 carbon atoms on the ring. The rings other than the first ring of polycyclic cycloalkynyl may be selected from saturated, unsaturated, and aromatic rings. Cycloalkynyl is a bicyclic molecule in which 1, 2, 3 or more atoms are shared between two rings. The term "fused cycloalkynyl" is polycyclic cycloalkynyl in which each ring shares two adjacent atoms with another ring. The rings other than the first ring of the fused polycyclic cycloalkynyl may be selected from saturated, unsaturated, and aromatic rings. The term "cycloalkynyl" may be used with the term "substituted" or "unsubstituted," and substituted cycloalkynyl refers to a group provided when one or more hydrogen atoms linked to a carbon atom on the ring are substituted with one or more independent substituents. Further, the term "cycloalkynyl" may be used with the term "hetero," and here, heterocycloalkynyl refers to a cycloalkynyl group including one or more hetero atoms on the ring. The term "cycloalkynyl" may be used to encompass all of substituted or unsubstituted cycloalkynyl, and substituted or unsubstituted heterocycloalkynyl.

The term "cycloalkynylene" is used to mean a divalent radical derived from cycloalkynyl. For example, the term "cycloalkynylene" may be used with the term "substituted" or "unsubstituted." For example, the term "cycloalkynylene" may be used with the term "hetero." The term "cycloalkynylene" may be used to encompass all of substituted or unsubstituted cycloalkynylene, and substituted or unsubstituted heterocycloalkynylene.

The term "aryl" is used to refer to a group having an aromatic ring, and a group derived from an aromatic compound arene. The term "aryl" includes a monocyclic group and a polycyclic group. The term "aryl" may be used with the term "hetero," and heteroaryl is used to refer to an aryl group having one or more hetero atoms on the ring. The term "aryl" may be used with the term "substituted" or "unsubstituted," and substituted aryl is used to refer to an aryl group when one or more hydrogen atoms linked to an atom on the ring are substituted with one or more substituents. The term "aryl" may be used to encompass all of substituted or unsubstituted aryl, and substituted or unsubstituted heteroaryl. Examples of aryls include phenyl, pyridyl, naphthyl, and biphenyl, but the present application is not limited thereto.

The term "arylene" is used to mean a divalent radical derived from aryl. For example, the term "arylene" may be used with the term "substituted" or "unsubstituted." For example, the term "arylene" may be used with the term "hetero." The term "arylene" may be used to encompass all of substituted or unsubstituted arylene, and substituted or unsubstituted heteroarylene.

The term "substituted" used herein means that, in a compound which has a normal atomic valence and is substituted, one or more hydrogen atoms on an atom are substituted with substituents including deuterium and hydrogen variants. When a substituent is oxygen (i.e., =O), it means that two hydrogen atoms were substituted. When one substituent is halogen (e.g., Cl, F, Br, or I), it means that one hydrogen atom was substituted with halogen. When there are two or more substituents in one group, the substituents present in the group may be the same or different. Unless defined otherwise, the type and number of substituents may be optional as long as they can be chemically achieved. For example, substituents may be selected from -R, =O, =S, -NO₂, -CR₃, -NR₂, -OR, -SR, -C(=O)R, -C(=O)CR₃, -C(=O)OR, and -C(=O)NR₂, wherein each R is independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH, but the present application is not limited thereto (i.e., a substituent is not -H). Representative examples of substituents include -C₁₋₄ alkyl, -C(=O)H, -C(=O)CH₃, -C(=O)OH, -C(=O)NH₂, -NH₂, =O, =S, -OH, -NO₂, and -SH, but the present application is not limited thereto. The term "substituted" or "unsubstituted" may be used with the term used to refer to the molecule itself or the term used to refer to a part of the molecule. For example, substituted C₁₀₋₂₀ alkylene may refer that one or more hydrogen atoms linked to the main chain are substituted with substituents, wherein each substituent may be independently selected.

The structure 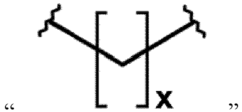 used within structural formulas or chemical formulas disclosed herein is used to mean Cₓ alkylene or Cₓ heteroalkylene, Cₓ alkenylene or Cₓ heteroalkenylene, or Cₓ alkynylene or Cₓ heteroalkynylene. Preferably, the structure 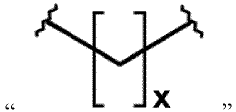 is used to mean Cₓ alkylene or Cₓ heteroalkylene. For example, the structure 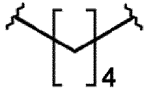 may be used to mean structures encompassing all of -C₄ alkylenes such as CH₂-CH₂-CH₂-CH₂-, and C₄ heteroalkylenes such as -CH₂-CH₂-O-CH₂- and -O-CH₂-CH₂-CH₂-. Here, when x is 0, it means a bond. That is, the structure 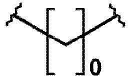 may be expressed as the structure

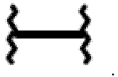

A compound used herein may have a specific geometric or stereoisomeric conformation. Unless specified otherwise, when a compound is disclosed in the present application, isomers of the compound, such as cis and trans isomers, (-)- and (+)-enantiomers, (R)- and (S)-enantiomers, a diastereoisomer, a (D)-isomer, an (L)-isomer, and a racemate, are included in the scope of the present application. That is, when there is no indication (e.g., *, 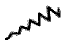 , 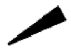 or 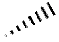 ) related to isomers in a chemical formula or structure disclosed herein, the disclosed chemical formula or structure is meant to include all possible isomers.

The term "amino acid" used herein may be used to refer to both an amino acid that is not bound to another amino acid, and an amino acid residue that is bound to a different amino acid included in a protein or peptide, and it may be appropriately interpreted depending on the content or context of the paragraph in which the term is used. The term "amino acid" used herein may be used with a meaning including both a natural amino acid and an unnatural amino acid. The natural amino acids used herein refer to a total of 20 amino acids synthesized through gene transcription and translation processes in the human body. Specifically, the natural amino acids include alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, 1), leucine (Leu, L), lysine (Lys K), methionine (Met, M), phenylalanine (Phe, F), proline (Pro, P), serine (Ser, S), threonine (Thr, T), tryptophan (Trp, W), tyrosine (Tyr, Y), and valine (Val, V). The unnatural amino acids used herein refer to amino acids that can be synthesized through a process other than gene transcription and translation processes in a human body, or synthesized from organisms other than a human. The unnatural amino acids may include, for example, ornithine (orn), diaminopropionic acid (Dap), diaminobytyric acid (Dab), and naphthylalanine. As described above, the term "amino acid" used herein may be used to refer to both amino acids that are not linked to other amino acids and amino acid residues that are bound to other amino acids contained in proteins or peptides. For example, alanine may be used to refer to alanine and/or an alanine residue. For example, arginine may be used to refer to arginine and/or an arginine residue. The term "amino acid" used herein may be used to include both of an L-type amino acid and a D-type amino acid. In some embodiments, if there is no description of L-type or D-type, it may be interpreted as an L-type amino acid.

The term "amino acid residue" used herein means a structure derived from an amino acid included in a compound, peptide, and/or protein (e.g., an antibody), which is covalently linked to a different part of the compound, peptide, and/or protein. For example, when alanine, arginine, and glutamic acid are linked by an amide bond to form a peptide having an ARE sequence, the peptide includes three amino acid residues, wherein A may represent an alanine residue, R may represent an arginine residue, and E may represent a glutamic acid residue. Further, as described above, in the peptide having an ARE sequence, the peptide may include three amino acids, wherein A may represent alanine, R may represent arginine, and E may represent glutamic acid. In another example, when aspartic acid, phenylalanine, and lysine are linked through an amide bond to form a peptide having a DFK sequence, the peptide includes three amino acid residues, wherein D may represent an aspartic acid residue, F may represent a phenylalanine residue, and K may represent a lysine residue. Further, as described above, in the peptide having a DFK sequence, the peptide may include three amino acids, wherein D may represent aspartic acid, F may represent phenylalanine, and K may represent lysine.

Unless otherwise stated, in the specification, an amino acid sequence is described in the direction from the N-terminus to the C-terminus using a single-letter or three-letter notation of amino acids. For example, in the case of RNVP, it represents a peptide in which arginine, asparagine, valine, and proline are sequentially linked in the direction of the N-terminus to the C-terminus. In still another example, in the case of Thr-Leu-Lys, it represents a peptide in which threonine, leucine, and lysine are sequentially linked to the direction of the N-terminus to the C-terminus. An amino acid that cannot be represented by the single-letter or three-letter notation is represented using different letters, and may be additionally explained. A sequence suggested in the specification may include a sequence having at least 90%, 91%, 92%, 93%, 94%, 95%, 96%, 97%, 98%, or 99% sequence identity with the suggested sequence as long as the desired functions of these sequences are the same or similar.

The term "click chemistry" used herein is the chemical concept that is introduced to explain complementary chemical functional groups and chemical reactions designed for two molecules to rapidly and stably form a covalent bond by K. Barry Sharpless of the Scripps Research Institute. The click chemistry of the specification does not mean a specific reaction, but rather the concept of a fast and stable reaction. In one embodiment, to form bonds between molecules using click chemistry, several conditions must be satisfied. These conditions are a high yield, excellent selectivity for reaction sites, modular operation to organically combine molecules, and rapid and accurate production of a product in a thermodynamically stable direction. The click chemistry of the specification includes the reaction of a pair with a reactivity to each other among click chemistry functional groups (e.g., terminal alkyne, azide, strained alkyne, diene, dienophile, trans-cyclooctene, alkene, thiol, tetrazine, triazine, dibenzocyclooctyne (DBCO) and bicyclononyne (bicyclo[6.1.0]non-4-yne)). Examples of the click-chemical reactions include the Huisgen 1,3-dipolar cycloaddition (refer to Tomoe et al. Journal of Organic Chemistry (2002) 67: 3075-3064); the Diels-Alder reaction; the inverse-demand Diels-Alder reaction; the nucleophilic addition to a small strained ring such as an epoxide and an aziridine; the nucleophilic addition to an activated carbonyl group; and the Staudinger ligation and the addition to a carbon-carbon double bond or triple bond.

The term "bio-orthogonal functional group" is used to refer to a biologically inactive chemical reaction group. That is, the term "bio-orthogonal functional group" is used to refer to a chemical functional group that participates in bio-orthogonal chemistry or a bio-orthogonal reaction to perform. The term "bio-orthogonal functional group" may refer to a bio-orthogonal chemical functional group or bio-orthogonal chemical group. A bio-orthogonal functional group refers to a group that does not react with an endogenous molecule or functional group in living cells or an organism. These bio-orthogonal functional groups are designed to react with specific groups in a complex biological system without interfering with a normal cellular process. The term "bio-orthogonal chemistry" was first suggested by Carolyn R. Bertozzi in 2003, and bio-orthogonal chemistry is still widely used in the fields of organic chemistry and conjugation. The term "bio-orthogonal" refers that a chemical reaction performed using a bio-orthogonal functional group that may be performed without interfering with a natural biological process. One of the main characteristics of a bio-orthogonal functional group is to react specifically and efficiently with the corresponding reactive group in a biological environment. Bio-orthogonal functional groups, bio-orthogonal chemistry, and bio-orthogonal reactions are described in detail in the documents [Sletten, Ellen M., and Carolyn R. Bertozzi, "Bioorthogonal chemistry: fishing for selectivity in a sea of functionality," Angewandte Chemie International Edition 48.38 (2009): 6974-6998; Mbua, Ngalle Eric, et al., "Strain-promoted alkyne-azide cycloadditions (SPAAC) reveal new features of glycoconjugate biosynthesis," ChemBioChem 12.12 (2011): 1912-1921; Bird, Robert E., et al., "Bioorthogonal chemistry and its applications," Bioconjugate Chemistry 32.12 (2021): 2457-2479; Devaraj, Neal K., "The future of bioorthogonal chemistry," ACS central science 4.8 (2018): 952-959; and Scinto, Samuel L., et al., "Bioorthogonal chemistry," Nature Reviews Methods Primers 1.1 (2021): 30], and the entire contents of these documents are incorporated in the specification by reference. Bio-orthogonal chemistry may considerably overlap with the broader field of click chemistry. As described above, strictly, click chemistry referring to a high-yield and modular reaction also includes a reaction that is not bio-orthogonal. In a specific embodiment, a bio-orthogonal functional group is not reactive with an antibody, but can be used to refer to a chemical group that has high reactivity with a group capable of a bio-orthogonal reaction with a bio-orthogonal functional group. Representative types of bio-orthogonal reactions (or bio-orthogonal chemistry) include Staudinger ligation, copper-free azide-alkyne cycloaddition, such as copper-catalyzed azide-alkyne cycloaddition (CuAAC) or strain-promoted azide-alkyne cycloaddition (SPAAC), tetrazine ligation, tetrazole ligation, oxime ligation, and isocyanide click reaction, but the present application is not limited thereto. Examples of bio-orthogonal functional groups include an azide, terminal alkyne, cyclic alkyne (e.g., cyclooctin), tetrazine, norbornene, cycloalkene (e.g., cyclooctene), tetrazol, oxime, or isocyanide group, but the present application is not limited thereto. For example, in the copper-free azide-alkyne cycloaddition (strain-promoted azide-alkyne cycloaddition; SPAAC), an azide group and a cyclooctin group are subjected to a bio-orthogonal reaction. A cyclooctin group participating in SPAAC may be monocyclic cyclooctin or polycyclic cyclooctin including fused polycyclic. Specific examples of cyclooctin groups participating in SPAAC include bicyclononyne (BCN), dibenzocyclooctyne (DBCO), aza-dibenzocyclooctyne (DIBAC), dibenzocyclooctynol (DIBO), difluorinated cyclooctyne (DIFO), biarylazacyclooctynone (BARAC), dimethoxyazacyclooctyne (DIMAC), and difluorobenzocyclooctyne (DIFBO), but the present application is not limited thereto.

The term "antibody" used herein is used to mean an immunoglobulin molecule or a fragment thereof. An immunoglobulin is generally well known, and has the ability to specifically bind to one or more antigens. The term "antibody" used herein is used to also include a fragment thereof, so it does not matter if it does not have the ability to bind to a specific antigen as in the case of the Fc fragment. Unless used for specific limitation relating to an antibody, it will be interpreted without particular limitation that the term "antibody" includes all of a monospecific antibody, a bispecific antibody, a trispecific antibody, a monoclonal antibody, a human antibody, a humanized antibody, a recombinant antibody, and a chimeric antibody. For example, an antibody may include two heavy chains and two light chains, and here, may have a structure in which the two heavy chains are connected with one or more bridges (e.g., a disulfide bond), one heavy chain and one light chain are connected with one or more bridges, and another heavy chain and another light chain are connected with one or more bridges. An antibody may be divided into an Fc region (or Fc domain) and an Fab region, the Fab region includes a site that can bind to an antigen, and the Fc region includes a constant region of a heavy chain. The term "antibody" used herein may be used to encompass both a conjugated antibody and an unconjugated antibody (e.g., free antibody).

The term "functional group" used herein is used to refer to a group having one or more functions, but the present application is not limited thereto. Ultimately, the field of antibody conjugates, such as an antibody-drug conjugate, aims to link a functional material designed to introduce a specific function to an antibody to the antibody. Functional groups containing a functional material may be divided into, for example, a group having a reactive group designed to react with another chemical functional group and a group having an active moiety. For example, a functional group may include a reaction group designed to react with one or more different chemical reaction groups. Here, a reaction group designed to react with another chemical reaction group may be, for example, a bio-orthogonal functional group. That is, a functional group may include one or more bio-orthogonal functional groups, wherein each bio-orthogonal functional group may be independently selected. In another example, a functional group may have one or more active moieties. Examples of active moieties may include drugs (e.g., toxins), imaging moieties (e.g., a fluorescent moiety, and a luminescent moiety containing a luminescent material such as luciferin), radioactive moieties, proteins with a specific function, peptides with a specific function, affinity materials (e.g., biotin, streptavidin, and aptamer), stabilizing materials, vitamins, DNA molecules, and polyethylene glycol (PEG) moieties, but the present application is not limited thereto. Here, one or more active moieties may each be independently selected. In the field for transferring a functional group to an antibody, it is obvious to those of ordinary skill in the related art that, after preparing an antibody comprising a bio-orthogonal functional group, an active moiety may be ultimately linked to the antibody through a bio-orthogonal reaction in which the bio-orthogonal functional group participates.

The term "radioactive moiety" used herein refers to a moiety having a ligand to a radioactive isotope designed to bind thereto and/or a moiety having a radioactive isotope. Radiolabelling is useful for diagnostic imaging, radioimmunotherapy (RIT), and radiotherapy. A radioactive moiety may be, for example, ¹⁸F, ¹¹C, ⁶⁷Cu, ⁹⁰Y, ¹²⁵I, ¹²³I, ¹²⁴I, ¹³¹I, ¹⁷⁷Lu, ¹⁸⁶Re, ¹⁸⁸Re, ²¹¹At, ²¹³Bi, ²²⁵Ac, or ^{99m}Tc, but the present application is not limited thereto.

The term "fluorescent moiety" used herein refers to a moiety containing a dye, protein, or dye reagent to be used for fluorescence. Dyes and molecules that can be used as dye reagents are well known in the art. A fluorescent moiety may include, for example, a green fluorescent protein (GFP), Cy3, Cy5, Texas Red, FITC, Rhodamine, or DAPI, but the present application is not limited thereto.

The term "drug" used herein is used to refer to a molecule having therapeutic efficacy for a certain disease or a part of the molecule. Drugs according to the present application include those known to have efficacy against a certain disease to those of ordinary skill in the art. Further, the term "drug" used herein may be used to include a conjugated drug and an unconjugated drug (e.g., free drug). Drugs may be, for example, one or more selected from auristatin, eribulin, tubulysin, geldanamycin (Kerr et al., 1997, Bioconjugate Chem. 8(6):781-784), maytansinoid (EP 1391213, ACR 2008, 41, 98-107), calicheamicin (U.S. Patent Publication No. 2009/0105461, Cancer Res. 1993, 53, 3336-3342), mertansine, daunomycin, doxorubicin, methotrexate, vindesine, SG2285 (Cancer Res. 2010, 70(17), 6849-6858), dolastatin, dolastatin analogs auristatin (U.S. Patent No. 5,635,483), cryptophycin, camptothecin, rhizoxin derivative, CC 1065 analog or a derivative thereof, duocarmycin, enediyne antibiotic, esperamicin, epothilone, a pyrrolobenzodiazepine (PBD) derivative, α-amanitin, toxoid, a toll-like receptor 5 (TLR5) agonist, a toll-like receptor 7 (TLR7) agonist, a toll-like receptor 8 (TLR8) agonist, 1,4,7-triazacyclononane-1,4,7-triacetic acid (NOTA), and 1,4,7,10-tetraazacyclododecane-1,4,7,10-tetraacetic acid (DOTA), or derivatives thereof, but the present application is not limited thereto.

The term "electron withdrawing group" is used to refer to a group or substituent that has the ability to withdraw electrons from another atom or group (e.g., an adjacent group) in a molecule, and interpreted to have a meaning generally understood by those of ordinary skill in the related art. Representative examples of electron withdrawing groups include halogen, a carbonyl group (e.g., -CHO), a sulfonyl group (e.g., -SO₃H), an ester group (e.g., -COOH), an amide group (e.g., -CONH₂), -CN, -NO₂, and a haloalkyl group (e.g., -CF₃), but the present application is not limited thereto.

The term "hydrophilic group" used in the present application is used to refer to a group increasing the hydrophilicity of an added molecule or adding hydrophilicity, and is interpreted to have a meaning generally understood by those of ordinary skill in the related art. Representative examples of hydrophilic groups include -OH, -CHO, -COOH, -CONH₂, -NH₂, -PO₄, -SO₃H, a PEG moiety, and a sugar moiety, but the present application is not limited thereto.

In the present application, if there is a mention of the amino acid sequence of the Fc domain (or Fc region) of an antibody, unless mentioned otherwise, a sequence number follows the EU numbering system. The EU numbering system has been widely used as the sequence system for the Fc domain since the sequence of IgG was studied by Edelman GM, et al. (The covalent structure of an entire gammaG immunoglobulin molecule, Proc Natl Acad Sci U S A., 1969 May;63(1):78-85.).

The term "connected" or "connection" used herein means that two or more factors present in one conceptualizable structure are connected directly or indirectly (e.g., by a different factor such as a linker), and is not intended to mean that an additional factor cannot be present between the two or more factors. For example, the description such as "factor B connected to factor A" is intended to refer to both the case in which one or more factors are included between factor A and factor B (i.e., when factor A is connected to factor B by one or more different factors), and the case in which one or more factors are not present between factor A and factor B (i.e., when factor A and factor B are directly connected), and is not interpreted as limiting. The term "sequence identity" used herein is a term used to be associated with the degree of similarity between two or more sequences. For example, the term "sequence identity" is used together with the term referring to a reference sequence and the term representing a ratio (e.g., percentage). For example, the term "sequence identity" may be used to explain the sequence similar to or substantially the same as the reference amino acid sequence. When described as a "sequence having at least 90% sequence identity with sequence A," the reference sequence here is sequence A. For example, the percentage of the sequence identity may be calculated by aligning a reference sequence and a sequence that becomes a target for the measurement of the percentage of sequence identity. A method of calculating and/or determining the percentage of sequence identity is not otherwise limited, and it may be calculated and/or determined by a reasonable method or algorithm that can be used by those of ordinary skill in the art.

### Conventional method of transferring functional group site-specifically to antibody

### Requirement for method of transferring functional group site-specifically to antibody

An antibody is a biomolecule that has the function of recognizing a specific molecule and is used for various industrial purposes. In one example, an antibody may be used to detect or search for (screen) a specific material, to confirm a path of transferring the specific material into the body or cells, and to induce an immune response to the specific material for treatment.

There have been attempts to improve antibodies to expand their functions. Representatively, attempts have been made to label foreign substances to complement or expand the function of antibodies. Typically, a fluorescent substance may be labeled with an antibody and used in a fluorescence assay, or an agent for treating a specific condition may be labeled with an antibody to maximize the therapeutic efficacy of the antibody. These attempts and technologies are collectively referred to as antibody labeling in the present application. The present application concerns a novel antibody labeling method.

Initially, antibody labeling was performed by randomly attaching a foreign material to an antibody, but this method had many problems. Antibodies produced in this method have the problem of reducing homogeneity. These antibodies have a problem of a heterogeneity effect because the number of attached materials is different and their binding sites are different. This problem is a major obstacle to the development of antibody-drug conjugate (ADC) technology that requires high safety and reproducibility.

In addition, there is a problem that the recognition effect of an antibody is significantly reduced. An antibody consists of an Fab domain (or Fab region) comprising an antigen-binding domain that recognizes an antigen and an Fc domain (or Fc region) involved in the crystallization of an antibody. Random labeling significantly reduced the recognition effect of an antibody by binding of a foreign material to the antigen-binding domain of the antibody or a site adjacent thereto.

As a result, there was a demand in the relevant technical field for technology to uniformly label an antibody in a site-specific manner. Although some technologies had been developed, most of them lacked technical and economic effects, such as genetically manipulating or modifying antibodies.

Later, technology was developed to site-specifically conjugate a functional material such as a drug without genetic modification of the antibody. That is, technology has been developed to site-specifically attach a drug to an antibody through a chemical reaction without additional manipulation of the antibody. Imparting such site-specificity is achieved by the use of a site-specific antibody interactome (e.g., Fc-binding peptide) and the use of a compound including the same. Hereinafter, before explaining the site-specific antibody interactome, examples of the structure of an antibody and a labeling site, which is the location where a functional material is connected, will be described.

### Structure of antibody

This description is directed to providing an explanation of the structure, academic system, and physiological action of an antibody provided to aid understanding of the present application, and the antibody subject to the scope of the present application is not limited by this description.

As was known previously, an antibody consists of two heavy chains and two light chains. Functionally, an antibody is simply divided into a fragment antigen-binding variable region (Fab region) comprising light chains and a fragment crystallizable region (Fc region) consisting of a part of a heavy chain. Fab has a paratope that binds to an antigen, and as was known previously, it is a region that allows an antibody to have specific binding activity to an antigen. The Fc region or Fc domain is a ligand against an Fc receptor (FcR) of a cell, and plays an important role in inducing an immune response. In addition, the Fc domain also plays an important role in increasing the half-life of an antibody by binding to a neonatal Fc receptor and allowing the antibody to be repeatedly internalized into cells.

From this, several desirable directions of antibody labeling may be derived. (1) First, labeling is preferably done in a location away from a paratope of an antibody. If labeling is done at or adjacent to the paratope, the binding ability of the antibody to an antigen may be greatly reduced. (2) Secondly, labeling is preferably done in a location away from the recognition site of FcR, including FcRn. When labeling is done at or adjacent to the recognition site of the receptor, the immune inducing function of the antibody may be reduced or the half-life of the antibody may be greatly reduced. Information on the binding activation motif of the Fc domain can be confirmed in DeLano, W. L. (2000). Convergent Solutions to Binding at a Protein-Protein Interface. Science, 287(5456), 1279-1283., and W. Lance Martin et al. (2001), Molecular Cell, Vol. 7, 867-877, April, 2001.

When there is a mention of the amino acid sequence for the Fc domain of an antibody in the present application, unless mentioned otherwise, a sequence number follows the EU numbering system. The EU numbering system has been widely used as the sequence system for the Fc domain since the sequence of IgG was studied by Edelman GM, et al. (The covalent structure of an entire gammaG immunoglobulin molecule, Proc Natl Acad Sci U S A., 1969 May;63(1):78-85.).

### Exemplary labeling site

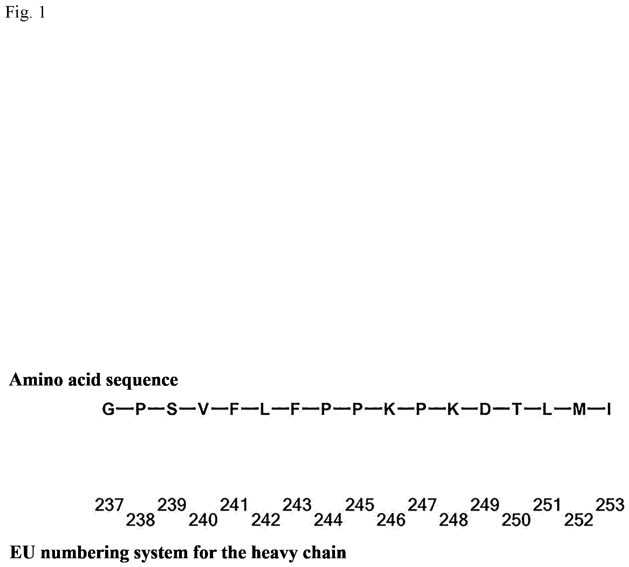

The labeling site (e.g., target site) of an antibody may be designed in consideration of the criteria for the labeling site (or target site), which are (1) it should be away from a paratope, and (2) it should be away from the recognition site of FcR, including FcRn. Among amino acids, those used in a bioconjugate reaction include lysine, cysteine, and tyrosine. Lysine 246 (Lys₂₄₆, or K246) and lysine 248 (Lys₂₄₈, or K248) present in the Fc domain of an antibody are residues that satisfy all conditions, that is, preferable labeling sites. K246 lysine and K248 lysine present in the Fc domain of the antibody are observed in an antibody comprising a human Fc domain. For example, human immunoglobulin G (IgG), trastuzumab, rituximab, bevacizumab, infliximab, adalimumab, and omalizumab include K246 and K248. For example, denosumab includes K247 and K249, which correspond to K246 and K248 of IgG1, respectively. In another example, dupilumab includes K251 and K253, which correspond to K246 and K248 of IgG1, respectively (refer to the documents [European Patent Application No. 19818561.3, Publication No. EP 3811978; and European Patent Application No. 18791007.0, Publication No. EP 3617235]). In IgG or trastuzumab, the sequence of the Fc region comprising the Lys₂₄₆ and Lys₂₄₈ sites is GPSVFLFPPKPKDTLMI (SEQ ID NO: 1), and the sequences and sequence numbers designated by the EU numbering system are shown in FIG. 1.

For example, the labeling site(s) may be one or more of an exposed lysine residue, an exposed tyrosine residue, an exposed serine residue, and an exposed threonine residue. Examples of the labeling sites that can be selected from human IgG1 are as follows (refer to the documents [European Patent Application No. 19818561.3, Publication No. EP 3811978; and European Patent Application No. 18791007.0, Publication No. EP 3617235]).
- Exposed lysine residues:
   CH2 domains (position 246, position 248, position 274, position 288, position 290, position 317, position 320, position 322, and position 338)
   CH3 domains (position 360, position 414, and position 439)
- Exposed tyrosine residues:
   CH2 domains (position 278, position 296, and position 300)
   CH3 domain (position 436)
- Exposed serine residues:
   CH2 domains (position 254, position 267, position 298, and position 324)
   CH3 domains (position 375, position 400, position 415, position 440, and position 442)
- Exposed threonine residues:
   CH2 domains (position 256, position 289, and position 307)
   CH3 domains (position 335, position 359, position 393, and position 437)

### Site-specific antibody interactome (SSAI)

The introduction of a functional material to a specific site (e.g., the above-described labeling site) of an antibody was achieved through technology using a site-specific antibody interactome. The technology of introducing functional materials (e.g., a bio-orthogonal functional group and a drug) at specific sites of an antibody using a site-specific antibody interactome and a compound including the same is described in detail in the documents [European Patent Application No. 19818561.3, Publication No. EP 3811978; PCT International Application No. PCT/KR2020/003282, Publication No. WO2020/184944; Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759; and Zeng, Yue, et al., "A Traceless Site-Specific Conjugation on Native Antibodies Enables Efficient One-Step Payload Assembly," Angewandte Chemie International Edition 61.36 (2022): e202204132], and the entire contents of each document are incorporated herein by reference.

The term "interactome" used in the present application refers to a protein or peptide involved in protein-protein interaction (PPI) between the protein or peptide. For example, a chaperone protein and its passenger protein are interactomes for each other. "Protein-protein interaction" refers to physical contact with high specificity between two or more protein or peptide molecules due to interaction. At this time, causative interactions include an electromagnetic force, a hydrogen bond, and a hydrophobic interaction, but the present application is not limited thereto.

The term "antibody interactome" used in the present application refers to an interactome for an antibody, including an immunoglobulin. The site-specific delivery of a functional group to an antibody may be achieved using an antibody interactome and a compound including the same. Among antibody interactomes, a peptide known to have binding activity to the Fc domain of an antibody is referred to as an Fc interactome or Fc-binding peptide (FcBP). The above-mentioned four documents ([European Patent Application No. 19818561.3, Publication No. EP 3811978; PCT International Application No. PCT/KR2020/003282, Publication No. WO2020/184944; Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759; and Zeng, Yue, et al., "A Traceless Site-Specific Conjugation on Native Antibodies Enables Efficient One-Step Payload Assembly," Angewandte Chemie International Edition 61.36 (2022): e202204132]) are the documents on a method of site-specifically transferring a molecule to be labeled to an antibody using FcBP and a compound including the same. FcBP may include, for example, any one sequence selected from the following amino acid sequences, or an amino acid sequence having at least 80% sequence identity therewith, but the present application is not limited thereto:
RGNCAYHKGQIIWCTYH (SEQ ID NO: 2); RGNCAYHKGQIVWCTYH (SEQ ID NO: 3); RGNCAYHKGQVVWCTYH (SEQ ID NO: 4); RGNCAYHKGQAVWCTYH (SEQ ID NO: 5); RGNCAYHKGQLLWCTYH (SEQ ID NO: 6); RGNCAYHKGQLIWCTYH (SEQ ID NO: 7); DCAYHKGQIVWCT (SEQ ID NO: 8); DCAYHKGQVVWCT (SEQ ID NO: 9); DCAYHKGQAVWCT (SEQ ID NO: 10); RGNCAYHKSQIIWCTYH (SEQ ID NO: 11); RGNCAYHKNQIIWCTYH (SEQ ID NO: 12); RGNCAYHKDQIIWCTYH (SEQ ID NO: 13); RGNCAYHKQQIIWCTYH (SEQ ID NO: 14); RGNCAYHKEQIIWCTYH (SEQ ID NO: 15); RGNCAYHKFQIIWCTYH (SEQ ID NO: 16); RGNCAYHKYQIIWCTYH (SEQ ID NO: 17); RGNCAYHKWQIIWCTYH (SEQ ID NO: 18); RGNCAYHKHQIIWCTYH (SEQ ID NO: 19); RGNCAYHKTQIIWCTYH (SEQ ID NO: 20); RGNCAYHKLQIIWCTYH (SEQ ID NO: 21); CAYHKLQIVWC (SEQ ID NO: 22); CAYHKLQLIWC (SEQ ID NO: 23); CAYHKSQIVWC (SEQ ID NO: 24); RGNCAYHKGQLVFCTYH (SEQ ID NO: 25); RGNCAYHKGQQVWCTYH (SEQ ID NO: 26); RGNCAYHKGQEVWCTYH (SEQ ID NO: 27); CAYHKGQLVWC (SEQ ID NO: 28); RGNCAYHKAQLVWCTYH (SEQ ID NO: 29); RGNCAYHKVQLVWCTYH (SEQ ID NO: 30); RGNCAYHKLQLVWCTYH (SEQ ID NO: 31); RGNCAYHKIQLVWCTYH (SEQ ID NO: 32); RGNCAYHKSQLVWCTYH (SEQ ID NO: 33); RGNCAYHKTQLVWCTYH (SEQ ID NO: 34); RGNCAYHKNQLVWCTYH (SEQ ID NO: 35); RGNCAYHKDQLVWCTYH (SEQ ID NO: 36); RGNCAYHKQQLVWCTYH (SEQ ID NO: 37); RGNCAYHKEQLVWCTYH (SEQ ID NO: 38); RGNCAYHKFQLVWCTYH (SEQ ID NO: 39); RGNCAYHKRQLVWCTYH (SEQ ID NO: 40); RGNCAYHKHQLVWCTYH (SEQ ID NO: 41); RGNCAYHKWQLVWCTYH (SEQ ID NO: 42); RGNCAYHKYQLVWCTYH (SEQ ID NO: 43); RGNCAYFKGQLVWCTYH (SEQ ID NO: 44); RGNCAYYKGQLVWCTYH (SEQ ID NO: 45); RGNCAYWKGQLVWCTYH (SEQ ID NO: 46); RGNCAYRKGQLVWCTYH (SEQ ID NO: 47); RGNCAYGKGQLVWCTYH (SEQ ID NO: 48); DCAYHKGQLVWC (SEQ ID NO: 49); NCAYHKGQLVWC (SEQ ID NO: 50); CAYHKGQLVWCT (SEQ ID NO: 51); CAYHKSQLVWC (SEQ ID NO: 52); GNCAYHKGQIIWCTYH (SEQ ID NO: 53); RGNCAYHEGQIIWCTYH (SEQ ID NO: 54); GPDCAYHRGELVWCTFH (SEQ ID NO: 55) (refer to the document [European Patent Application No. 19818561.3, Publication No. EP 3811978]); DCAWHDapGELVWCT (SEQ ID NO: 56) (Dap: diaminopropionic acid); DCAWHDabGELVWCT (SEQ ID NO: 57) (Dab: diaminobutyric acid); DCA WHOrnGEL VWCT (SEQ ID NO: 58) (Orn: ornithine); DCAWHKGELVWCT (SEQ ID NO: 59); DCAWHLGELVWCT (SEQ ID NO: 60) (refer to the documents [PCT International Application No. PCT/KR2020/003282, Publication No. WO2020/184944; and Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759]); RGNCAYHRGKLVWCTYH (SEQ ID NO: 61) (refer to the document [Zeng, Yue, et al., "A Traceless Site-Specific Conjugation on Native Antibodies Enables Efficient One-Step Payload Assembly," Angewandte Chemie International Edition 61.36 (2022): e202204132]); RGNCAYHKGQLVWCTYH (SEQ ID NO: 62); GPDCAYHDapGELVWCTFH (SEQ ID NO: 63); GPDCAYHDabGELVWCTFH (SEQ ID NO: 64); GPDCAYHOrnGELVWCTFH (SEQ ID NO: 65); GPDCAYHKGELVWCTFH (SEQ ID NO: 66); GPDCAYHRGDapLVWCTFH (SEQ ID NO: 67); GPDCAYHRGDabLVWCTFH (SEQ ID NO: 68); GPDCAYHRGOmLVWCTFH (SEQ ID NO: 69); and GPDCAYHRGKLVWCTFH (SEQ ID NO: 70).

Furthermore, FcBP is also described in detail in the following documents, and it will be clear to those of ordinary skill in the related art that the FcBPs disclosed in the following documents can be used in a method provided by one embodiment of the present application without limitation. This is because the method provided by one embodiment of the present application relates to a method of increasing the yield of an antibody comprising a functional group, which is a product, by adding a thiol-reactive additive in the process of transferring a functional group to an antibody using a compound comprising FcBP and a thioester group (that is, a compound for transferring a functional group).

Additional documents describing FcBP in detail:
(1) US Patent Application No. 10/149835, Patent No. US 7608681 B2;
(2) US Patent Application No. 15/575138, Patent No. US US 10227383 B2;
(3) DeLano, Warren L., et al., "Convergent solutions to binding at a protein-protein interface," Science 287.5456 (2000): 1279-1283;
(4) Sockolosky, Jonathan T., Saul Kivimae, and Francis C. Szoka, "Fusion of a short peptide that binds immunoglobulin G to a recombinant protein substantially increases its plasma half-life in mice," PLoS One 9.7 (2014): e102566;
(5) Yue Zeng. et al., "A Traceless Site-Specific Conjugation on Native Antibodies Enables Efficient One-Step Payload Assembly," Angewandte Chemie (2022): e202204132;
(6) Viktoriia Postupalenko., et al., "Template directed synthesis of antibody Fc Conjugates with concomitant ligand release," Chemical Science 13 (2022): 3965-3976;
(7) Cao, Yu J., et al., "Synthesis of precision antibody conjugates using proximity-induced chemistry," Theraostics 11.18 (2021): 9107-9117;
(8) Muguruma, Kyohei., et al., "Novel Hybrid Compound of a Plinabulin Prodrug with an IgG Binding Peptide for Generating a Tumor Selective Noncovalent-Type Antibody-Drug Conjugate," Bioconjugate Chemistry 27.7 (2016): 1606-1613; and
(9) Park, Jisoo., at al., "Peptide-directed photocrosslinking for site-specific conjugation of IgG," Bioconjugate Chemistry 29.10 (2018): 3240-3244.

Modification typically added to a peptide or protein may be added to FcBP. The modified FcBP refers to FcBP to which modification that is commonly performed on a peptide or protein in the related art has been added. Modified FcBP has been used in some studies. For example, modifications may include the conjugation of a fatty acid group, the addition of a hydroxyl group, the imidation of the C terminus, the acetylation of the N terminus, PEGylation (e.g., the conjugation of a PEG moiety to the C terminus and/or the N terminus), and/or the addition of a linker or the like. For example, the PEG moiety may include 2 to 40 ethylene glycol units.

### Overview of conventional method of transferring functional group site-specifically to antibody

The site-specific transfer of a functional group to an antibody using an Fc-binding peptide is achieved by a compound comprising an Fc-binding peptide. There are two cases of site-specific transfer of a functional group to an antibody using an Fc-binding peptide, which had been studied conventionally, and these cases are as follows:
(Case 1) A functional group is transferred to an antibody, together with an Fc-binding peptide, through the reaction between a compound comprising the Fc-binding peptide and the antibody.
(Case 2) A functional group is transferred to an antibody without an Fc-binding peptide through the reaction between a compound comprising the Fc-binding peptide and the antibody.

`Case 1' is described in detail in the documents [European Patent Application No. 18791007.0, Publication No. EP 3617235; and PCT International Application No. PCT/KR2020/001145, Publication No. WO2020/153774]. `Case 2' is described in detail in the documents [European Patent Application No. 19818561.3, Publication No. EP 3811978; and PCT International Application No. PCT/KR2020/003282, Publication No. WO2020/184944]. As in Case 1, when FcBP remains in the antibody, a process of removing FcBP from a conjugate may be additionally required. This is because the FcBP conjugated to the antibody has a negative effect on the half-life of the antibody.

Among the above two cases, Case 2 is based on a reaction in which an Fc-binding peptide is released during the reaction process and only a functional group is transferred to an antibody, and thus, in the antibody-drug conjugate prepared by the reaction mechanism of Case 2 (unlike Case 1), the half-life is not reduced or no additional process of removing FcBP is needed. Case 2 allows the transfer of a functional group to an antibody to be achieved in one step. That is, a one-step reaction allows the transfer of a functional group such as a bio-orthogonal functional group to an antibody. Conjugation accomplished by the reaction mechanism of Case 2 in which FcBP is released through the reaction is referred to as traceless crosslinking, traceless reaction, or traceless conjugation (refer to the documents [Site-Selective Antibody-Drug Conjugation by a Proximity-Driven S to N Acyl Transfer Reaction on a Therapeutic Antibody; and A Traceless Site-Specific Conjugation on Native Antibodies Enables Efficient One-Step Payload Assembly]).

### Factors that are used in conventional method of transferring functional group site-specifically to antibody

As described above, at least the following two factors are required to transfer a functional group site-specifically to an antibody:
(1) a compound for transferring a functional group to an antibody, including FcBP and a functional group to be transferred; and
(2) an antibody.

Here, the compound comprising FcBP has a reaction site with a reactive group (e.g., a nucleophile such as -NH₂) of an antibody. Here, the reaction site is located between FcBP and a functional group to be transferred in the compound for transferring a functional group to the antibody. The reaction between the compound for transferring a functional group and the antibody may be described more specifically as the reaction of a reaction site in the compound for transferring a functional group and a nucleophile of the antibody, and at the same time as this reaction, the FcBP in the compound for transferring a functional group is released and the functional group in the compound for transferring a functional group is transferred to a specific site of the antibody. An antibody's nucleophile associated with the specific site of the antibody is located in the Fc domain. For example, the antibody's nucleophile may be present at the above-described labeling site, and for example, the labeling site may be present in the Fc domain. For example, the labeling site may be a lysine residue located in the Fc domain. For example, the labeling site may be one or more selected from K246, K248, K274, K288, K290, K317, K320, K322, K338, K360, K414, and K439.

A compound for transferring a functional group to an antibody is induced by FcBP contained in the compound for transferring a functional group at a predetermined labeling site of the antibody (located in the Fc domain). This induction by FcBP allows a nucleophile of the labeling site to react with the reaction site included in the compound for transferring a functional group designed to react with the nucleophile, thereby achieving the transfer of a functional group to the labeling site.

Methods of transferring a functional group site-specifically to an antibody will be described in detail in the above-described documents. Hereinafter, site-specific conjugation methods using a compound which comprises a thioester group for transferring a functional group are described in detail in the documents [PCT International Application No. PCT/KR2020/003282, Publication No. WO2020/184944; Site-Selective Antibody-Drug Conjugation by a Proximity-Driven S to N Acyl Transfer Reaction on a Therapeutic Antibody; A Traceless Site-Specific Conjugation on Native Antibodies Enables Efficient One-Step Payload Assembly, etc.].

### Example of conventional method of transferring functional group site-specifically to antibody - use of compound, for transferring functional group, comprising thioester group

The above documents disclose compounds comprising a thioester group, designed for a reaction site with a nucleophile of the antibody, to transfer a functional group to an antibody. The thioester group or the compound, for transferring the functional group, comprising the thioester group acts as a donor of an acyl group, and transfers the acyl group to a nucleophile (e.g., -NH₂) of an antibody through a reaction with the nucleophile of the antibody. Here, as described above, FcBP contained in the compound for transferring a functional group serves to guide the compound for transferring a functional group to the Fc domain of the antibody. Furthermore, FcBP positions the two elements sufficiently close to allow an amide group at specific site(s) (e.g., K246 and/or K248) of the antibody to react with a thioester group included in the compound for transferring a functional group.

Hereinafter, to prepare an antibody comprising a functional group, a reaction between the antibody and a compound for transferring a functional group to the antibody is illustrated. The following example is based on the contents described in the documents [PCT International Application No. PCT/KR2020/003282, Publication No. WO2020/184944].

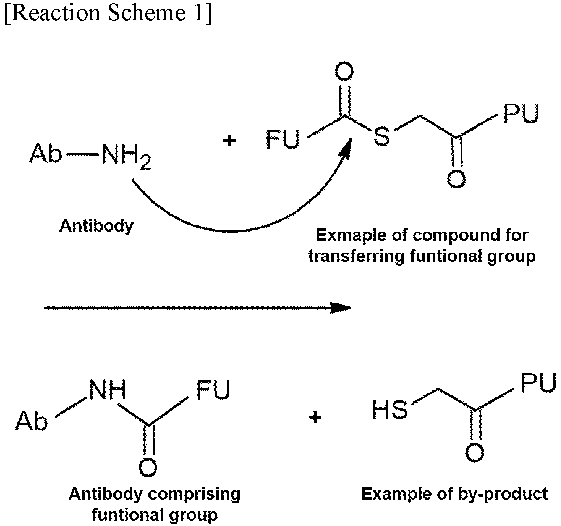

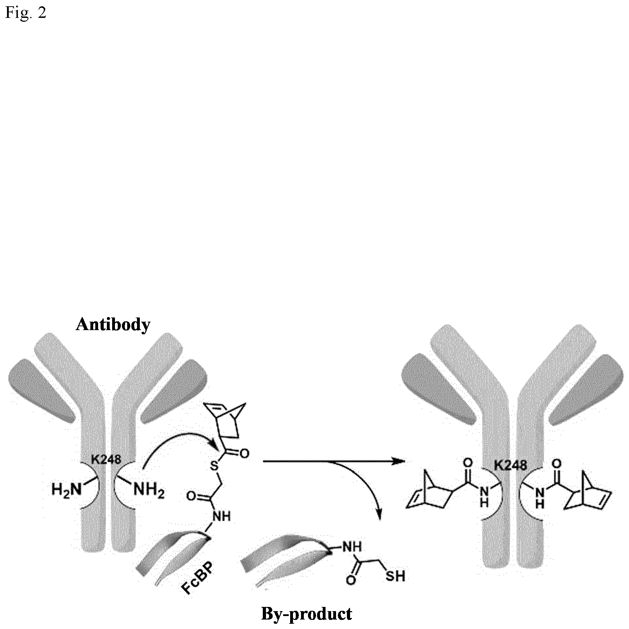

Reaction Scheme 1 illustrates the reaction between an antibody and a compound for transferring a functional group to an antibody. Here, as shown above, the compound for transferring a functional group to an antibody (that is, a compound for transferring a functional group) includes (1) a functional unit (FU) comprising a functional group, (2) a peptide unit (PU) comprising FcBP, and (3) a thioester group (-C(=O)S-). By the reaction between the amide group, which is the nucleophilic group of the antibody, and the thioester group of the compound for transferring a functional group, an acyl group of the thioester group is transferred to the nucleophilic group of the antibody. By the reaction between the antibody and the compound for transferring a functional group, an antibody comprising the functional group and a by-product (thiol-containing compound) are produced. The reaction between a compound participating in the reaction and the antibody is explained in further detail in FIG. 2 (refer to the document [Site-Selective Antibody-Drug Conjugation by a Proximity-Driven S to N Acyl Transfer Reaction on a Therapeutic Antibody]).

As shown in FIG. 2, a functional group such as a bio-orthogonal functional group (norbornene) is transferred to a specific site (K248) of an antibody through S-to-N acyl transfer using a thioester group. As a result, each bio-orthogonal chemical group is transferred to a target site (e.g., K248) located on each of the two heavy chains of the antibody.

Likewise, when a compound comprising the thioester group and FcBP, and transferring a functional group is used, the functional group may be site-specifically transferred to an antibody. The use of a thioester group not only exhibits higher site selectivity than the use of another reaction group (e.g., NHS ester) with a similar function, but also enables the reaction between an antibody and a compound for transferring a functional group under a physiological condition (e.g., the condition of about pH 7.4).

However, a problem was discovered in the above reaction mechanism that ensures high site selectivity. Hereinafter, problems found by the inventors of the present application and the invention provided by the present application to solve these problems will be described in detail.

### Problems occurring due to use of compound for transferring functional group to antibody, including thioester group - newly found problems

### Found problems

The inventors of the present application discovered the problem that the production yield of an antibody comprising a functional group was lower when using the above-described reaction for transferring a functional group site-specifically to an antibody using a thioester group. Here, the final product produced by the reaction of an antibody and a compound for transferring a functional group may be an antibody comprising two functional groups (here, since the antibody contains two heavy chains and thus provides two labeling sites, when a functional group is connected to each labeling site, an antibody comprising two functional groups is prepared), or an antibody comprising one functional group (here, the antibody includes two heavy chains, and when a functional group is connected to a labeling site located on one of the heavy sites, an antibody comprising one functional group is prepared). Here, to show the number of functional groups connected to an antibody, similar to an antibody-drug conjugate, parameters of a functional group-to-antibody ratio (FAR) may be used. In the field of antibody-drug conjugates, for the number of drugs connected to an antibody, parameters of a drug-to-antibody ratio (DAR) are used. For example, DAR2 ADC represents ADC in which two drugs are connected to an antibody. Similarly, FAR 2 indicates that two functional groups are connected to an antibody, and FAR 1 indicates that one functional group is connected to an antibody. That is, the antibody comprising two functional groups may be referred to as FAR 2 antibody, and the antibody comprising one functional group may be referred to as FAR 1 antibody. For example, FIG. 2 shows that, finally, an antibody comprising two functional groups (norbornene) was produced, which can be understood to mean that the FAR 2 antibody was produced. There are no other limitations on functional groups, and since functional groups can be designed to include one or more bio-orthogonal chemical groups or drugs, examples of FAR 2 antibodies encompass DAR 1, DAR 2, DAR 3, DAR 4, DAR 5, DAR 6, DAR 7, and DAR 8 antibody-drug conjugates (ADCs).

Referring to Reaction Scheme 1 and FIG. 2, it can be seen that (1) an antibody comprising functional groups and (2) a by-product are produced by the reaction between a compound for transferring functional groups, including a thioester group and an antibody. It was predicted that the produced by-product would not inhibit the reaction between the antibody and the compound for transferring functional groups.

However, the inventors of the present application newly found the problems that a reaction for transferring a functional group to an antibody using a thioester group (here, the yield indicates the yield of an antibody comprising a functional group) exhibits a lower yield and an insufficient reaction speed.

Further, the inventors of the present application found that the lower yield or reaction speed is caused by the resulting by-product.

To solve the above problems, the present application provides an invention characterized by capping a thiol group of the by-product with a thiol-reactive additive.

### Exploring cause of problems

As described above, there was a problem in that the production yield of an antibody comprising a functional group or the reaction speed of a reaction for preparing the antibody was lower. There may be a variety of causes for a lower production yield. For example, the production yield may be affected by a low reaction speed in the first place, inappropriate reaction conditions, inappropriate concentration of a reaction material, or interference with the reaction by the material produced thereby.

As described above, initially, it was predicted that the resulting by-product would not inhibit the reaction of the antibody and the compound for transferring a functional group.

In addition, as shown above, the by-product includes a free thiol group at an end. Since there is no group that can react with thiol in a general antibody, it was predicted that the by-product would not interfere with the reaction of an unreacted antibody and an unreacted compound for transferring a functional group.

Accordingly, it was difficult to easily predict a cause of the lower production yield of the antibody comprising a functional group.

As a result, the inventors of the present application were able to increase the production yield or production speed of the antibody comprising a functional group in the reaction by capping a thiol group of the by-product with a thiol-reactive additive (i.e., removing a thiol group). As a result of inferring backwards from the result of an experiment disclosed in the present application, the inventors of the present application found that the by-product is a factor that has a negative effect on the production yield. The reaction inhibition mechanism of the by-product predicted inversely from the experimental result will be described below.

### Reaction inhibition mechanism of by-product

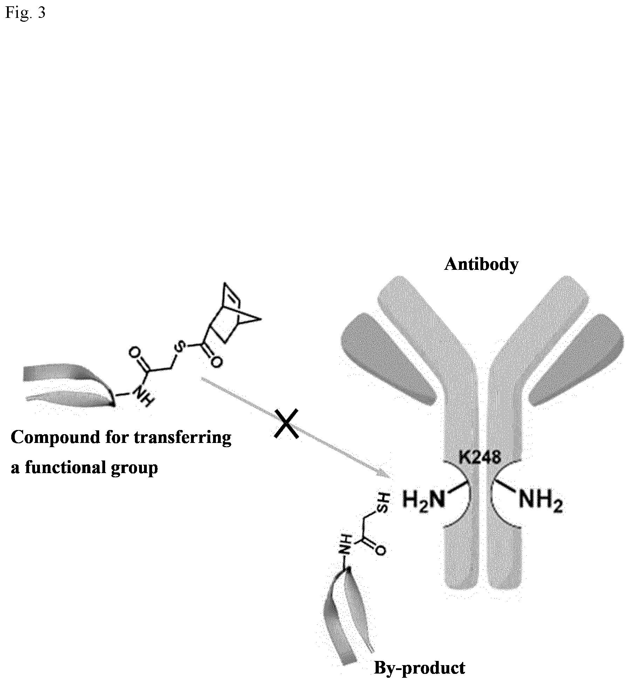

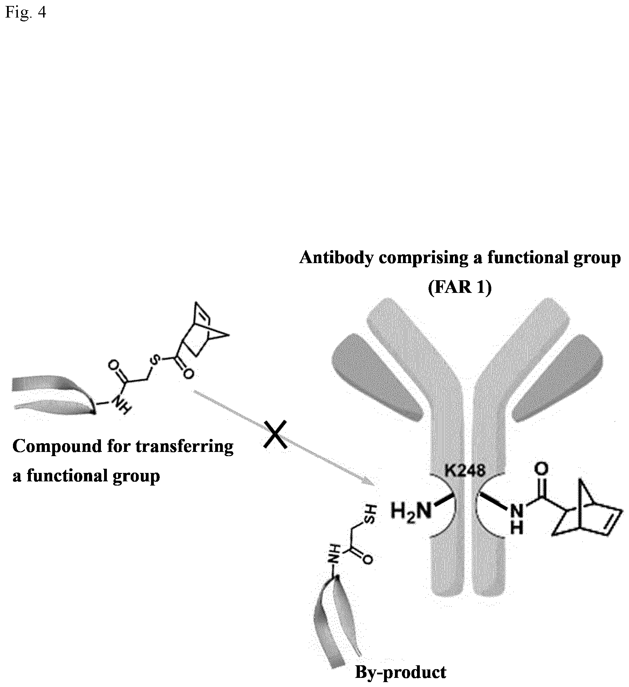

Through the following examples, the mechanism of a by-product for inhibiting the reaction of an antibody and an agent for transferring a first chemical functional group for a functional group antibody is described. This is to help with understanding of the present application, and the scope of the present application is not limited by the examples below. In order to more easily understand the reaction inhibition mechanism of the by-product, FIGS. 3 and 4 illustrating reaction inhibition may be referred to. FIG. 3 is a diagram illustrating that the resulting by-product inhibits the reaction of an unreacted antibody and an unreacted compound for transferring a functional group. FIG. 4 is a diagram illustrating that the resulting by-product inhibits the reaction of an antibody comprising a functional group of FAR 1 and a compound for transferring a functional group.

The resulting by-product can act as a competitive material for a compound for transferring a functional group in the reaction of an antibody and a compound for transferring a functional group.

In one example, a by-product may be located at a reaction site of the Fc domain of an antibody, which reacts with a compound for transferring a functional group, by FcBP contained in the by-product. Further, a thiol group of the by-product may chemically act with a nucleophile at the reaction site of the antibody, and here, the by-product may inhibit the reaction between a compound for transferring a functional group and an antibody.

For example, the by-product may be located at the reaction site where the compound for transferring a functional group reacts with the antibody. Here, a thiol group of the by-product may chemically act with a nucleophile of the antibody, and the by-product may inhibit the reaction of the compound for transferring a functional group and the antibody. For example, the nucleophile of the antibody may be an amino group. Here, the chemical action between the thiol group of the by-product and the amino group of the antibody may be electrostatic interaction. For example, the electrostatic interaction may be a hydrogen bond. Here, the amino group of the antibody may be an amino group of lysine contained in the Fc domain of the antibody. Here, the amino group of the antibody may be one or more amino groups selected from lysine 246, lysine 248, lysine 274, lysine 288, lysine 290, lysine 317, lysine 320, lysine 322, lysine 338, lysine 360, lysine 414, and lysine 439, which are located in the Fc domain of the antibody.

For example, the by-product may be located at a reaction site where a compound for transferring a functional group and an antibody react, and here, a thiol group of the by-product may chemically act with an amino group of the antibody. Here, the by-product may inhibit the reaction of the compound for transferring a functional group and the antibody. For example, H of the thiol group of the by-product and N of the amino group may have electrostatic interaction, or S of the thiol group of the by-product and H of the amino group may have electrostatic interaction. Here, the electrostatic interaction may be a hydrogen bond.

When the by-product inhibits the reaction of the compound for transferring a functional group and the antibody during the process of preparing an antibody comprising a functional group, it may not be possible to obtain a desired amount of an antibody (FAR 1 or FAR 2) comprising a functional group, or it may take more time to obtain a desired amount of an antibody comprising a functional group.

The inventors of the present application resolved the problem that a desired amount of an antibody comprising a functional group is not obtained and the problem that it takes more time to obtain a desired amount of an antibody comprising a functional group through a method of removing a thiol group of the by-product. When the by-product that inhibits a reaction is removed during the process of preparing an antibody comprising a functional group, it can be understood that the yield of the antibody comprising a functional group will increase.

To solve the above-described problems, the present application provides a method of preparing an antibody comprising a functional group additionally using a thiol-reactive additive. By removing the thiol-containing by-product using a thiol-reactive additive, the problem of inhibition reaction between a compound for transferring a functional group and an antibody, which is caused by the thiol-containing by-product. The thiol-reactive additive reacts with the thiol group of the thiol-containing by-product to cap the thiol group. The thiol-capping by-product may be referred to as a second by-product to avoid confusion with the thiol-containing by-product, and when the second by-product and the thiol-containing by-product are described together, the thiol-containing by-product is referred to as a first by-product. In some embodiments, the second by-product produced by reacting the first by-product with the thiol-reactive additive may not inhibit the reaction between the compound for transferring a functional group and the antibody. This is because the thiol group that causes the inhibition of the reaction of the first by-product has been removed. As a result, the resulting second by-product may not affect the yield of the antibody comprising a functional group without inhibiting the reaction between the compound for transferring a functional group and the antibody during the process of preparing an antibody (FAR 1 or FAR 2) comprising a functional group. It is assumed that the addition of a new group to the thiol group (e.g., capping of the thiol group) not only prevents the interaction between the thiol group and a nucleophile of the antibody but also reduces the binding affinity of the by-product comprising FcBP with the Fc domain of the antibody.

The main characteristic of the method provided by the present application is to increase the production yield of an antibody comprising a functional group by removing the thiol-containing by-product that inhibits the reaction between the compound for transferring a functional group and the antibody.

Hereinafter, a method of increasing the yield of the antibody comprising a functional group provided by the present application will be described in detail.

### Overview of method of increasing production yield of antibody comprising functional group using thiol-reactive additive

As described above, the inventors of the present application developed a method of increasing the production yield of an antibody comprising a functional group using a thiol-reactive additive. The following three factors are essentially involved in the method of increasing the production yield of an antibody comprising a functional group using a thiol-reactive additive:
(1) a thiol-reactive additive;
(2) an antibody; and
(3) a compound for transferring a functional group to the antibody.

Methods of transferring a functional group to the specific site of an antibody using a compound for transferring the functional group to the antibody, in which among these three factors the antibody and the compound for transferring the functional group to the antibody are involved, were previously studied in detail, and are disclosed in detail in the following documents [European Patent Application No. 19818561.3, Publication No. EP 3811978; PCT International Application No. PCT/KR2020/003282, Publication No. WO2020/184944; Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759; and Zeng, Yue, et al., "A Traceless Site-Specific Conjugation on Native Antibodies Enables Efficient One-Step Payload Assembly," Angewandte Chemie International Edition 61.36 (2022): e202204132].

The use of the method of increasing the production yield of an antibody comprising a functional group using a thiol-reactive additive is limited to a use of a compound for transferring a functional group to an antibody, comprising a thioester group. This is because the compound for transferring a functional group to an antibody, comprising a thioester group produces a thiol-containing by-product by reacting with the antibody and a thiol-reactive additive is used for removing the reaction inhibition effect of the thiol-containing by-product. The thiol-reactive additive may pre-exist in a composition before the reaction of the antibody with the compound for transferring a functional group. The thiol-reactive additive may be added into the composition after the reaction of the antibody with the compound for transferring a functional group. In this way, it is more important that the thiol-reactive additive participate in the reaction of the antibody with the compound for transferring a functional group, and the timing of addition or participation in the reaction of the thiol-reactive additive is not particularly limited.

Hereinafter, among the three factors used in the method of increasing the production yield of an antibody comprising a functional group using a thiol-reactive additive, the compound for transferring a functional group and the antibody, which had been studied previously, and the method of preparing an antibody comprising a functional group using the same will be described in detail.

### Factor 1 used in method of site-specifically transferring functional group to antibody - compound for transferring functional group

### Overview of compound for transferring functional group to antibody

As described above, a compound for transferring a functional group includes a thioester group. Further, the compound for transferring a functional group includes an Fc-binding peptide (FcBP). Further, the compound for transferring a functional group includes a functional group to be transferred to an antibody. That is, the compound for transferring a functional group includes the following three essential factors: a thioester group; FcBP; and a functional group.

In some embodiments, the compound for transferring a functional group may be a compound having the structure of Formula 1:

[Formula 1] FU-RU-PU.

Here, FU is a functional unit (FU) comprising a functional group. The functional group may include, for example, one or more selected from one or more bio-orthogonal chemical groups, and one or more active moieties. Here, each bio-orthogonal chemical group or active moiety included in the functional group may be independently selected. Bio-orthogonal chemical groups or active moieties will be described in detail below.

Here, RU is a reactive unit comprising a thioester group. The reactive unit includes a thioester group designed for the reaction with a nucleophile of an antibody. As described above, by the reaction of the nucleophile of the antibody with the thioester group of the compound for transferring a functional group, an acyl group connected to the S atom of the compound for transferring a functional group is transferred from the compound for transferring a functional group to the antibody.

Here, PU is a peptide unit comprising FcBP. The peptide unit and FcBP will be described in detail below.

### Reactive unit and thioester group

As described above, the compound for transferring a functional group includes a reactive unit (RU) comprising a thioester group. The reactive unit may be a reactive unit for the reaction with the antibody.

The thioester group may be expressed as the structure of -C(=O)S- or -SC(=O)-, or as the structure of 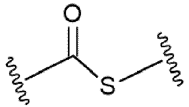 or 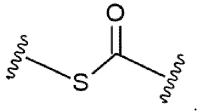 Here, waves represent that the thioester group is connected with another part of the compound for transferring a functional group. In a specific embodiment, the thioester group may be expressed as the structure 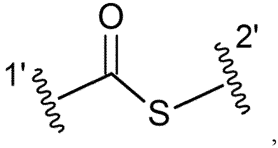 wherein 1' may indicate the part connected to a functional unit, and 2' may indicate the part connected to a peptide unit. That is, the carbonyl group (-C(=O)-) of the thioester group is located closer to the functional unit, and the -S- of the thioester group is located closer to the peptide unit. The reason that the structure is designed as above is to ensure the acyl group to be transferred to the antibody through the reaction of the nucleophile (e.g., -NH₂) of the antibody with the thioester group. Here, the acyl group is used to refer to a group comprising the -C(=O)- of the thioester group and the functional unit, and does not include the -S- of the thioester group. When the compound for transferring a functional group reacts with an amino group of the antibody, the acyl group is transferred to the amino group of the antibody from the compound for transferring a functional group, which may be referred to as S to N acyl transfer.

In some embodiments, the reactive unit may include a linker group other than the thioester group. For example, the linker group may be designed to adjust the distance between the peptide unit comprising FcBP and the thioester group. In another example, the linker group may be designed to adjust the distance between the functional unit comprising a functional group and the thioester group. For example, the length of the linker group may be 1 to 50 based on the number of atoms in the main chain. In a specific embodiment, the length of the linker group may be 1 to 10 based on the number of atoms in the main chain. The linker group may include one or more selected from one or more independently selected hetero atoms and one or more independently selected cyclic groups in the main chain, but the present application is not limited thereto. Further, the linker group may include one or more independently selected substituents, but the present application is not limited thereto. Here, when a cyclic group is present in the main chain, counting of the number of atoms in the main chain may be done based on two atoms that form bonds with other parts, not a ring on the ring for convenience. Here, counting is done in such a way that the smallest number can be achieved. For example, when the ring of 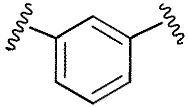 is present in the main chain of the linker group, the number of atoms in the main chain counted by the ring is 3. For example, when the ring of 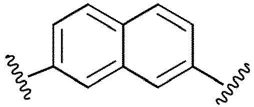 is present in the main chain of the linker group, the number of atoms in the main chain counted by the ring is 5.

In some embodiments, the reactive unit may have the following structure:

-D¹-X-D²-.

Here, X is a thioester group. X may be connected with a functional unit or peptide unit via D¹. X may be connected with a functional unit or peptide unit via D².

Here, D¹ may be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, or substituted or unsubstituted arylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, - C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and - SH. When D¹ is a bond, X is directly connected with a functional unit or peptide unit.

Here, D² may be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, substituted or unsubstituted arylene, or substituted or unsubstituted heteroarylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When D² is a bond, X is directly connected with a functional unit or peptide unit.

In a specific embodiment, D¹ may be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -NO₂, -OH, and -SH.

In a specific embodiment, D² may be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -NO₂, -OH, and -SH.

In some embodiments, the reactive unit may have the following structure:

-D¹¹-D¹²-D¹³-X-D²¹-D²²-D²³-.

Here, X is a thioester group. X may be connected with a functional unit or peptide unit via -D¹¹-D¹²-D¹³-. X may be connected with a functional unit or peptide unit via -D²¹-D²²-D23-.

Here, each of D¹¹, D¹², and D¹³ may independently be a bond, substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, or substituted or unsubstituted arylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When all of D¹¹, D¹², and D¹³ are bonds, X is directly connected with a functional unit or peptide unit.

Here, each of D²¹, D²², and D²³ may independently be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, substituted or unsubstituted arylene, or substituted or unsubstituted heteroarylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, - C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When all of D²¹, D²², and D²³ are bonds, X is directly connected with a functional unit or peptide unit.

In a specific embodiment, each of D¹¹, D¹², and D¹³ may independently be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -OH, and -SH.

In a specific embodiment, each of D²¹, D²², and D²³ may independently be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -NO₂, -OH, and -SH.

For a proper reaction between the thioester group and the nucleophile at a specific site of the antibody, the distance between the thioester group and FcBP needs to be appropriately designed. This is because FcBP induces the thioester group near the nucleophile of the antibody. In a specific embodiment, X may be connected with a functional unit via -D¹¹-D¹²-D¹³-, and connected with a peptide unit via -D²¹-D²²-D²³-. Here, all of D¹¹, D¹², and D¹³ may be bonds. Here, each of D²¹, D²², and D²³ may independently be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, - C(=O)OH, -C(=O)NH₂, -NH₂, =O, =S, -NO₂, -OH, and -SH. Here, the length of -D²¹-D²²-D²³- may be 1 to 10 based on the number of atoms in the main chain. Here, X may be - C(=O)S-, S may be connected with -D²¹-D²²-D²³-, and the carbonyl group (C(=O)) may be connected with -D¹¹-D¹²-D¹³-.

In a specific embodiment, the reactive unit may have any one structure selected from the following structures, but the present application is not limited thereto: 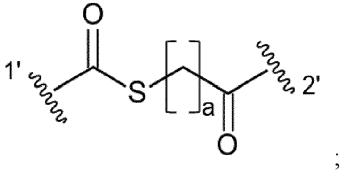 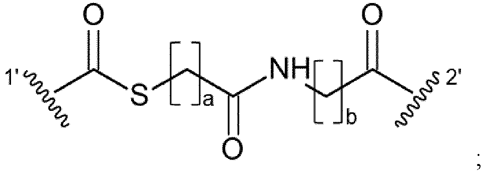 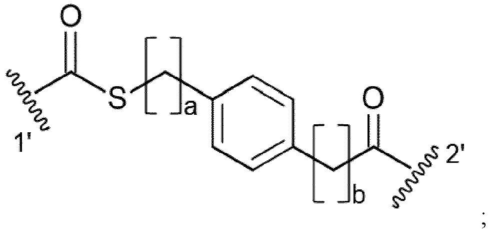 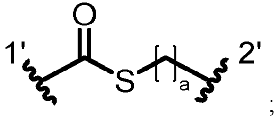 and

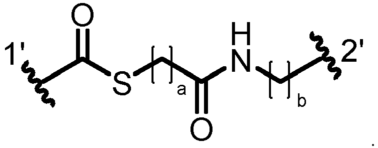

Here, a is an integer of 0 to 5.

Here, b is an integer of 0 to 5.

Here, 1' indicates a part connected with a functional unit, and 2' indicates a part connected with a peptide unit.

### Functional unit and functional group

As described above, the compound for transferring a functional group includes a functional unit (FU) comprising a functional group. The functional group is used to refer to a group with one or more functions, but the present application is not limited thereto. In some embodiments, the functional group may include a functional group and/or an active moiety, which are/is designed to react with another chemical reaction group such as a bio-orthogonal functional group or click chemistry functional group.

In some embodiments, the functional group may include a bio-orthogonal functional group. The bio-orthogonal functional group refers to a chemical functional group or group that participates in a bio-orthogonal chemical reaction. In some embodiments, the bio-orthogonal functional group may be a group participating in any one bio-orthogonal reaction selected from Staudinger ligation; copper-free azide-alkyne cycloaddition, comprising copper-catalyzed azide-alkyne cycloaddition (CuAAC) and strain-promoted azide-alkyne cycloaddition (SPAAC); tetrazine ligation; tetrazole ligation; oxime ligation; and isocyanide click reaction. In some embodiments, the bio-orthogonal functional group may be any one selected from an azide, terminal alkyne, cyclic alkyne (e.g., cyclooctin), tetrazine, norbornene, cycloalkene (e.g., cyclooctene), tetrazol, oxime, and an isocyanide group. The functional group may include one or more bio-orthogonal functional groups, and when a plurality of bio-orthogonal functional groups are included in the functional groups, each bio-orthogonal functional group may be independently selected.

In some embodiments, the functional group may include an active moiety. The active moiety may be, for example, any one selected from drugs (e.g., toxins), imaging moieties (e.g., a fluorescent protein such as a fluorescent dye and GFP; and a luminescent material such as luciferin), radioactive moieties, proteins with a specific function, peptides with a specific function, affinity materials (e.g., biotin, streptavidin, and aptamer), stabilizing materials, vitamins, DNA molecules, and hydrophilic moieties such as a polyethylene glycol (PEG) moiety. The functional group may include one or more active moieties, and when the functional group includes a plurality of active moieties, each active moiety may be independently selected.

In some embodiments, the active moiety may be a drug. The drug may be, for example, any one selected from auristatin, eribulin, tubulysin, geldanamycin (Kerr et al., 1997, Bioconjugate Chem. 8(6):781-784), maytansinoid (EP 1391213, ACR 2008, 41, 98-107), calicheamicin (U.S. Patent Publication No. 2009/0105461, Cancer Res. 1993, 53, 3336-3342), mertansine, daunomycin, doxorubicin, methotrexate, vindesine, SG2285 (Cancer Res. 2010, 70(17), 6849-6858), dolastatin, dolastatin analogs auristatin (U.S. Patent No. 5,635,483), cryptophycin, camptothecin, a rhizoxin derivative, a CC 1065 analog or its derivative, duocarmycin, an enediyne antibiotic, esperamicin, epothilone, a pyrrolobenzodiazepine (PBD) derivative, α-amanitin, a toxoid, a toll-like receptor 5 (TLR5) agonist, a toll-like receptor 7 (TLR7) agonist, a toll-like receptor 8 (TLR8) agonist, 1,4,7-triazacyclononane-1,4,7-triacetic acid (NOTA), and 1,4,7,10-tetraazacyclododecane-1,4,7,10-tetraacetic acid (DOTA), or a derivative thereof, but the present application is not limited thereto.

In some embodiments, the functional unit may include one or more linker groups in addition to the functional group. For example, the linker group may be designed to adjust the distance between the functional group and the reactive unit.

In some embodiments, the functional group may include one or more linker groups, in addition to the bio-orthogonal chemical reaction group and/or the active moiety. For example, the linker group may be used for the conformation of each bio-orthogonal chemical functional group and/or active moiety or the adjustment in their distance.

In some embodiments, the functional unit may have the following structure:

FG-D³-

Here, FG is a functional group (FG). The functional group may be connected with the reactive unit comprising a thioester group via D³.

Here, D³ may be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, or substituted or unsubstituted arylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, - C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and - SH. When D³ is a bond, FG is directly connected with the reactive unit.

In a specific embodiment, D³ may be a bond, or substituted or unsubstituted C₁₋₁₀ alkylene, substituted or unsubstituted C₁₋₁₀ heteroalkylene, substituted or unsubstituted C₂₋₁₀ alkenylene, substituted or unsubstituted C₂₋₁₀ heteroalkenylene, substituted or unsubstituted C₂₋₁₀ heteroalkylene, substituted or unsubstituted C₂₋₁₀ alkynylene, substituted or unsubstituted C₂₋₁₀ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, - C(=O)OH, -C(=O)NH₂, -NH₂, =O, =S, -NO₂, -OH, and -SH.

In some embodiments, the functional unit may have the following structure:

FG-D³¹-D³²-D³³-

Here, FG is a functional group (FG). The functional group may be connected with the reactive unit comprising a thioester group via -D³¹-D³²-D³³-.

Here, D³¹, D³², and D³³ may independently be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, or substituted or unsubstituted arylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When all of D³¹, D³², and D³³ are bonds, FG is directly connected with the reactive unit.

In a specific embodiment, D³¹, D³², and D³³ may independently be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, - C(=O)OH, -C(=O)NH₂, -NH₂, =O, =S, -OH, and -SH.

### Peptide unit and FcBP

As described above, the compound for transferring a functional group includes a peptide unit (PU) comprising an Fc-binding peptide (FcBP).

In some embodiments, FcBP may have a length of 5 to 50 amino acid residues. In some embodiments, FcBP may have a length of 5 to 30 amino acid residues. In a specific embodiment, FcBP may have a length of 8 to 20 amino acid residues. In a specific embodiment, FcBP may have a length of 11 to 19 amino acid residues.

The binding affinity of FcBP with an antibody or the Fc domain of an antibody may be expressed as a dissociation constant (Kd). In some embodiments, the dissociation constant (Kd) of FcBP for the antibody may be 10 µM, 1 µM (1×10⁻⁶ M), 900 nM, 800 nM, 700 nM, 600 nM, 500 nM, 450 nM, 400 nM, 350 nM, 300 nM, 250 nM, 200 nM, 180 nM, 160 nM, 140 nM, 120 nM, 100 nM, 90 nM, 80 nM, 70 nM, 60 nM, 50 nM, 40 nM, 30 nM, 20 nM, 10 nM, 5 nM, 1 nM (1×10⁻⁹M), 0.5 nM, 0.1 nM, 0.05 nM, or 0.01 nM or less, or may be within the range set by any two values selected from the above-mentioned values. In some embodiments, the dissociation constant (Kd) of FcBP for the Fc domain of the antibody may be 10 µM, 1 µM (1×10⁻⁶M), 900 nM, 800 nM, 700 nM, 600 nM, 500 nM, 450 nM, 400 nM, 350 nM, 300 nM, 250 nM, 200 nM, 180 nM, 160 nM, 140 nM, 120 nM, 100 nM, 90 nM, 80 nM, 70 nM, 60 nM, 50 nM, 40 nM, 30 nM, 20 nM, 10 nM, 5 nM, 1 nM (1×10⁻⁹ M), 0.5 nM, 0.1 nM, 0.05 nM, or 0.01 nM or less, or may be within the range set by any two values selected from the above-mentioned values.

In some embodiments, modification may be added to FcBP. Modification may be, for example, any one or more selected from the conjugation of a fatty acid group, the addition of a hydroxyl group, the amidation of the C terminus, the acetylation of the N terminus, PEGylation (e.g., the conjugation of a PEG moiety to the C terminus and/or the N terminus), and the addition of a linker, but the present application is not limited thereto.

In some embodiments, FcBP may include the following amino acid sequence (refer to the document [PCT International Application No. PCT/KR2020/003282, Publication No. WO2020/184944]):
(Xaa)₁₋₃-C-(Xaa)₂-H-Xa¹-G-Xa²-L-V-Xa³-C-(Xaa)₁₋₃ (SEQ ID NOs: 72 to 79),

Here,
each Xaa may independently be any amino acid except cysteine,
Xa¹ may be selected from cysteine, leucine, diaminopropionic acid (Dap), diaminobytyric acid (Dab), ornithine (orn), and lysine,
Xa² may be glutamic acid or asparagine, and
Xa³ may be selected from tryptophane, naphthylalanine, and phenylalanine.

In a specific embodiment, a peptide comprising the amino acid sequence may consist of 13 or more and 17 or less amino acid residues. In a specific embodiment, a cysteine 2 to 4 amino acids apart from the N terminus and a cysteine 2 to 4 amino acids apart from the C terminus may be optionally connected with each other. In a specific embodiment, X₃ may be NH₂. In a specific example, (Xaa)₂ may be AW. In a specific example, Xa² may be glutamic acid. In a specific example, Xa³ may be tryptophan.

In a specific embodiment, FcBP may include the following amino acid sequence (refer to the document [PCT International Application No. PCT/KR2020/003282, Publication No. WO2020/184944]):
D-C-(Xaa)₂-H-Xa¹-G-Xa²-L-V-Xa³-C-T (SEQ ID NO: 80).

Here,
each Xaa may independently be any amino acid except cysteine,
Xa¹ may be selected from cysteine, leucine, diaminopropionic acid (Dap), diaminobytyric acid (Dab), ornithine (orn), and lysine,
Xa² may be glutamic acid or asparagine, and
Xa³ may be selected from tryptophane, naphthylalanine, and phenylalanine.

In some embodiments, FcBP may include any one sequence selected from the following amino acid sequences, or a sequence having about 80%, 81%, 82%, 83%, 84%, 85%, 86%, 87%, 88%, 90%, 91%, 92%, 93%, 94%, 95%, 96%, 97%, 98%, or 99% sequence identity with the same:
RGNCAYHKGQIIWCTYH (SEQ ID NO: 2); RGNCAYHKGQIVWCTYH (SEQ ID NO: 3); RGNCAYHKGQVVWCTYH (SEQ ID NO: 4); RGNCAYHKGQAVWCTYH (SEQ ID NO: 5); RGNCAYHKGQLLWCTYH (SEQ ID NO: 6); RGNCAYHKGQLIWCTYH (SEQ ID NO: 7); DCAYHKGQIVWCT (SEQ ID NO: 8); DCAYHKGQVVWCT (SEQ ID NO: 9); DCAYHKGQAVWCT (SEQ ID NO: 10); RGNCAYHKSQIIWCTYH (SEQ ID NO: 11); RGNCAYHKNQIIWCTYH (SEQ ID NO: 12); RGNCAYHKDQIIWCTYH (SEQ ID NO: 13); RGNCAYHKQQIIWCTYH (SEQ ID NO: 14); RGNCAYHKEQIIWCTYH (SEQ ID NO: 15); RGNCAYHKFQIIWCTYH (SEQ ID NO: 16); RGNCAYHKYQIIWCTYH (SEQ ID NO: 17); RGNCAYHKWQIIWCTYH (SEQ ID NO: 18); RGNCAYHKHQIIWCTYH (SEQ ID NO: 19); RGNCAYHKTQIIWCTYH (SEQ ID NO: 20); RGNCAYHKLQIIWCTYH (SEQ ID NO: 21); CAYHKLQIVWC (SEQ ID NO: 22); CAYHKLQLIWC (SEQ ID NO: 23); CAYHKSQIVWC (SEQ ID NO: 24); RGNCAYHKGQLVFCTYH (SEQ ID NO: 25); RGNCAYHKGQQVWCTYH (SEQ ID NO: 26); RGNCAYHKGQEVWCTYH (SEQ ID NO: 27); CAYHKGQLVWC (SEQ ID NO: 28); RGNCAYHKAQLVWCTYH (SEQ ID NO: 29); RGNCAYHKVQLVWCTYH (SEQ ID NO: 30); RGNCAYHKLQLVWCTYH (SEQ ID NO: 31); RGNCAYHKIQLVWCTYH (SEQ ID NO: 32); RGNCAYHKSQLVWCTYH (SEQ ID NO: 33); RGNCAYHKTQLVWCTYH (SEQ ID NO: 34); RGNCAYHKNQLVWCTYH (SEQ ID NO: 35); RGNCAYHKDQLVWCTYH (SEQ ID NO: 36); RGNCAYHKQQLVWCTYH (SEQ ID NO: 37); RGNCAYHKEQLVWCTYH (SEQ ID NO: 38); RGNCAYHKFQLVWCTYH (SEQ ID NO: 39); RGNCAYHKRQLVWCTYH (SEQ ID NO: 40); RGNCAYHKHQLVWCTYH (SEQ ID NO: 41); RGNCAYHKWQLVWCTYH (SEQ ID NO: 42); RGNCAYHKYQLVWCTYH (SEQ ID NO: 43); RGNCAYFKGQLVWCTYH (SEQ ID NO: 44); RGNCAYYKGQLVWCTYH (SEQ ID NO: 45); RGNCAYWKGQLVWCTYH (SEQ ID NO: 46); RGNCAYRKGQLVWCTYH (SEQ ID NO: 47); RGNCAYGKGQLVWCTYH (SEQ ID NO: 48); DCAYHKGQLVWC (SEQ ID NO: 49); NCAYHKGQLVWC (SEQ ID NO: 50); CAYHKGQLVWCT (SEQ ID NO: 51); CAYHKSQLVWC (SEQ ID NO: 52); GNCAYHKGQIIWCTYH (SEQ ID NO: 53); RGNCAYHEGQIIWCTYH (SEQ ID NO: 54); GPDCAYHRGELVWCTFH (SEQ ID NO: 55) (refer to the document [European Patent Application No. 19818561.3, Publication No. EP 3811978]); DCAWHDapGELVWCT (SEQ ID NO: 56) (Dap: diaminopropionic acid); DCAWHDabGELVWCT (SEQ ID NO: 57) (Dab: diaminobutyric acid); DCAWHOrnGELVWCT (SEQ ID NO: 58) (Orn: ornithine); DCAWHKGELVWCT (SEQ ID NO: 59); DCAWHLGELVWCT (SEQ ID NO: 60) (refer to the documents [PCT Internaltional Application No. PCT/KR2020/003282, Publication No. WO2020/184944; and Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759]); RGNCAYHRGKLVWCTYH (SEQ ID NO: 61) (refer to the document [Zeng, Yue, et al., "A Traceless Site-Specific Conjugation on Native Antibodies Enables Efficient One-Step Payload Assembly," Angewandte Chemie International Edition 61.36 (2022): e202204132]); RGNCAYHKGQLVWCTYH (SEQ ID NO: 62); GPDCAYHDapGELVWCTFH (SEQ ID NO: 63); GPDCAYHDabGELVWCTFH (SEQ ID NO: 64); GPDCAYHOrnGELVWCTFH (SEQ ID NO: 65); GPDCAYHKGELVWCTFH (SEQ ID NO: 66); GPDCAYHRGDapLVWCTFH (SEQ ID NO: 67); GPDCAYHRGDabLVWCTFH (SEQ ID NO: 68); GPDCAYHRGOmLVWCTFH (SEQ ID NO: 69); and GPDCAYHRGKLVWCTFH (SEQ ID NO: 70).

In some embodiments, the cysteine residue adjacent to the N terminus and the cysteine residue adjacent to the C terminus of FcBP may be covalently linked (e.g., through a disulfide bond).

In some embodiments, FcBP may be connected with another part of the compound for transferring a functional group via the 1^{st}, 2^{nd}, 3^{rd}, 4^{th}, 5^{th}, 6^{th}, 7^{th}, 8^{th}, 9^{th}, 10^{th}, 11^{th}, 12^{th}, 13^{th}, 14^{th}, 15^{th}, 16^{th}, 17^{th}, 18^{th}, 19^{th}, or 20^{th} amino acid residue.

In some embodiments, the peptide unit may include one or more linker groups, in addition to FcBP. For example, the linker group may be designed to adjust the distance between FcBP and the reactive unit.

### Factor 2 used in method of site-selectively transferring functional group to antibody - antibody

A compound for transferring a functional group and an antibody are involved in the conventional method of site-specifically transferring a functional group to an antibody.

In some embodiments, the antibody may be a human antibody or humanized antibody. In some embodiments, the antibody may be an animal antibody, other than a human antibody. In some embodiments, the antibody may be a full-length antibody. In some embodiments, the antibody may be an antibody fragment. In some embodiments, the antibody may be a wild-type antibody or a manipulated antibody.

In some embodiments, the antibody may be IgG. In some embodiments, IgG may be IgG1, IgG2, IgG3, or IgG4.

In some embodiments, the antibody may be any one selected from adalimumab (Humira), rituximab (Rituxan), trastuzumab (Herceptin), bevacizumab (Avastin), infliximab (Remicade), pembrolizumab (Keytruda), nivolumab (Opdivo), eculizumab (Soliris), alemtuzumab (Lemtrada, Campath), daratumumab (Darzalex), ipilimumab (Yervoy), golimumab (Simponi), tocilizumab (Actemra), ranibizumab (Lucentis), secukinumab (Cosentyx), ixekizumab (Taltz), dupilumab (Dupixent), ustekinumab (Stelara), palivizumab (Synagis), durvalumab (Imfinzi), atezolizumab (Tecentriq), omalizumab (Xolair), vedolizumab (Entyvio), abciximab (ReoPro), basiliximab (Simulect), alefacept (Amevive), daclizumab (Zinbryta), and elotuzumab (Empliciti).

In some embodiments, the antibody may include the Fc domain of a human antibody. In some embodiments, the antibody may include a constant domain of a human antibody. In some embodiments, the antibody may include a heavy chain of a human antibody. In some embodiments, the antibody may include the constant domain of a heavy chain of a human antibody.

In some embodiments, the antibody may include the Fc domain of human IgG (e.g., IgG1). In some embodiments, the antibody may include a constant domain of human IgG. In some embodiments, the antibody may include a heavy chain of human IgG. In some embodiments, the antibody may include the constant domain of a heavy chain of human IgG.

In some embodiments, the antibody may be an antibody having binding ability to any one of EpCAM, CD2, CD3, CD4, CD5, CD6, CD11, CD19, CD20, CD22, CD26, CD30, CD33, CD37, CD38, CD40, CD44, CD56, CD79, CD105, CD138, EphA receptors, EphB receptors, EGFR, EGFRvIII, HER2, HER3, mesothelin, cripto, alphavbeta3, alphavbeta5, CLDN 18.2, and alpha v beta 6 integrin.

In some embodiments, the antibody may be anti-CLDN18.2 antibody (refer to the document [Korean Patent Application No. 10-2021-7023724]).

In some embodiments, the antibody may include an amino acid of GPSVFLFPPKPKDTLMI (SEQ ID NO: 1), or an amino acid sequence having about 80%, 81%, 82%, 83%, 84%, 85%, 86%, 87%, 88%, 90%, 91%, 92%, 93%, 94%, 95%, 96%, 97%, 98%, or 99% sequence identity with the same.

### Product 1 produced by reaction of antibody and compound for transferring functional group -antibody comprising functional group

By the reaction, contact, or mixing of an antibody and a compound for transferring a functional group, an antibody comprising a functional group is produced. That is, a functional group is transferred to an antibody. For example, when the functional group includes a bio-orthogonal functional group, the functional group comprising a bio-orthogonal functional group is transferred to an antibody, and an antibody comprising the bio-orthogonal functional group is prepared. The antibody comprising the bio-orthogonal functional group may have a reaction with payload including a bio-orthogonal functional group (e.g., a second bio-orthogonal functional group) designed to having a bio-orthogonal reaction with the bio-orthogonal functional group (e.g., a first bio-orthogonal functional group) and an active moiety, thereby preparing a conjugate in which the active moiety is linked to the antibody. When the active moiety is a drug, an antibody-drug conjugate is prepared. In another example, when the functional group includes an active moiety, the functional group comprising an active moiety is transferred to an antibody, thereby preparing an antibody comprising the active moiety. When the active moiety is a drug, an antibody-drug conjugate is prepared.

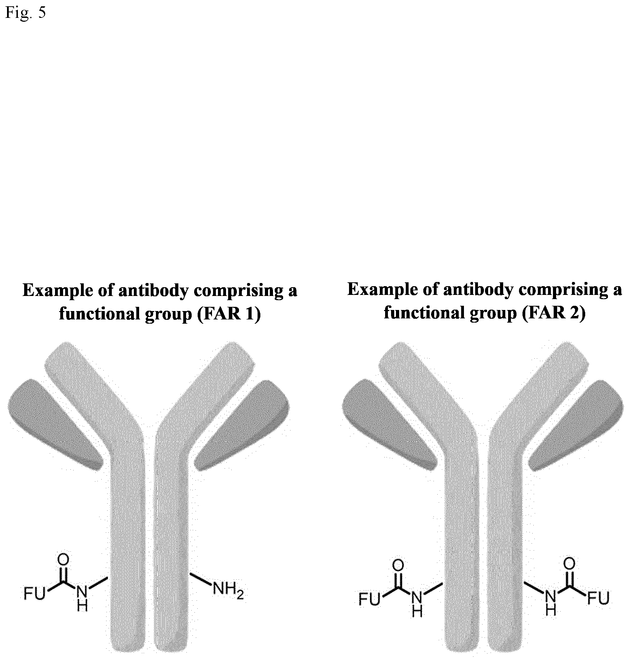

In some embodiments, the antibody comprising a functional group may include one functional group. As described above, the antibody comprising one functional group may be referred to as an antibody comprising a functional group of functional group-to-antibody ratio (FAR) 1. Examples of FAR 1 antibodies comprising a functional group are shown in FIG. 5.

In some embodiments, the antibody comprising a functional group may include two functional groups. More specifically, the antibody comprising one functional group may react with the compound for transferring a functional group, thereby preparing an antibody comprising two functional groups. In some embodiments, one functional group may be linked to a first target site (e.g., a first amino acid residue) in the Fc region of one heavy chain of an antibody, and the other functional group may be linked to a first target site (e.g., a first amino acid residue) in the Fc region of another heavy chain of the antibody. For example, one functional group may be linked to K248 of a heavy chain, and the other functional group may be linked to K248 of another heavy chain of the antibody, but the present application is not limited thereto. In some embodiments, one functional group may be linked to a first target site (e.g., a first amino acid residue) of one heavy chain, and the other functional group may be linked to a second target site (a second amino acid residue) of the Fc region of another heavy chain of the antibody. For example, one functional group may be linked to K248 of one heavy chain, and the other functional group may be linked to K246 of another heavy chain of the antibody. In some embodiments, one functional group may be linked to a first target site (e.g., a first amino acid residue) in the Fc region of one heavy chain of an antibody, and the other functional group may be linked to a first target site (e.g., a first amino acid residue) in the Fc region of another heavy chain of the antibody. In this way, when the antibody comprising a functional group includes a plurality of functional groups, sites where the functional groups are linked are not particularly limited. An antibody comprising two functional groups may be referred to as a FAR 2 antibody comprising functional groups. Examples of FAR 2 antibodies comprising functional groups are shown in FIG. 5.

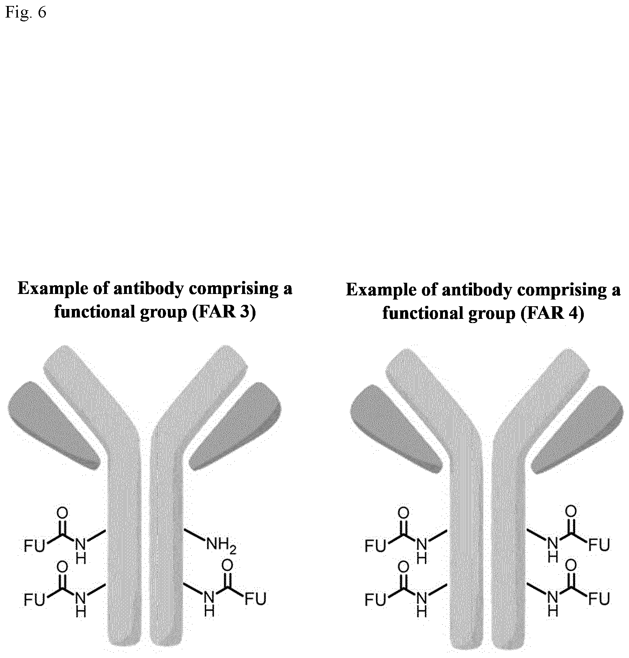

In some embodiments, the antibody comprising a functional group may include three functional groups. More specifically, the antibody comprising two functional groups may react with a compound for transferring a functional group, thereby preparing an antibody comprising three functional groups. The antibody comprising three functional groups may be referred to as a FAR 3 antibody comprising functional groups. Examples of FAR 3 antibodies comprising functional groups are shown in FIG. 6.

In some embodiments, the antibody comprising a functional group may include four functional groups. More specifically, the antibody comprising three functional groups may react with a compound for transferring a functional group, thereby preparing an antibody comprising four functional groups. The antibody comprising four functional groups may be referred to as a FAR 4 antibody comprising functional groups. Examples of FAR 4 antibodies comprising functional groups are shown in FIG. 6.

In some embodiments, the antibody comprising a functional group may include more than four functional groups.

In a specific embodiment, the antibody comprising a functional group may include one or two functional groups. In a specific embodiment, the antibody comprising a functional group may include two functional groups. In a specific embodiment, the antibody comprising a functional group may include two functional groups, wherein one of them is linked to a first target site (e.g., a first amino acid residue) of one heavy chain of the antibody, and the other functional group may be linked to a first target site (e.g., a second amino acid residue) of another heavy chain of the antibody.

### Product 2 produced by reaction of antibody and compound for transferring functional group - by-product comprising thiol group

### Overview of by-product comprising thiol group

By the reaction, contact, or mixing of an antibody and a compound for transferring a functional group, in addition to the antibody comprising a functional group, a thiol-containing by-product (i.e., by-product) is produced. The by-product includes a peptide unit (refer to Reaction Scheme 1) comprising a thiol group and FcBP. As described above, the by-product may be induced to the Fc region or designed target site of the antibody by FcBP. In addition, as described above, the thiol group may inhibit the reaction between the antibody and the compound for transferring a functional group at the target stie. Hereinafter, the structure of the by-product will be described in detail.

### Structure of by product

As described above, the by-product is produced by the rection between the antibody and the compound for transferring a functional group. Therefore, the by-product includes a thiol group derived from a thioester group and FcBP derived from FcBP of the compound for transferring a functional group. That is, the by-product includes a thiol group and FcBP.

In some embodiments, the by-product may be a compound having the structure of Formula 2:

[Formula 2] TU-PU

Here, TU is a thiol-containing unit. Here, the thiol group is derived from a thioester group of the compound for transferring a functional group. Since an acyl group linked to S of the thioester group of the compound for transferring a functional group is transferred to the antibody from the compound for transferring a functional group, the remaining part connected with the thiol group of the by-product is the same as a corresponding part (i.e., a part that is not an acyl group) of the compound for transferring a functional group.

Here, PU comprising FcBP is a peptide unit. Here, the structure of the peptide unit is the same as the structure of a peptide unit of the compound for transferring a functional group used in the reaction with the antibody.

The thiol-containing unit includes a thiol group. The structure of the thiol group may be expressed as the structure -SH or 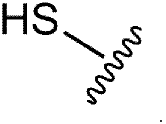 Here, waves represent that the thiol group is connected with another part of the by-product.

In some embodiments, TU may include a linker group, in addition to the thiol group. For example, the linker group may be designed to adjust the distance between the peptide unit comprising FcBP and the thioester group. For example, the length of the linker group may be 1 to 50 based on the number of atoms in the main chain. In a specific embodiment, the length of the linker group may be 1 to 10 based on the number of atoms in the main chain. The linker group may include one or more independently selected hetero atoms and one or more independently selected cyclic groups in the main chain, but the present application is not limited thereto.

In some embodiments, the thiol-containing unit (TU) may have the following structure:

X'-D²-

Here, X' is a thiol group. X' is connected with the peptide unit via D².

Here, D² may be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, substituted or unsubstituted arylene, or substituted or unsubstituted heteroarylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When D² is a bond, X' is directly connected with the peptide unit.

In a specific embodiment, D² may be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -NO₂, -OH, and -SH.

In some embodiments, the thiol-containing unit may have the following structure:

X'-D²¹-D²²-D²³-.

Here, X' is a thiol group. X' may be connected with the peptide unit via -D²¹-D²²-D²³-.

Here, each of D²¹, D²², and D²³ may independently be a bond, or substituted or unsubstituted C₁₋₂₀ alkylene, substituted or unsubstituted C₁₋₂₀ heteroalkylene, substituted or unsubstituted C₂₋₂₀ alkenylene, substituted or unsubstituted C₂₋₂₀ heteroalkenylene, substituted or unsubstituted C₂₋₂₀ alkynylene, substituted or unsubstituted C₂₋₂₀ heteroalkynylene, substituted or unsubstituted C₃₋₂₀ cycloalkylene, substituted or unsubstituted C₃₋₂₀ heterocycloalkylene, substituted or unsubstituted C₃₋₂₀ cycloalkenylene, substituted or unsubstituted C₃₋₂₀ heterocycloalkenylene, substituted or unsubstituted C₃₋₂₀ cycloalkynylene, substituted or unsubstituted C₃₋₂₀ heterocycloalkynylene, substituted or unsubstituted arylene, or substituted or unsubstituted heteroarylene. Here, "substituted" means that one or more hydrogen atoms are substituted with one or more substituents. For example, each substituent may be independently selected from -R, =O, =S, -NO₂, -CR₃, -NR₂, -OR, -SR, -C(=O)R, - C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. When all of D²¹, D²², and D²³ are bonds, X' is directly connected with the peptide unit.

In a specific embodiment, each of D²¹, D²², and D²³ may independently be a bond, or substituted or unsubstituted C₁₋₆ alkylene, substituted or unsubstituted C₁₋₆ heteroalkylene, substituted or unsubstituted C₂₋₆ alkenylene, substituted or unsubstituted C₂₋₆ heteroalkenylene, substituted or unsubstituted C₂₋₆ heteroalkylene, substituted or unsubstituted C₂₋₆ alkynylene, substituted or unsubstituted C₂₋₆ heteroalkynylene, substituted or unsubstituted C₃₋₈ cycloalkylene, substituted or unsubstituted C₃₋₈ heterocycloalkylene, substituted or unsubstituted C₃₋₈ cycloalkenylene, substituted or unsubstituted C₃₋₈ heterocycloalkenylene, substituted or unsubstituted C₃₋₈ cycloalkynylene, substituted or unsubstituted C₃₋₈ heterocycloalkynylene, substituted or unsubstituted arylene, or substituted or unsubstituted heteroarylene. Here, "substituted" means that one or more hydrogen atoms are substituted with one or more substituents. Here, each substituent may be independently selected from C₁₋₄ alkyl, -C(=O)H, -C(=O)CH₃, -C(=O)OH, -C(=O)NH₂, -NH₂, =O, =S, -NO₂, -OH, and -SH. The length of -D²¹-D²²-D²³- may be 0 to 10 based on the number atoms in the main chain.

As described above, the structure of the peptide unit is the same as the compound for transferring a functional group. The structure of the peptide unit was described in detail in the paragraph describing the compound for transferring a functional group.

### Method of increasing production yield of antibody comprising functional group using thiol-reactive additive

### Overview of method of increasing production yield of antibody comprising functional group using thiol-reactive additive

As described above, the by-product (i.e., a thiol-containing by-product) produced by the reaction of the antibody and the compound for transferring a functional group inhibits the reaction between remaining antibodies and the compound for transferring a functional group, or the reaction between the antibody comprising one functional group and the compound for transferring a functional group. This is because the by-product includes FcBP and a thiol group. The inventors of the present application developed a method of increasing the production yield of an antibody comprising a functional group by removing the thiol group of the by-product using a thiol-reactive additive (i.e., removing a thiol-containing by-product).

In some embodiments, when the thiol-reactive additive is used, compared to when the thiol-reactive additive is not used, the yield of the antibody comprising a functional group may increase by a factor of about 1.1, 1.2, 1.3, 1.4, 1.5, 1.6, 1.7, 1.8, 1.9, 2, 2.2, 2.4, 2.5, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, 10, 11, 12, 13, 14, 15, 16, 17, 18, 19, 20, or more. In some embodiments, the yield may be calculated from the amount of the antibody comprising a functional group produced after the reaction. In some embodiments, the yield may be calculated based on the amount of antibody initially involved in the reaction and the amount of antibody comprising a functional group produced after the reaction. In some embodiments, the yield may be calculated by analyzing a peak deduced from the result of chromatography analysis (e.g., peak area). In some embodiments, the yield of the antibody comprising a functional group may be measured based on the FAR 1 antibody comprising a functional group. In some embodiments, the yield of the antibody comprising a functional group may be measured based on the FAR 2 antibody comprising functional group (e.g., an antibody comprising two bio-orthogonal chemical functional groups). In some embodiments, the yield of the antibody comprising a functional group may be measured based on the antibody comprising one or more functional groups (including both FAR 1 and FAR 2). Preferably, the yield of the antibody comprising a functional group may be measured based on the antibody comprising a desired functional group (e.g., FAR 2). For example, the ratio of the FAR 2 antibody confirmed in the reacted or mixed composition increased from 20% (when not using the thiol-reactive additive) to 80% (when using a thiol-reactive additive), and the yield of the antibody comprising a functional group increased by a factor of 4.

In some embodiments, the yield may be measured a first time after the initiation of the reaction or mixing of the antibody and the compound for transferring a functional group. Here, the first time may be about 30 min, 1 hr, 2 hr, 3 hr, 4 hr, 5 hr, 6 hr, 7 hr, 8 hr, 9 hr, 10 hr, 11 hr, 12 hr, 13 hr, 14 hr, 15 hr, 16 hr, 17 hr, 18 hr, 19 hr, 20 hr, 21 hr, 22 hr, 23 hr, 24 hr, 26 hr, 28 hr, 30 hr, 32 hr, 34 hr, 36 hr, 38 hr, 40 hr, 44 hr, 48 hr, 3 days, 4 days, 5 days, or more, but the present application is not limited thereto. Those of ordinary skill in the art may design an appropriate time for preparing an antibody comprising a functional group, and when this time is too short, an appropriate amount of the antibody comprising a functional group cannot be obtained. In addition, when this time is too long, the production cost may increase, or a desired material may be denatured. That is, those of ordinary skill in the art may design an appropriate time as a reaction time, and the yield may be measured after the designed reaction time has elapsed (i.e., at the time of completing the designed reaction).

Mixing of the thiol-reactive additive, the antibody, and the compound for transferring a functional group to the antibody may be performed in various orders. For example, the thiol-reactive additive, the antibody, and the compound for transferring a functional group may be mixed at the same time. In another example, the thiol-reactive additive and the antibody may be mixed first, and then the compound for transferring a functional group may be mixed. In still another example, the thiol-reactive additive and the compound for transferring a functional group may be mixed first, and then the antibody may be mixed. In yet another example, the antibody and the compound for transferring a functional group may be mixed first, and then the thiol-reactive additive may be mixed. In a specific embodiment, the thiol-reactive additive may be present before or after the reaction of the antibody and the compound for transferring a functional group in a composition. That is, the thiol-reactive additive and the antibody may be mixed first, or the thiol-reactive additive and the compound for transferring a functional group may be mixed first. When the thiol-reactive additive is present in the reaction of the antibody and the compound for transferring a functional group, since the thiol-reactive additive may immediately remove the by-product, it is preferable that the thiol-reactive additive be previously present in the composition.

In some embodiments, the method of increasing the yield of the antibody comprising a functional group may include: mixing a thiol-reactive additive, a compound for transferring a functional group, and an antibody.

In some embodiments, the method of increasing the yield of the antibody comprising a functional group may include: mixing a thiol-reactive additive and an antibody; and adding a compound for transferring a functional group to the mixture.

In some embodiments, the method of increasing the yield of an antibody comprising a functional group may include: mixing a thiol-reactive additive and a compound for transferring a functional group; and adding an antibody to the mixture.

In some embodiments, the thiol-reactive additive may be used at about 1, 2, 3, 4, 5, 6, 7, 8, 0, 10, 12, 14, 16, 18, 20, 22, 24, 26, 28, 30, 34, 38, 42, 46, 50, 54, 58, 62, 66, 70, 75, 80, 85, 90, 95, 100, 110, 120, 140, 160, 180, 200, 240, 280, 320, 360, 400, 450, 500, 550, 600, 650, 700, 800, 900, 1000, 1100, 1200, 1300, 1400, 1500, 1600, 1700, 1800, 1900, 2000, 2200, 2400, 2600, 2800, 3000, 3500, 4000, 4500, 5000, 6000, 7000, 8000, 9000, 10000, 20000, 30000, 40000, 50000, 100000 equiv., or more than the above-mentioned values, or an equivalent within the range comprising two values selected from the above-mentioned values based on the antibody.

In some embodiments, the reaction in which the thiol-reactive additive is involved may be performed at pH of about 4, 4.2, 4.4, 4.6, 4.8, 5, 5.2, 5.4, 5.6, 5.8, 6, 6.2, 6.4, 6.6, 6.8, 7, 7.1, 7.2, 7.3, 7.4, 7.5, 7.6, 7.7, 7.8, 8, 8.2, 8.4, 8.6, 8.8, 9, or 10, or at pH in the range determined by two values selected from the above-mentioned values. In a specific embodiment, the reaction in which the thiol-reactive additive participates may be performed at pH of about 6.8, 7, 7.2, 7.4, 7.6, 7.8, or 8, or at pH in the range determined by two values selected from the above-mentioned values.

In some embodiments, the time for reaction between the compound for transferring a functional group and the antibody may be about 30 min, 1 hr, 2 hr, 3 hr, 4 hr, 5 hr, 6 hr, 7 hr, 8 hr, 9 hr, 10 hr, 11 hr, 12 hr, 13 hr, 14 hr, 15 hr, 16 hr, 17 hr, 18 hr, 19 hr, 20 hr, 21 hr, 22 hr, 23 hr, 24 hr, 26 hr, 28 hr, 30 hr, 32 hr, 34 hr, 36 hr, 38 hr, 40 hr, 44 hr, 48 hr, 3 days, 4 days, 5 days or more, or within the range generated by two values selected from the above-mentioned values, but the present application is not limited thereto.

The thiol-reactive additive is a compound having reactivity with a thiol group. The thiol-reactive additive will be described in detail below.

### Thiol-reactive additive

The thiol-reactive additive is a compound having reactivity with a thiol group. In some embodiments, the thiol-reactive additive must at least satisfy the following conditions: having reactivity with a thiol group. That is, the thiol-reactive additive includes a thiol-reactive group having reactivity with a thiol group. In some embodiments, the thiol-reactive additive must satisfy the following two conditions: having reactivity with a thiol group, and having little or no reactivity with the antibody. This is because when the thiol-reactive additive has reactivity with the antibody or has high reactivity with the antibody, it causes changes in the structure and/or function of the antibody. Such unintended changes in the structure and/or function of the antibody are likely to cause unpredictable side effects in the use of an antibody-based drug.

In some embodiments, the thiol-reactive additive may have one or more characteristics: having reactivity with a thiol group, having little or no reactivity with an antibody, having little or no reactivity with a thioester group, and having little or no reactivity with FcBP.

In some embodiments, the thiol-reactive additive is represented as "Ad." The thiol-reactive additive reacts with a thiol group of the by-product to cap the thiol group of the by-product. That is, the thiol-reactive additive reacts with a thiol-containing by-product to remove the thiol-containing by-product. Here, the thiol-containing by-product may be referred to as a first by-product, and a new by-product produced by reacting the thiol-reactive additive with the thiol-containing by-product may be referred to as a second by-product. The second by-product no longer includes a thiol group.

An exemplary reaction scheme for the reaction between the thiol-reactive additive and the thiol-containing by-product is illustrated below.

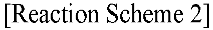

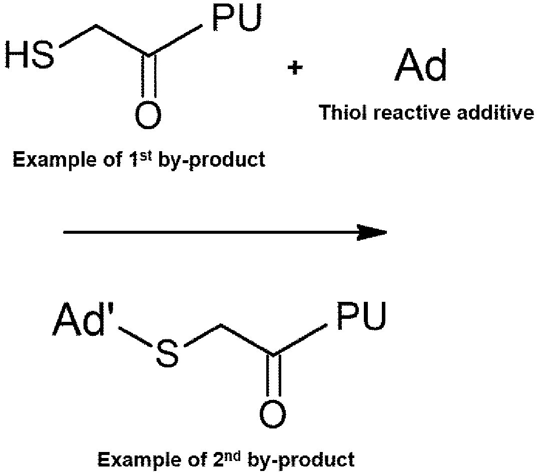

As illustrated in Reaction Scheme 2, the second by-product is produced by reacting the thiol-reactive additive and the first by-product. The second by-product is a form in which H of the thiol group is substituted with Ad' derived from the thiol-reactive additive. In Reaction Scheme 2, Ad' is the structure derived from the thiol-reactive additive, and the group formed by the reaction between the thiol-reactive additive and the first by-product. For example, the second by-product may have the same structure as the first by-product except that the thiol group is capped.

In some embodiments, the second by-product may be a compound having the structure of Formula 3 below.

[Formula 3] TU'-PU

Here, PU is a peptide unit, which was described in detail in the related paragraph.

Here, TU' is a capped thiol-containing unit, and includes the structure of Ad'-S-, which is a capped thiol. The remaining structure except Ad'-S- (if present) corresponds to the part excluding the thiol group in TU of the first by-product, and is as described for TU.

In some embodiments, the thiol-reactive additive has reactivity with a thiol group. In some embodiments, the thiol-reactive additive may include a thiol-reactive group having reactivity with a thiol group. In some embodiments, the thiol-reactive additive may be a small molecule compound. In some embodiments, the thiol-reactive additive may be a peptide or protein. In some embodiments, the molecular weight of the thiol-reactive additive may be 30 g/mol, 40 g/mol, 50 g/mol, 60 g/mol, 70 g/mol, 80 g/mol, 90 g/mol, 100 g/mol, 110 g/mol, 120 g/mol, 130 g/mol, 140 g/mol, 150 g/mol, 160 g/mol, 170 g/mol, 180 g/mol, 190 g/mol, 200 g/mol, 220 g/mol, 240 g/mol, 260 g/mol, 280 g/mol, 300 g/mol, 350 g/mol, 400 g/mol, 450 g/mol, 500 g/mol, 600 g/mol, 700 g/mol, 800 g/mol, 900 g/mol, 1000 g/mol, 1100 g/mol, 1200 g/mol, 1300 g/mol, 1400 g/mol, 1500 g/mol, 1600 g/mol, 1700 g/mol, 1800, g/mol 1900 g/mol, 2000 g/mol, 2500 g/mol, 3000 g/mol, 3500 g/mol, 4000 g/mol, 4500 g/mol, 5000 g/mol, 6000 g/mol, 7000 g/mol, 8000 g/mol, 9000 g/mol, or 10000 g/mol or less, or within the range determined by two values selected from the above-mentioned values. In a specific embodiment, the molecular weight of the thiol-reactive additive may be 50 g/mol, 60 g/mol, 70 g/mol, 80 g/mol, 90 g/mol, 100 g/mol, 110 g/mol, 120 g/mol, 130 g/mol, 140 g/mol, 150 g/mol, 160 g/mol, 170 g/mol, 180 g/mol, 190 g/mol, 200 g/mol, 220 g/mol, 240 g/mol, 260 g/mol, 280 g/mol, 300 g/mol, 350 g/mol, 400 g/mol, 450 g/mol, 500 g/mol, 600 g/mol, 700 g/mol, 800 g/mol, 900 g/mol, or 1000 g/mol or less, or within the range determined by two values selected from the above-mentioned values. In some embodiments, the thiol-reactive additive may be referred to as a thiol scavenger.

In some embodiments, the thiol-reactive additive may be a compound that undergoes an electrophilic substitution reaction. In some embodiments, the thiol-reactive additive may be an electrophilic substitution reaction molecule. For example, the thiol-reactive additive may include a group that undergoes an electrophilic substitution reaction.

In some embodiments, the thiol-reactive additive may be a compound that undergoes a nucleophilic substitution reaction. In some embodiments, the thiol-reactive additive may be a nucleophilic substitution reaction molecule. For example, the thiol-reactive additive may include a group that undergoes a nucleophilic substitution reaction.

In some embodiments, the thiol-reactive additive may be a compound that undergoes an SN2 reaction. In some embodiments, the thiol-reactive additive may be an SN2 reaction molecule. For example, the thiol-reactive additive may include a group that undergoes an SN2 reaction.

In some embodiments, the thiol-reactive additive may be a compound that undergoes a thiol-disulfide exchange reaction. In some embodiments, the thiol-reactive additive may be a thiol-disulfide exchange molecule. For example, the thiol-reactive additive may include a disulfide group.

In some embodiments, the thiol-reactive additive may be a compound that undergoes a thiolate disulfide interchange reaction. In some embodiments, the thiol-reactive additive may be a thiolate disulfide interchange reaction molecule. For example, the thiol-reactive additive may include a disulfide group.

In some embodiments, the thiol-reactive additive may be a compound that produces a compound having absorbance to light of a specific wavelength after the reaction with a thiol. The specific wavelength may be, for example, 350 to 500 nm, 380 to 450 nm, or 400 to 420 nm. For example, the following 5,5'-dithiobis-(2-nitrobenzoic acid) (DTNB) is converted into 2-nitro-5-thiobenzoate (TNB), which is ionized to TNB²⁻ having the maximum absorbance to light of 412 nm, by the reaction with a thiol-containing compound. TNB²⁻ appears yellow, and may be quantified by measuring absorbance in visible light at 412 nm. The extinction coefficient of TNB²⁻ is known as 14,150 M⁻¹ cm⁻¹ in a diluted solution, and as 13,700 M⁻¹ cm⁻¹ in a solution with a high salt concentration. The reaction scheme for the reaction between the DTNB and the thiol-containing compound is illustrated below.

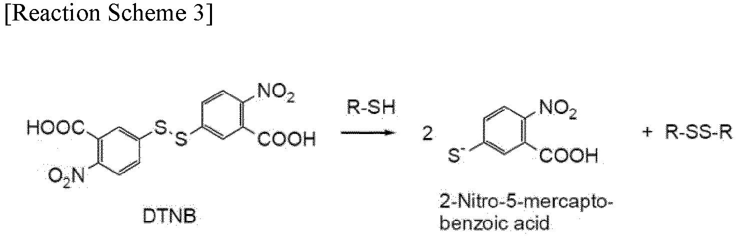

Hereinafter, exemplary embodiments that can be used as a thiol-reactive additive will be described.

### Exemplary compound (1) as thiol-reactive additive

In some embodiments, a thiol-reactive additive may be diphenyl disulfide or a derivative thereof. The diphenyl disulfide is a compound having the structure of Formula 4 below:

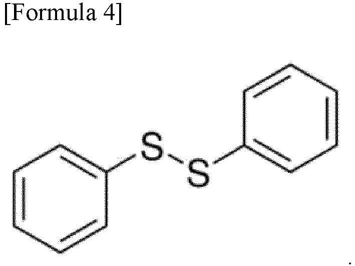

A derivative of the diphenyl disulfide may be, for example, any one of a dipyridyldisulfide such as 2,2'-dipyridyldisulfide or 4,4'-dipyridyl disulfide, 5,5'-dithiobis-(2-nitrobenzoic acid) (DTNB), also referred to as Ellman's reagent, 2,2'-dithiodibenzoic acid, 4-aminophenyl disulfide, 2-aminophenyl disulfide, bis(4-chlorophenyl) disulfide), bis(2-nitrophenyl) disulfide, p-tolyl disulfide, bis(4-nitrophenyl) disulfide, bis(2-nitrophenyl) disulfide), bis(4-methoxyphenyl) disulfide, and bis(2-methoxyphenyl) disulfide, or a derivative thereof, but the present application is not limited thereto.

In some embodiments, the thiol-reactive additive may be a compound having the structure of Formula 5 below:

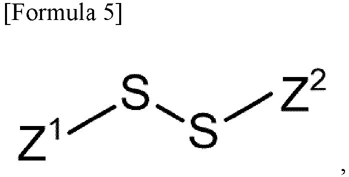

Here, Z¹ is substituted or unsubstituted C₅₋₁₀ aryl, or substituted or unsubstituted C₅₋₁₀ heteroaryl. Here, substituted C₅₋₁₀ aryl or substituted C₅₋₁₀ heteroaryl has one or more substituents, and here, each substituent may be independently selected from -R, =O, =S, -NO₂, -CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂. Here, R may be each independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. In a specific embodiment, each substituent may be independently selected from C₁₋₄ alkyl, -C(=O)H, -C(=O)CH₃, -C(=O)OH, -C(=O)NH₂, -NH₂, =O, =S, -NO₂, -SO₃H, -OSO₃H, -OPO₃H₂, -OH, and -SH. In a specific embodiment, substituted aryl or substituted heteroaryl may include one or two substituents. In a specific embodiment, Z¹ may be substituted or unsubstituted phenyl, or substituted or unsubstituted pyridyl.

Here, Z² is substituted or unsubstituted C₅₋₁₀ aryl, or substituted or unsubstituted C₅₋₁₀ heteroaryl. Here, substituted C₅₋₁₀ aryl or substituted C₅₋₁₀ heteroaryl has one or more substituents, wherein each substituent may be independently selected from -R, =O, =S, -NO₂, - CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂, and each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. In a specific embodiment, each substituent may be independently selected from C₁₋₄ alkyl, -C(=O)H, - C(=O)CH₃, -C(=O)OH, -C(=O)NH₂, -NH₂, =O, =S, -NO₂, -SO₃H, -OSO₃H, -OPO₃H₂, -OH, and -SH. In a specific embodiment, substituted aryl or substituted heteroaryl may include one or two substituents. In a specific embodiment, Z² may be substituted or unsubstituted phenyl, or substituted or unsubstituted pyridyl.

In some embodiments, the thiol-reactive additive may be a compound having the structure of Formula 6:

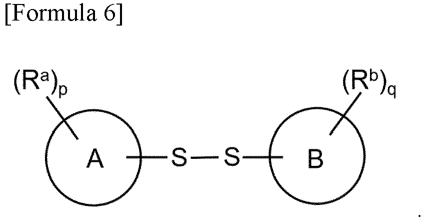

Here, A is 5-membered aromatic ring or 6-membered aromatic ring, and here, A may optionally include one or more hetero atoms on the ring. Here, each hetero atom may be independently selected from N, O, and S. In a specific embodiment, A may include a benzene ring, a pyridine ring, a pyrimidine ring, a pyrrole ring, a furan ring, a thiophene ring, or an imidazole ring.

Here, p may be an integer of 0 to 4. In a specific embodiment, p may be an integer of 0 to 2.

Here, each R^{a} may independently be -R, =O, =S, -NO₂, -CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. In a specific embodiment, each R^{a} may be independently selected from C₁₋₄ alkyl, -C(=O)H, -C(=O)CH₃, -C(=O)OH, - C(=O)NH₂, -NH₂, =O, =S, -NO₂, -SO₃H, -OSO₃H, -OPO₃H₂, -OH, and -SH. In some embodiments, one or more R^{a} may be hydrophilic groups. Each hydrophilic group may be independently selected from -OH, -CHO, -COOH, -CONH₂, -NH₂, a PEG moiety, and a sugar moiety. In some embodiments, one or more R^{a} may be electron withdrawing groups (EWGs). Each EWG may be independently selected from halogen, a carbonyl group (e.g., -CHO), a sulfonyl group (e.g., -SO₃H), -COOH, -CN, -NO₂, and a haloalkyl group (e.g., -CF₃).

Here, B is a 5-membered aromatic ring or a 6-membered aromatic ring, wherein B may selectively include one or more hetero atoms on the ring. Here, each hetero atom may be independently selected from N, O, and S. In a specific embodiment, B may include a benzene ring, a pyridine ring, a pyrimidine ring, a pyrrole ring, a furan ring, a thiophene ring, or an imidazole ring.

Here, q may be an integer of 0 to 4. In a specific embodiment, p may be an integer of 0 to 2.

Here, each R^{b} may be independently selected from -R, =O, =S, -NO₂, -CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. In a specific embodiment, each R^{b} may be independently selected from C₁₋₄ alkyl, -C(=O)H, -C(=O)CH₃, - C(=O)OH, -C(=O)NH₂, -NH₂, =O, =S, -NO₂, -SO₃H, -OSO₃H, -OPO₃H₂, -OH, and -SH. In some embodiments, one or more R^{b} may be hydrophilic groups. Each hydrophilic group may be independently selected from -OH, -CHO, -COOH, -CONH₂, -NH₂, a PEG moiety, and a sugar moiety. In some embodiments, one or more R^{b} may be electron withdrawing groups (EWGs). Each EWG may be independently selected from halogen, a carbonyl group (e.g., - CHO), a sulfonyl group (e.g., -SO₃H), -COOH, -CN, -NO₂, and a haloalkyl group (e.g., -CF₃).

In some embodiments, a compound having the structure of Formula 6 may be referred to as diphenyl disulfide or a derivative thereof.

In some embodiments, the thiol-reactive additive may be a compound having the structure of Formula 7:

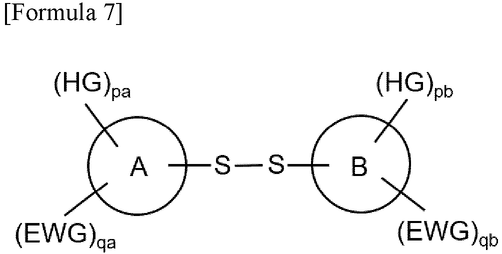

Here, EWG may be an electron withdrawing group, and each EWG may be independently selected from halogen, a carbonyl group (e.g., -CHO), a sulfonyl group (e.g., - SO₃H), -COOH, -CN, -NO₂, and a haloalkyl group (e.g., -CF₃).

Here, HG may be a hydrophilic group, and each hydrophilic group may be independently selected from -OH, -CHO, -COOH, -CONH₂, -NH₂, a PEG moiety, and a sugar moiety.

Here, pa may be an integer of 0 to 2.

Here, qa may be an integer of 0 to 2.

Here, pb may be an integer of 0 to 2.

Here, qb may be an integer of 0 to 2.

In some embodiments, the compound having the structure of Formula 7 may be referred to as diphenyl disulfide or a derivative thereof.

### Exemplary compound (2) as thiol-reactive additive

In some embodiments, the thiol-reactive additive may be maleimide or a derivative thereof. Maleimide is a compound having the structure of Formula 8 below:

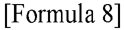

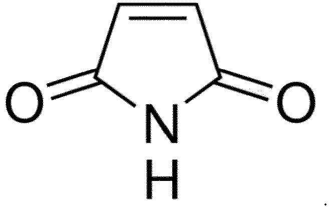

A derivative of maleimide may be, for example, any one of N-methylmaleimide, N-ethylmaleimide, N-phenylmaleimide, N-benzylmaleimide, 3-maleimidopropionic acid, N-tert-butylmaleimide, and N-cyclohexylmaleimide, or a derivative thereof.

In some embodiments, the thiol-reactive additive may be a compound having the structure of Formula 9 below:

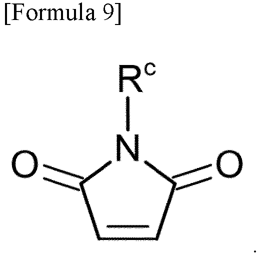

Here, R^{c} may be selected from -R, =O, =S, -NO₂, -CR₃, -CH₂CR₃, -NR₂, -OR, -SR, - SO₂R, -OSO₂R, -PO₂R₂, -OPOR₂, -C(=O)R, -C(=O)CR₃, -C(=O)OR, and -C(=O)NR₂, wherein each R may be independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH. In a specific embodiment, R^{c} may be selected from C₁₋₄ alkyl, C₃₋₈ cycloalkyl, C₃₋₈ heterocycloalkyl, C₃₋₈ cycloalkenyl, C₃₋₈ heterocycloalkenyl, -C(=O)H, -C(=O)CH₃, -CH₂CH₂C(=O)OH, -C(=O)OH, -C(=O)NH₂, -NH₂, -NO₂, -SO₃H, -OSO₃H, -OPO₃H₂, -OH, and -SH.

### Exemplary compound (3) as thiol-reactive additive

Hereinafter, compounds that can be used as a thiol-reactive additive are exemplified. In a specific embodiment, the thiol-reactive additive may be a compound having any one structure of the following formulas: 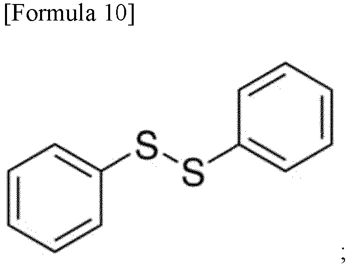 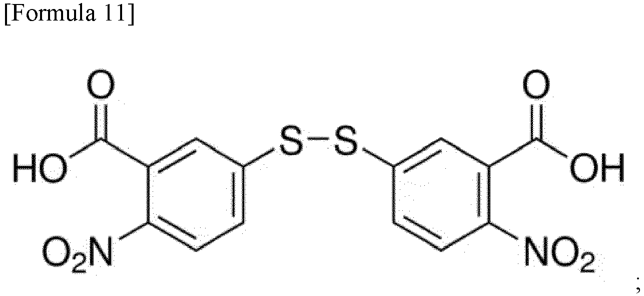 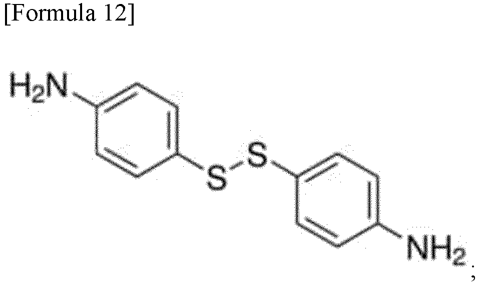 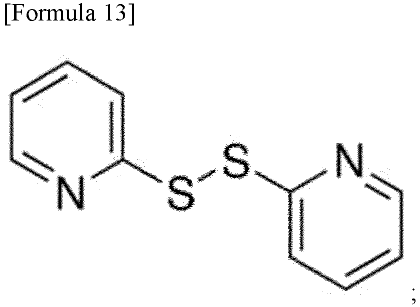 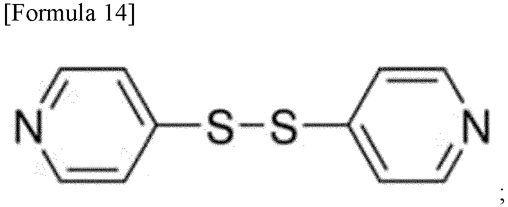 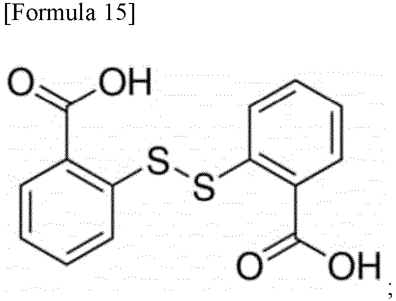 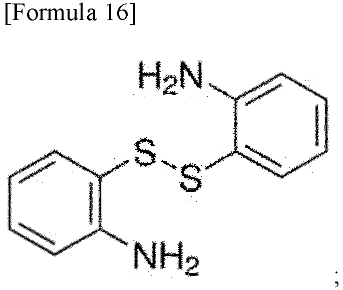 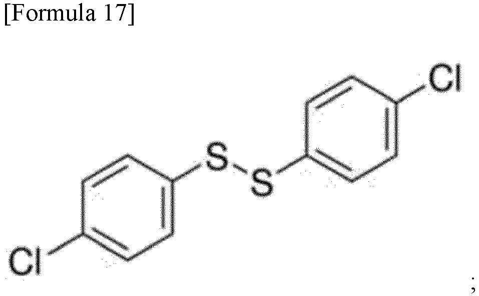 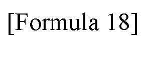 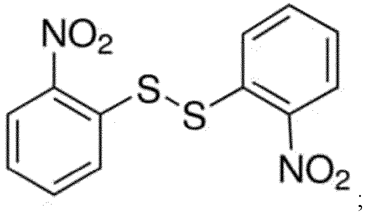 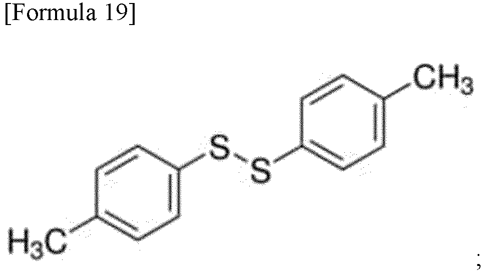 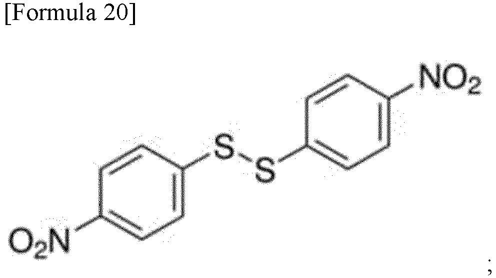 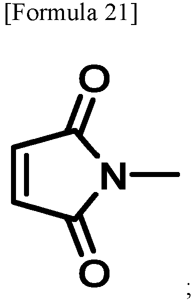 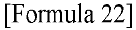 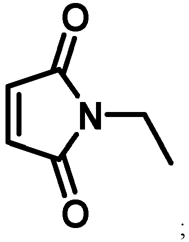 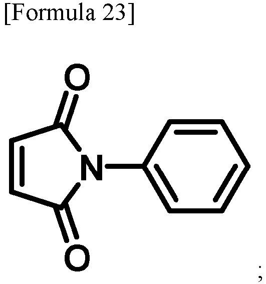 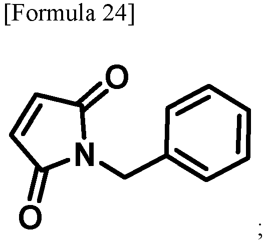 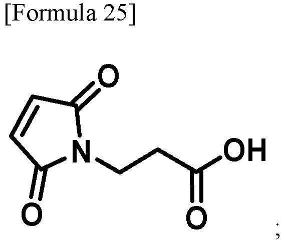 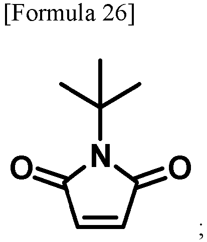 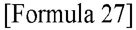 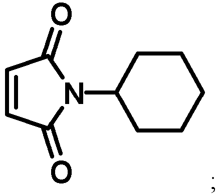 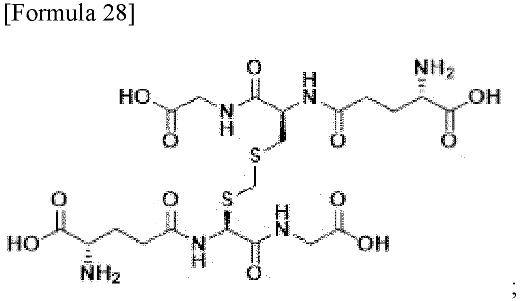 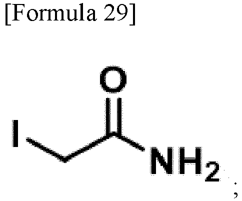 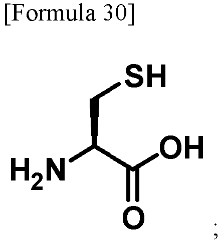 and

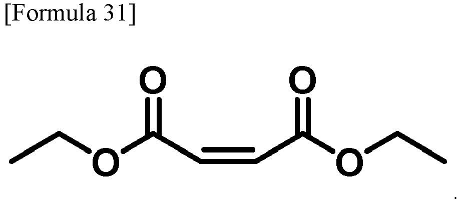

### Kit including thiol-reactive additive

In some embodiments of the present application, a kit including a thiol-reactive additive may be provided. The kit including a thiol-reactive additive may be used to increase the production yield of an antibody comprising a functional group. In some embodiments, a kit including a thiol-reactive additive for increasing the production yield of an antibody comprising a functional group may be provided. Further, a kit, which includes a thiol-reactive additive, for preparing an antibody comprising a functional group may be provided.

In some embodiments, the kit including a thiol-reactive additive may include a thiol-reactive additive, and a compound for transferring a functional group to an antibody. In some embodiments, the kit including a thiol-reactive additive may include a thiol-reactive additive, a precursor of a compound for transferring a functional group, and FcBP. The precursor of the compound for transferring a functional group is described in detail in the document [PCT International Application No. PCT/KR2020/003282, Publication No. WO2020/184944], and is referred to as a compound that is used to prepare the compound for transferring a functional group through a reaction with FcBP. In some embodiments, the kit including a thiol-reactive additive may further include an antibody. In a specific embodiment, the kit including a thiol-reactive additive may include a thiol-reactive additive, a compound for transferring a functional group, and an antibody.

### Composition including thiol-reactive additive

In some embodiments of the present application, a composition including a thiol-reactive additive may be provided. The composition including a thiol-reactive additive may be used to increase the production yield of an antibody comprising a functional group. In some embodiments, a composition that includes a thiol-reactive additive used to increase the production yield of an antibody comprising a functional group may be provided. Further, a composition, which includes a thiol-reactive additive, for preparing an antibody comprising a functional group may be provided.

In some embodiments, the composition including a thiol-reactive additive may include a thiol-reactive additive, and a composition for transferring a functional group to an antibody. In some embodiments, the composition including a thiol-reactive additive may include a thiol-reactive additive, a precursor of a compound for transferring a functional group, and FcBP. The precursor of a compound for transferring a functional group is described in detail in the document [PCT International Application No. PCT/KR2020/003282, Publication No. WO2020/184944], and is referred to as a compound used to prepare a compound for transferring a functional group through a reaction with FcBP. In some embodiments, the composition including a thiol-reactive additive may further include an antibody. In a specific embodiment, the composition including a thiol-reactive additive may include a thiol-reactive additive, a compound for transferring a functional group, and an antibody.

### Exemplary embodiment of method of increasing production yield of antibody comprising functional group using thiol-reactive additive

Hereinafter, an exemplary embodiment of a method of increasing the production yield of an antibody comprising a functional group using a thiol-reactive additive of the present application is provided. In some embodiments, the method of increasing a production yield of an antibody comprising a functional group using a thiol-reactive additive may be referred to as a method of preparing an antibody comprising a functional group.

Throughout the specification, terms used herein may be interpreted to include plural concepts even when they are described in a singular form. That is, it will be recognized that terms described in a singular form may be interpreted to include plural concepts as needed.

A01. A method of increasing a production yield of an antibody comprising a functional group using a thiol-reactive additive, wherein the method includes:
(a) contacting an antibody with a compound for transferring a functional group in a reaction composition,
   wherein the compound for transferring a functional group includes a thioester group, a functional group (FG), and an Fc-binding peptide (FcBP),
   the thioester group of the compound for transferring a functional group reacts with a nucleophile of the antibody, and
   as a result of the reaction between the thioester group and the nucleophile of the antibody,
   an antibody comprising a functional group, and a by-product comprising a thiol group and FcBP are produced, wherein the thiol group of the by-product is produced by the reaction of the thioester group of the compound for transferring a functional group with the nucleophile of the antibody, and
   the by-product has a reaction inhibitory effect of inhibiting a reaction of an antibody, which has not yet reacted, with a compound for transferring a functional group, which has not yet reacted, in the reaction composition; and
(b) contacting the by-product with the thiol-reactive additive in a reaction composition to remove the reaction inhibitory effect of the by-product,
   wherein the thiol-reactive additive is a compound having reactivity with a thiol group,
   the thiol-reactive additive reacts with the thiol group of the by-product, and
   as a result of the reaction between the thiol-reactive additive and the thiol group, the thiol group of the by-product is capped.

A02. The method of A01, wherein
the by-product is removed by capping the thiol group of the by-product.

A03. The method of any one of A01 and A02, wherein
the reaction inhibition effect of the by-product is removed by capping the thiol group of the by-product.

A04. The method of any one of A01 to A03, wherein
the thiol-reactive additive is a compound having little or no reactivity with the antibody.

A05. The method of any one of A01 to A04, wherein
the thiol-reactive additive is a small molecule compound.

A06. The method of any one of A01 to A05, wherein
the thiol-reactive additive has a molecular weight of 50 g/mol or more and 2000 g/mol or less.

A07. The method of any one of A01 to A06, wherein
the thiol-reactive additive has a molecular weight of 100 g/mol or more and 1000 g/mol or less.

A08. The method of any one of A01 to A07, wherein
the thiol-reactive additive is a compound that undergoes a thiol-disulfide exchange reaction.

A09. The method of any one of A01 to A08, wherein
the thiol-reactive additive is a compound that undergoes a thiolate disulfide interchange reaction.

A10. The method of any one of A01 to A09, wherein
the thiol-reactive additive includes a disulfide group.

A11. The method of any one of A01 to A10, wherein
the thiol-reactive additive is diphenyl disulfide or a derivative thereof.

A12. The method of any one of A01 to A11, wherein
the thiol-reactive additive is diphenyl disulfide, dipyridyldisulfide, 5,5'-dithiobis-(2-nitrobenzoic acid) (DTNB), 2,2'-dithiodibenzoic acid, 4-aminophenyl disulfide, 2-aminophenyl disulfide, bis(4-chlorophenyl disulfide), bis(2-nitrophenyl disulfide), p-tolyl disulfide, bis(4-nitrophenyl) disulfide, bis(2-nitrophenyl) disulfide, bis(4-methoxyphenyl) disulfide, or bis(2-methoxyphenyl) disulfide.

A13. The method of any one of A01 to A11, wherein
the thiol-reactive additive is 5,5'-dithiobis-(2-nitrobenzoic acid) (DTNB).

A14. The method of any one of A01 to A11, wherein
the thiol-reactive additive is a compound having the structure of Formula 6: 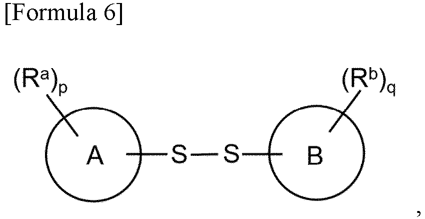 where
A is a 5- or 6-membered aromatic ring, wherein A optionally includes one or more heteroatoms in the ring, and the heteroatoms are each independently selected from N, O, and S,
p is an integer of 0 to 4,
each R^{a} is independently selected from -R, -NO₂, -CR₃, -NR₂, -OR, -SR, -SO₂R, -OSO₂R, -PO₂R₂, -OPOR₂, -C(=O)R, -C(=O)CR₃, -C(=O)OR, and -C(=O)NR₂,
B is a 5- or 6-membered aromatic ring, wherein B optionally includes one or more heteroatoms in the ring, and the heteroatoms are each independently selected from N, O, and S,
q is an integer of 0 to 4,
each R^{b} is independently selected from -R, -NO₂, -CR₃, -NR₂, -OR, -SR, -SO₂R, - OSO₂R, -PO₂R₂, -OPOR₂, -C(=O)R, -C(=O)CR₃, -C(=O)OR, and -C(=O)NR₂, and
each R is independently selected from H, halogen, C₁₋₆ alkyl, C₃₋₁₀ cycloalkyl, C₃₋₁₀ heterocycloalkyl, aryl, heteroaryl, -OH, -NH₂, =O, =S, and -SH.

A15. The method of A14, wherein
at least one of R^{a} is an electron withdrawing group or a hydrophilic group.

A16. The method of any one of A14 and A15, wherein
at least one of R^{b} is an electron withdrawing group or a hydrophilic group.

A17. The method of any one of A01 to A07, wherein
the thiol-reactive additive is maleimide or a derivative thereof.

A18. The method of A17, wherein
the thiol-reactive additive is maleimide, N-methylmaleimide, N-ethylmaleimide, N-phenylmaleimide, N-benzylmaleimide, 3-maleimidopropionic acid, N-tert-butylmaleimide, or N-cyclohexylmaleimide.

A19. The method of A17, wherein
the thiol-reactive additive is tert-butylmaleimide.

A20. The method of A17, wherein
the thiol-reactive additive is a compound having the structure of Formula 9: 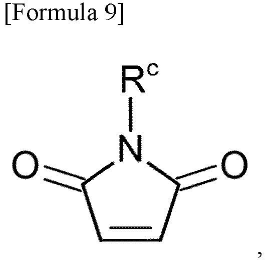 where R^{c} is selected from -R, =O, =S, -NO₂, -CR₃, -CH₂CR₃, -NR₂, -OR, -SR, -SO₂R, - OSO₂R, -PO₂R₂, -OPOR₂, -C(=O)R, -C(=O)CR₃, -C(=O)OR, and -C(=O)NR₂, wherein each R is independently selected from H, halogen, C₁₋₆alkyl, C₃₋₁₀ cycloalkyl, C₃₋₁₀ heterocycloalkyl, aryl, heteroaryl, -OH, -NH₂, and -SH.

A21. The method of any one of A01 to A07, wherein
the thiol-reactive additive is a compound having any one of the structures of Formulas 10 to 31.

A22. The method of any one of A01 to A21, wherein
the compound for transferring a functional group has the structure of Formula 1:

[Formula 1] FU-RU-PU,

Where
FU is a functional unit comprising a functional group (FG),
RU is a reactive unit comprising a thioester group, and
PU is a peptide unit comprising an Fc-binding peptide (FcBP).

A23. The method of any one of A01 to A22, wherein
the Fc-binding peptide has a length of 8 to 20 amino acid residues.

A24. The method of any one of A01 to A23, wherein
the Fc-binding peptide includes any one amino acid sequence selected from SEQ ID NOs: 2 to 80, or an amino acid sequence having 80% or more sequence identity therewith.

A25. The method of any one of A01 to A24, wherein
the functional group includes one or more bio-orthogonal functional groups.

A26. The method of A25, wherein
the bio-orthogonal functional group is independently a group participating in any one bio-orthogonal reaction selected from Staudinger ligation, copper-free azide-alkyne cycloaddition, such as copper-catalyzed azide-alkyne cycloaddition (CuAAC) or strainpromoted azide-alkyne cycloaddition (SPAAC), tetrazine ligation, tetrazole ligation, oxime ligation, and isocyanide click reaction.

A27. The method of A25, wherein
the bio-orthogonal functional group is independently any one selected from azide, terminal alkyne, cycloalkyne, tetrazine, norbornene, cycloalkene, tetrazole, oxime, and isocyanide groups.

A28. The method of any one of A01 to A24, wherein
the functional group includes one or more active moieties.

A29. The method of A28, wherein
the active moiety is independently selected from a drug, an imaging moiety, a ligand for a radioactive isotope, an affinity material, a stabilization material, a vitamin, and a hydrophilic moiety.

A30. The method of any one of A01 to A29, wherein
the reaction inhibition effect of the by-product is exhibited because the Fc-binding peptide included in the by-product guides the by-product to a reaction site on the antibody, where the reaction between the compound for transferring the functional group and the antibody occurs, and the thiol group included in the by-product interacts with the antibody at the reaction site.

A31. The method of any one of A01 to A29, wherein
the production yield of the antibody comprising a functional group in this method is 1.5 times or more compared to that when the thiol-reactive additive is not used.

A32. The method of A31, wherein
the production yield of the antibody comprising a functional group is measured based on an antibody comprising two functional groups.

A33. The method of any one of A01 to A32, wherein
(a) the contacting of the antibody with the compound for transferring a functional group, and (b) the contacting of the by-product with the thiol-reactive additive in this method are performed by mixing the thiol-reactive additive, the antibody, and the compound for transferring the functional group.

A34. The method of A33, wherein
in the mixing of the thiol-reactive additive, the antibody, and the compound for transferring a functional group,
the thiol-reactive additive and the antibody are first mixed and then mixed with the compound for transferring a functional group.

A35. The method of A33, wherein
in the mixing of the thiol-reactive additive, the antibody, and the compound for transferring a functional group,
the thiol-reactive additive and the compound for transferring a functional group are first mixed and then mixed with the antibody.

Hereinafter, the invention provided by this application will be described in further detail with reference to experimental examples or examples. These experimental examples are merely provided to more fully describe this application, and it will be obvious to those of ordinary skill in the art that the scope of the contents disclosed in the specification is not limited to the above experimental examples.

### Experimental Examples

### Materials and instruments

### Compounds and antibodies

Compounds were purchased from commercial providers and used without purification. 5-Exo-norbornene carboxylic acid, (NH₄)₂SO₄), *N*-Boc-ethylenediamine, triisopropylsilane (TIS), and 1,2-ethanedithiol (EDT) were purchased from Sigma-Aldrich (St. Louis, MO, USA). *N*-methylmaleimide, A-ethylmaleimide, *N*-cyclohexylmaleimide, *N*-phenylmaleimide, *N-*benzylmaleimide, *N*-*tert*-butylmaleimide, and 3-maleimidopropionic acid were purchased from Tokyo Chemical Industry (Tokyo, Japan). *tert*-Butyl thioglycolate was purchased from Angene Chemical (Telangana, India). 4-Methyltetrazine NHS ester was purchased from Broadpharm Inc.; San Diego, CA, USA). Fmoc-PEGs-OH was purchased from Quanta Biodesign (Plain City, OH, USA). DM1-SMCC was purchased from eNovation Chemicals LLC (Bridgewater Township, NJ, USA). DBCO-C6-NHS was purchased from Lumiprobe (Hong Kong). All amino acids, rink amide resins, and coupling reagents were purchased from Aapptec (Louisville, KY, USA), GL Biochem Ltd. (Shanghai, China), and Combi-Blocks (San Diego, CA, USA). Solvents were used without distillation. Trifluoroacetic acid (TFA), *N,N-*diisopropylethylamine (DIPEA), ammonium hydroxide (ammonia solution), diethylamine, Na₂HPO₄, *N,N*-dimethylformamide (DMF), dichloromethane (DCM), methanol (MeOH), hexane (Hex), ethyl acetate (EA), and diethylether were obtained from Daejung (Siheung, Korea). High performance liquid chromatography (HPLC)-grade acetonitrile (ACN), isopropanol, and water were purchased from Thermo Fisher Scientific (Waltham, MA, USA). Trastuzumab was purchased from Roche (Basel, Switzerland).

### Characterization and purification of small molecule and peptide

Thin-layer chromatography was performed on a pre-coated silica gel F₂₅₄ plate (Merck, Billerica, MA, USA), and observed under UV (254 nm) or after staining with KMnO₄ or ninhydrin solutions. All synthesized compounds were purified by Combi-flash purification using a pre-filled silica gel cartridge. All synthetic peptides were purified by preparative-HPLC (Waters, Milford, MA, USA; with XBridge^{™} prep C18, 5 µm, 4.6 × 250 mm column).

### Deglycosylation of antibody for intact mass spectrometry

Two microliters of samples (75 mg/mL) were combined with 8 µL of 6M urea buffer and 50 mM Tris-HCl (pH 8.0) to a total reaction volume of 99 µL, and 500 units (1 µL) of PNGaseF (glycerol-free) were added. The samples were gently mixed, and cultured using the Rapid Enzyme Digestion System (ASTA, HST REDS, Suwon, Korea) at 400 W for 90 minutes at 37 °C.

### Digestion in solution for analysis of antibody-linker binding site

An ADC sample (100 µg) was digested in a solution. Briefly, the sample was denatured with 8M urea, reduced with 10 mM DTT (30 min at 37 °C), and then alkylated with 25 mM iodoacetamide (at room temperature for 20 minutes). Chymotrypsin or IdeS enzyme was added to 1 µg chymotrypsin or IdeS:50 µg protein (i.e., 1:50 ratio), and the sample was cultured overnight at 37 °C. After incubation, the digest was desalted using a C18 SPE cartridge.

### Instruments

Centrifugation was performed with a Fleta 4 centrifuge (Hanil, Korea).

All peptides and tetrazine-containing payloads were characterized using HPLC (Waters, XBridge^{®}, C18, 4.6 × 250 mm, 5 µm).

For HPLC, the HPLC Alliance system (a 2996 PAD detector and a 2695 separations module) manufactured by Waters was used.

As a mass spectrometer used in analysis of small molecule materials such as all peptides and payloads, LC/MS in which Quattro Premier XE (Waters) and Acquity (Waters) LC system were combined was used.

All antibodies including an ADC precursor and ADC were characterized using HIC-HPLC (Thermo Fisher Scientific, MAbPac^{™}, HIC-butyl, 4.6 × 100 mm, 5 µm) and sizeexclusion chromatography (SEC)-HPLC (Thermo Fisher Scientific, MAbPac^{™}, SEC-1, 300 Å 4 × 300 mm, 5 µm).

The mass spectra of small molecules and peptides were obtained using electrospray ionization (ESI) and time-of-flight (TOF) analyzers, such as Quattro Premier XE mass spectrophotometer (Waters) and COSMOSIL COSMOCORE 2.6 C₁₈ column (2.6 µm, 2.1 × 50 mm, Nacalai Tesque, Kyoto, Japan).

The masses of all antibodies were obtained using a TOF analyzer (MALDI-TOF) using matrix-associated laser desorption ionization and sinapinic acid (SA) as matrices. MALDI-TOF mass spectra were obtained using Ultraflex III mass spectrometer (Bruker Daltonics, Billerica, MA, USA) equipped with a 337 nm pulsed nitrogen laser operated in positive and linear modes.

Ultra-performance liquid chromatography (UPLC) and MS were used in mass spectrometry. The UPLC-MS analysis of antibodies was performed using Orbitrap Fusion Tribrid (Thermo Fisher Scientific) combined with Waters Acquity UPLC and Waters BEH300 C4 column (1.7 µm, 2.1 × 100 mm).

To analyze a binding site, the extracted peptide was analyzed using a quadrupole Orbitrap mass spectrometer (Q-Exactive, Thermo Fisher Scientific) combined with the EasynLC system (Thermo Fisher Scientific). The peptide was reconstituted in 0.1% formic acid and separated on an analytical column (C18, 2 µm particle size, 50 µm id × 15 cm length, Thermo Fisher Scientific).

### Parameters of instrument

### Characterization of peptides and tetrazine-containing payloads using C18-HPLC

C18-HPLC was performed at a flow rate of 1 mL/min. All compounds were analyzed under the same illusion conditions [initial 80% mobile phase A (0.1% TFA in H₂O) for 1 min followed by a 20-80% gradient of mobile phase B (0.075% TFA in ACN) in A for 15 min]. The chromatograms of all peptides were acquired at 280 nm, and the chromatograms of the payloads were acquired at 254 nm.

### Characterization of antibodies using HIC-HPLC

HIC-HPLC was performed at a flow rate of 1 mL/min. All antibodies were analyzed under the same conditions [initial 100% mobile phase A (1.5 M (NH₄)₂SO₄, 50 mM Na₂HPO₄ at pH 7.0, 5% isopropanol) for 1 min followed by a 0-100% gradient of mobile phase B (50 mM sodium phosphate at pH 7.0, 20% isopropanol) in A for 15 min]. All chromatograms were acquired at 280 nm.

### Characterization of antibodies using SEC-HPLC

SEC-HPLC was performed at a flow rate of 0.2 mL/min. All antibodies were analyzed under the same conditions [isocratic mobile phase D (1× PBS) for 20 min]. All chromatograms were acquired at 280 nm.

### Mass spectrometry

**ESI-MS:** A column temperature was set to 25 °C, and a flow rate of 0.3 mL/min was used. A compound was injected into a column at a volume of 5 µL. Mobile phase A consisted of water containing 0.05% TFA, whereas mobile phase B consisted of acetonitrile with 0.05% TFA. Initial gradient conditions were 20% B for 20 minutes; then, B gradually increased to 80% for 3.5 minutes, and subsequently, the gradient decreased from 80% B to 20% B for 0.1 minutes.

**UPLC-MS:** A column temperature was set to 65 °C, and a flow rate of 0.4 mL/min was used. 10 µL of each antibody was injected into a column. Mobile phase A consisted of 0.1% formic acid and water, and mobile phase B consisted of 0.1% formic acid and acetonitrile. The gradient started at 5% B for 0.5 minutes, and then gradually increased to 80% for 10 minutes. Afterward, the gradient increased from 80% B to 100% B for 0.1 minutes. The column was washed at 100% B for 2 minutes, and equilibrated at 2% B for 3 minutes.

**Orbitrap fusion Tribrid/data analysis:** The intact mass of the antibody was investigated by an Orbitrap Fusion Tribrid mass spectrometer using +3.2 spray voltage. The MS system was operated in an intact protein mode using an ion transfer tube at 275 °C and a vaporizer at 350 °C. A full scan was acquired from m/z 600 to 6000 at a resolution of 15.000. Intact mass spectra were analyzed using the intact protein workflow in BioPharma Finder 3.1 integrated software, and time-solved deconvolution was performed using the ReSpect algorithm in combination with the sliding window integration. Deconvolution spectra were annotated by inputting an amino acid sequence having variable modifications corresponding to a total of 16 disulfide bonds and 6 possible glycan combinations (G0/G0F, G0F/G0F, G0F/G1F, G1F/G1F, G1F/G2F, and G2F/G2F).

**MALDI-TOF:** Antibodies were dissolved in H₂O at a concentration of 1 mg/mL, and the saturated solution of SA in 0.1% TFA in the ratio of ACN:H₂O (1: 1) was used as a matrix. Antibodies (2 µL) were spotted onto a crushed steel 384 target plate, mixed with 2 µL of the matrix solution, and vacuum-dried. FlexControl software (version 3.0) was used to acquire spectra under the following instrument conditions: a mass range of m/z 2,000-300,000, a bias suppressed up to m/z 5,000, shots accumulated repeatedly over 1000 times, a laser frequency of 100 Hz, a voltage of 25 kV in ion source I, a voltage of 23 kV in ion source II, and 9 kV on a lens.

### 1. Selection of thiol-reactive additives

The inventors of the present application selected a compound having selective reactivity toward a thiol group as a thiol-reactive additive. Examples of the selected thiol-reactive additives are shown in Table 1.

**Table 1. Selected compounds that can be used as thiol-reactive additive**

| **Chemical name** | **Chemical structure** | **Formula No.** |
|---|---|---|
| Diphenyl disulfide | 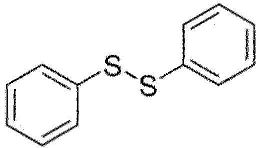 | 10 |
| 5,5'-Dithiobis-(2-nitrobenzoic acid) | 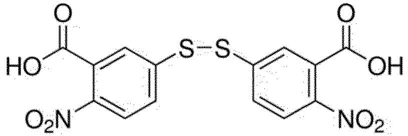 | 11 |
| 4-Aminophenyl disulfide | 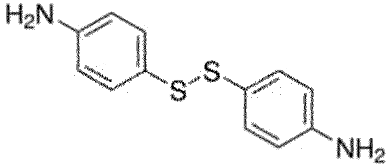 | 12 |
| 2,2'-Dipyridyldisulfide | 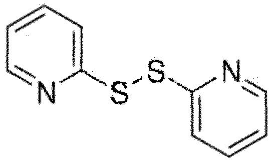 | 13 |
| 4,4'-Dipyridyl disulfide | 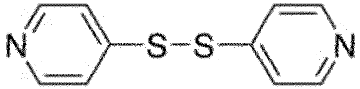 | 14 |
| 2,2'-Dithiodibenzoic acid | 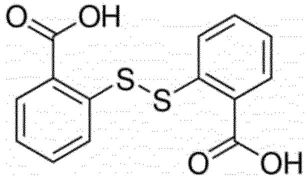 | 15 |
| 2-Aminophenyl disulfide | 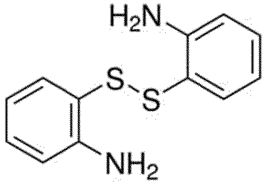 | 16 |
| Bis(4-chlorophenyl disulfide) | 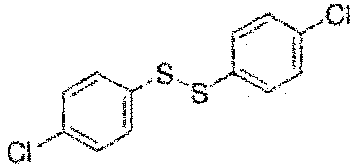 | 17 |
| Bis(2-nitrophenyl) disulfide | 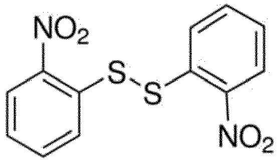 | 18 |
| Di-p-tolyl Disulfide | 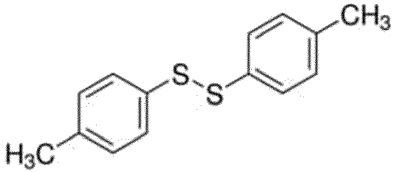 | 19 |
| Bis(4-nitrophenyl) disulfide | 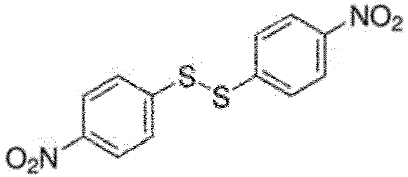 | 20 |
| N-Methylmaleimide | 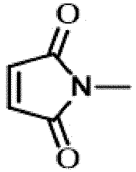 | 21 |
| N-Ethylmaleimide | 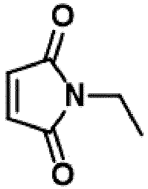 | 22 |
| N-Phenylmaleimide | 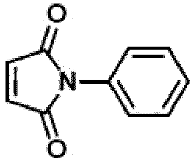 | 23 |
| N-Benzylmaleimide | 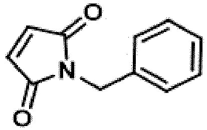 | 24 |
| 3 -Maleimidopropionic acid | 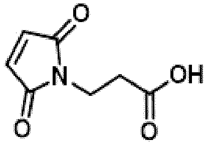 | 25 |
| N-Tert-butylmaleimide | 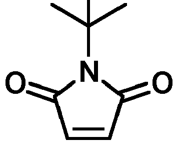 | 26 |
| N-Cyclohexylmaleimide | 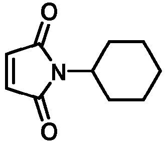 | 27 |
| Glutathione disulfide | 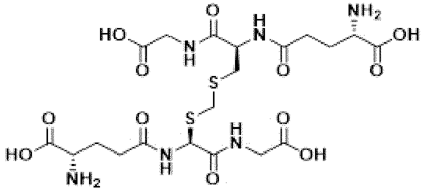 | 28 |
| Iodoacetamide | 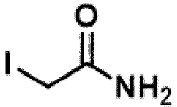 | 29 |
| L-Cysteine | 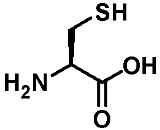 | 30 |
| 1,4-Dimethyl but-2-enedioate | 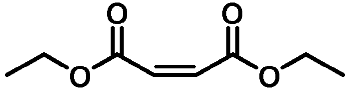 | 31 |

### 2. Preparation of Fc-binding peptides

### 2. 1. Preparation of L6Dap FcBP, L6Dab FcBP, L6Orn FcBP, and L6Lys FcBP

The inventors of the present application confirmed the effect of a thiol-reactive additive by adding a thiol-reactive additive in the process of preparing an antibody comprising a functional group or an antibody-drug conjugate. In order to prepare an antibody comprising a functional group, a reaction between the compound for transferring a functional group to an antibody and the antibody is required. The thiol-reactive additive is used in this reaction. Hereinafter, a method of preparing a compound for transferring a functional group to an antibody will be described. The compound for transferring a functional group to an antibody includes a part of an Fc-binding peptide. The method of preparing an Fc binding peptide will be preferentially described. An Fc-binding peptide and a synthetic method thereof are described in detail in the documents [PCT International Application No. PCT/KR2020/003282 (Publication No. WO2020/184944 A1); and Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759], and the entire contents of each are incorporated herein by reference.

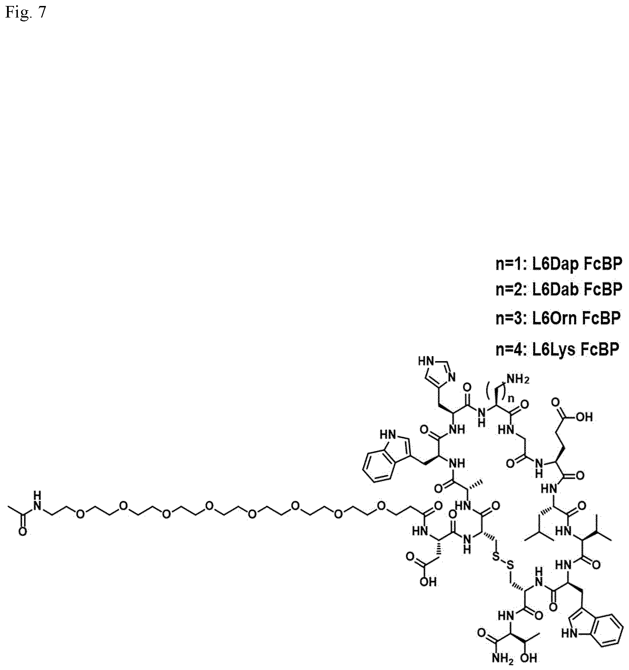

The inventors of the present application prepared L6Dap FcBP, L6Dab FcBP, L6Orn FcBP, and L6Lys FcBP using a solid synthesis method. The structure of the synthesized FcBP is illustrated in FIG. 7 (Dap: diaminopropionic acid; Dab: diaminobutyric acid; Orn: ornithine; Lys: lysine). Information on the synthesized L6Dap FcBP, L6Dab FcBP, L6Orn FcBP, and L6Lys FcBP is as follows:
- Sequence: Ac-PEG8-DCAWH-(Dap, Dab, Orn, or Lys)-GELVWCT-amide (All L-form amino acid)
- N-term: acetylation (= Ac)
- C-term: Amidation (=amide)
- Cys-Cys disulfide oxidation

L6Dap FcBP, L6Dab FcBP, L6Orn FcBP, and L6Lys FcBP were synthesized with reference to PCT International Application No. PCT/KR2020/003282 (Publication No. WO2020/184944 A1). Specifically, the section of "Synthesis and structural identification of 1.2. site-specific Fc interactome (SSFI)" in "Experimental Example" of International Application No. PCT/KR2020/003282 was referred to. To help those of ordinary skill in the art, the method of synthesizing FcBP will be described below.

### List and introduction order of Fmoc amino acids used herein

Fmoc-L-Thr(tBu)-OH, Fmoc-Cys(Trt)-OH, Fmoc-L-Trp(Boc)-OH, Fmoc-L-Val-OH, Fmoc-L-Leu-OH, Fmoc-L-Glu(OtBu)-OH, Fmoc-Gly-OH, Fmoc-X(Boc)-OH, Fmoc-L-His(Trt)-OH, Fmoc-L-Trp(Boc)-OH Fmoc-Ala-OH, Fmoc-Cys(Trt)-OH, Fmoc-Asp(tBu)-OH.

Here, Fmoc-X(Boc)-OH is Fmoc-Dap(Boc)-OH, Fmoc-Dab(Boc)-OH, Fmoc-Orn(Boc)-OH, or Fmoc-Lys(Boc)-OH.

### Introduction of amino acids

The amount of a reagent used in the following process was 0.25 mmole. 0.5 g of a rink amide resin (0.48 mmole/g, PeptideInternational, USA) was put into a synthesis reactor, 1 mmole each of an Fmoc-amino acid block was weighed, and prepared from the C-terminus to the N-terminus in the order of peptide amino acid sequences.

The reaction of attaching an activated residue to a clear amide resin by activating an Fmoc-amino acid was sequentially performed with the C-terminal amino acid.

Fmoc removal was performed in DMF containing 20% piperidine, and the activation and introduction of residues were performed by mixing amino acids prepared according to the sequence with 2 mL of a 0.5 M HOBt-containing DMF solution, 2 mL of a 0.5 M HBTU-containing DMF solution, and 174 µL of DIPEA for 5 minutes, and then pouring the resulting mixture into a reactor containing a resin to mix it for 2 hours.

The confirmation of the introduction reaction was performed by the Kaiser test method, and when it was confirmed that there was no reaction, the introduction reaction was repeated one more time, or capping was performed with a 20% Ac2O-contianing DMF solution. The resin was sufficiently washed with DMF and DCM before moving on to the next step in each introduction reaction and Fmoc removal process. This process was repeated until the target peptide sequence was completed.

### Introduction of H-PEG8-OH

After the introduction of amino acids was done, to introduce H-PEG8-OH to the N-terminus, 1 mL of 0.5 M Fmoc-N-amido-dPEG8-acid in a DMF solution, 1 mL of a 0.5 M HBTU-containing DMF solution, 1 mL of a 0.5 M HOBt-containing DMF solution, and 87 µL of DIPEA were mixed for 5 minutes, and then poured into a reactor containing a reaction resin to mix it for 2 hours.

The progression of the reaction was checked using the Kaiser test method, and it was determined that unreacted amine remained, the reaction time was extended by 1 to 3 hours or the reaction solution was removed, and then the above reaction process was repeated. The removal of the N-terminal Fmoc protecting group was performed using DMF containing 20% piperidine, and then the peptide-attached resin was dried and weighed.

250 mg of the resin to which the peptide which had been prepared in this process was attached was stirred with 2 mL of a mixture of TFA, TIS, water, and EDT (94:1.0:2.5:2.5) at room temperature for 120 minutes to cleave the peptide from the resin. The cleaved mixture was filtered, the filtrate was concentrated to about half with nitrogen gas, and then ether was poured to precipitate the peptide. The precipitated peptide was washed three or more times with ether and dried with nitrogen gas. The dried precipitate was dissolved in water containing 0.1% TFA-30% CAN, stirred for 6 hours, and then concentrated.

The concentrate was dissolved in a 0.01 M ammonium acetate buffer (pH 6.5) solution containing 5%-DMSO-20%-CAN at a concentration of 0.1 mg/mL, and stirred for 3 days in an air-exposed state. The procession of the disulfide bond formation reaction was observed by HPLC, and when it was judged to be no longer processing, the reaction solution was lyophilized to obtain a peptide precipitate.

### Purification and analysis

The peptide precipitate obtained by lyophilization in (c) was isolated under prep-LC primary purification conditions, further purified under prep-LC secondary conditions and then lyophilized. The Prep-LC purification conditions were as follows:
- Conditions for Prep-LC primary purification
   Name of instrument: Water 2525pump, Waters 2487 UV detector; Column: Waters, XBridge Prep C18 5µm OBD 19x250 mm; Mobile phase A/B: 0.1% TFA-20% ACN/0.1% TFA in 80% ACN; Gradient: 0 min -> 30 min, B 0% -> B 100%; flow rate: 17 mL/min; Detection wavelength: UV 280 nm.
- Conditions for Prep-LC secondary purification

Name of instrument: Water 2525pump, Waters 2487 UV detector; Column: Waters, XBridge Prep C18 5µm OBD 19x250mm; Mobile phase A/B: 0.1% TFA-30% ACN/0.1% TFA in 70% ACN; Gradient: 0 min -> 30 min, B 0% -> B 100%; Flow rate: 15 mL/min; Detection wavelength UV 280 nm.

It was confirmed by HPLC for analysis that each obtained peptide had a purity of 90% or more. Specifically, for HPLC, a Waters HPLC Alliance system, a 2996 PAD detector, and a 2695 separations module were employed, and analysis was performed under conditions including initial 80% mobile phase A (0.1% TFA in H₂O) for 1 min followed by a 20 to 80% gradient of mobile phase B (0.075% TFA in ACN) in A for 15 min at a flow rate of 1 mL/min. The molecular weight of the synthesized peptide was confirmed using a mass spectrometer (Bruker, Ultraflextreme) (measurement matrix: a-cyano-4-hydroxycinnamic acid (CHCA) & 2,5-dihydroxybenzoic acid (DHB)).

Mass spectrometry results are as follows:
- L6Dap FcBP: 1968.21 g/mol (Calculated molecular weight); 984.71 g/mol (M+2H)²⁺ (Measured molecular weight)
- L6Dab FcBP: 1982.24 g/mol (Calculated molecular weight); 992.33 g/mol (M+2H)²⁺ (Measured molecular weight)
- L6Orn FcBP: 1996.26 g/mol (Calculated molecular weight); 999.13 g/mol (M+2H)²⁺ (Measured molecular weight)
- L6Lys FcBP: 2010.29 g/mol (Calculated molecular weight); 2011.89 g/mol (M+H)⁺ (Measured molecular weight)

### 2.2. Structures of L6Dap FcBP, L6Dab FcBP, L6Orn FcBP, and L6Lys FcBP

The structures of the L6Dap FcBP, L6Dab FcBP, L6Orn FcBP, and L6Lys FcBP synthesized by the above-described process are shown in FIG. 7.

### 3. Preparation of compound for transferring functional group to antibody

### 3.1. Preparation of compound for preparing functional group to antibody (1): FcBP-N3 (Compound 7)

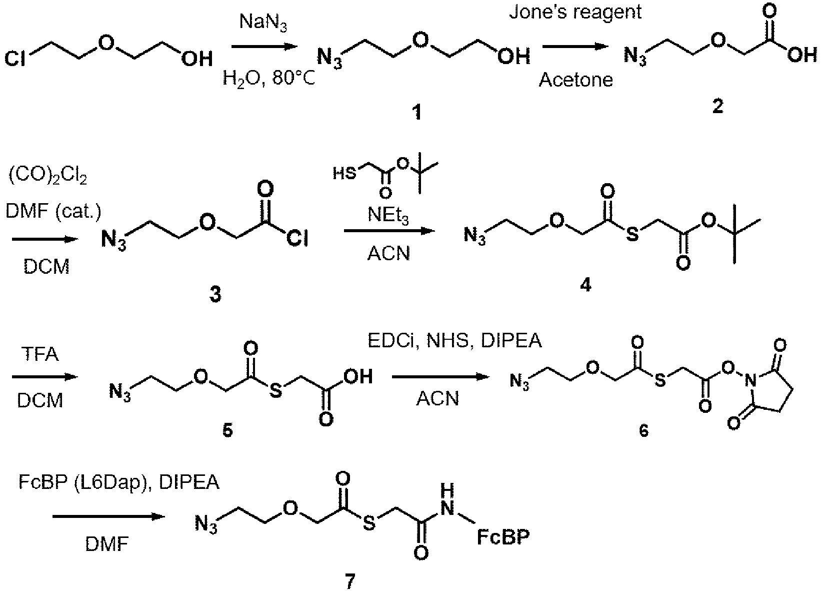

### Preparation of Compound 1

2 mL (18.94 mmol, 1.0 equiv.) of 2-(chloroethoxy)ethanol was dissolved in 12 mL of water, 3.08 g (47.35 mmol, 2.5 equiv.) of NaN₃ was added, and the resulting mixture was stirred at 80 °C for 16 hours. 20 mL of 5% NaOH was slowly added dropwise to terminate the reaction, and the resulting product was extracted in an organic layer using a diethyl ether solvent. Afterward, the organic layer was dried with Na₂SO₄ and a product was obtained through concentration under reduced pressure (2.47 g, 99%). The obtained product was used in the next reaction without an additional purification process.

### Preparation of Compound 2

2.47 g (18.84 mmol, 1.0 equiv.) of Compound 1 was dissolved in 50 mL of an acetone solvent, 75.36 mL (75.36 mmol, 4.0 equiv.) of Jones reagent at 0 °C was slowly added dropwise, and then the resulting mixture was stirred for 3 hours. Afterward, the resulting mixture was stirred at room temperature for 1 hour, and a saturated sodium bicarbonate solution was slowly added dropwise at 0 °C to terminate the reaction. The resulting product was extracted in an organic layer using an ethylacetate solvent. Afterward, the organic layer was dried with Na₂SO₄ and the resulting product was obtained through concentration under reduced pressure (2.69 g, 98%). The obtained product was used in a subsequent reaction without an additional purification process.

### Preparation of Compound 3

2.69 g (18.58 mmol, 1.0 equiv.) of Compound 2 was dissolved in 50 mL of dichloromethane solvent, and 2.43 mL (27.86 mmol, 1.5 equiv.) of oxalyl chloride and one droplet of dimethylformamide were slowly added dropwise at 0 °C. The reaction solvent was stirred for 18 hours, and then oxalyl chloride was removed through concentration under reduced pressure. To completely remove oxalyl chloride, concentration under reduced pressure was performed three more times by adding a dichloromethane solvent, and the residue was obtained by vacuum-drying. The obtained product (2.42 g, 80%) was used in a subsequent process without an additional purification process.

### Preparation of Compound 4

1.047 g (8.1 mmol, 1.5 equiv.) of 1,1-dimethylethyl 2-mercaptoacetate was dissolved in 30 mL of a dichloromethane solvent, mixed with DIPEA, and then stirred for 5 minutes. 0.8 g (5.40 mmol, 1.0 equiv.) of Compound 3 dissolved in the dichloromethane solvent was stirred at room temperature for 16 hours. After the termination of the reaction, the resulting mixture was mixed with a 10% citric acid solution to extract the product in an organic layer. Afterward, the organic layer was dried using Na₂SO₄ and concentrated under reduced pressure, and then Compound 4 (0.73 g, 99%) was successfully purified through column chromatography.

### Preparation of Compound 5

0.73 g (2.66 mmol, 1.0 equiv.) of Compound 4 was dissolved in 10 mL of dichloromethane solvent, and 10 mL of TFA was slowly added dropwise at 0 °C. The reaction solution was stirred for 3 hours, and then TFA was removed through concentration under reduced pressure. To completely remove TFA, dichloromethane solvent was added and concentrated under reduced pressure two more times by adding a dichloromethane solvent, and then the residue was obtained by vacuum drying. The obtained product was used in a subsequent reaction without an additional purification process.

### Preparation of Compound 6

0.05 g (0.228 mmol, 1.0 equiv.) of Compound 5 was dissolved in 3 mL of an acetone solvent, 0.03 g (0.255 mmol, 1.12 equiv.) of NHS and a catalytic amount of DMAP were added, and then the resulting mixture was stirred at room temperature for 5 minutes. Afterward, an EDCi solution dissolved in a dichloromethane solvent was slowly added dropwise to the reaction solution at 0 °C and stirred for 18 hours. After the reaction, the solvent was removed through concentration under reduced pressure, and then Compound 6 was successfully obtained by column chromatography.

### Preparation of Compound 7

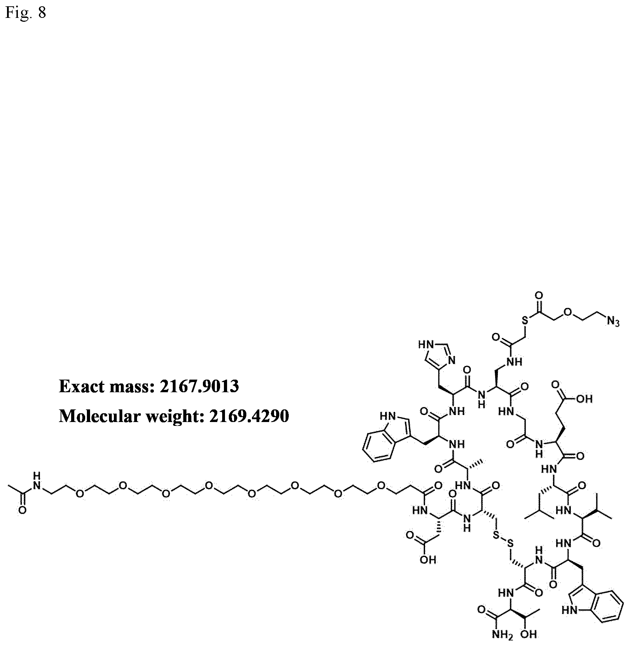

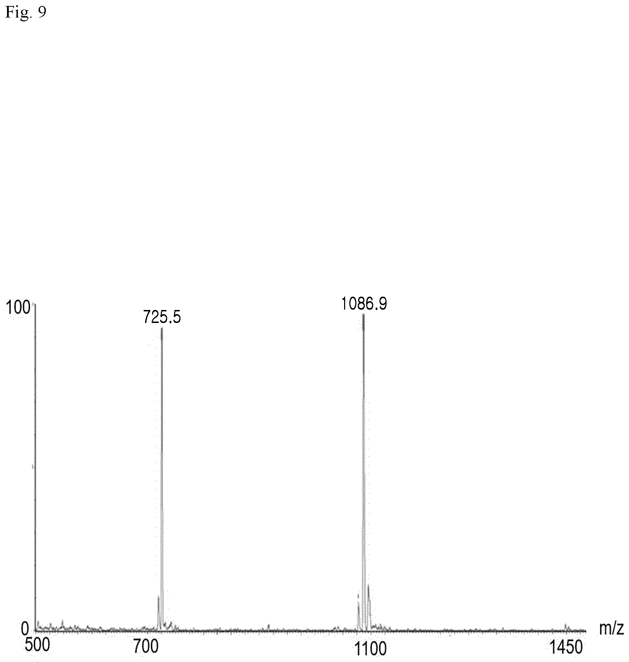

5 mL of a dimethylformamide solution was added to Compound 6 (0.09 mmol, 1.2 equiv.), FcBP (L6Dap FcBP) (0.08 mmol, 1.0 equiv.) peptide composite that had been synthesized by a solid phase peptide synthesis (SPPS) method was added, and diisopropylethylamine (0.32 mmol, 4.2 equiv.) was added to allow a reaction at room temperature for 1 hour. After the reaction, Prep-HPLC (C18) was purified and lyophilized to obtain Compound 7 (105 mg, 63.2%). The detailed structure of Compound 7 (FcBP-N₃) is shown in FIG. 8. The mass of the obtained Compound 7 was analyzed using a mass spectrometer (using analysis instrument combined with Waters, Quatro Premier XE and Acquity Waters LC system), and the mass spectrometry data of the synthesized Compound 7 is shown in FIG. 9 ([M+2H]2⁺: 1086.9; [M+3H]³⁺: 725.5).

### 3.2. Preparation of compound for transferring functional group to antibody (2): FcBP-C6PEG4 (Compound 17)

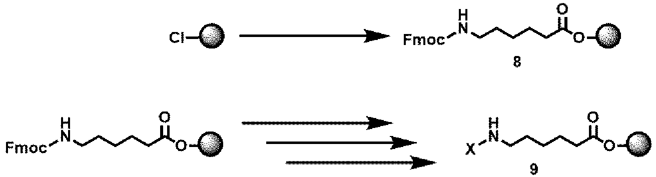

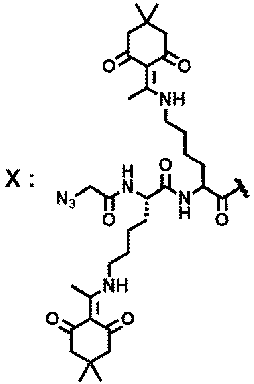

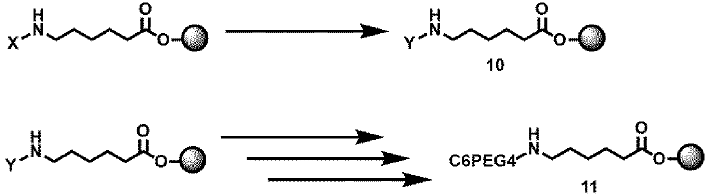

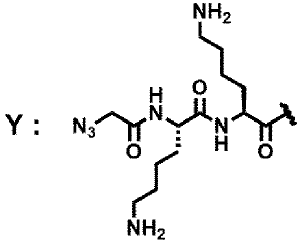

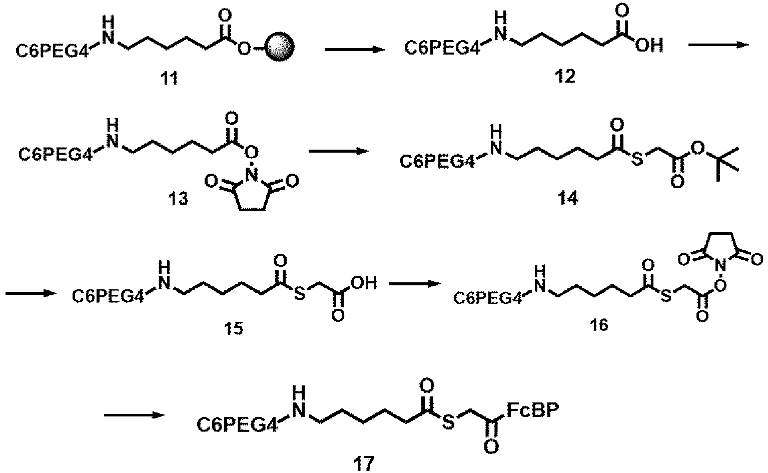

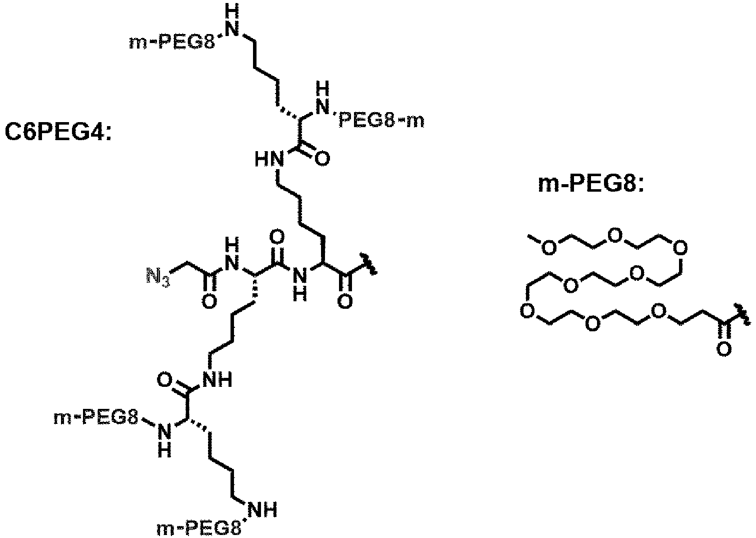

### Preparation of Compound 8

Synthesis was performed using a solid phase synthesis method. 50 mL of dichloromethane was added to 2 g of 2-chlorotrityl chloride resin (1.4 mmol/g) and stirred for 30 minutes. After stirring, a solvent was removed, and for a subsequent reaction, fluorenylmethoxycarbonyl-L-6-aminohexaoic acid (Fmoc-L-6-aminohexanoic acid) (5.6 mmol, 2.0 equiv.) and N,N'-diisopropylethylamine (11.2 mmol, 4.0 equiv.) were mixed with 40 mL of dichloromethane and then put into a resin. After stirring at room temperature for 2 hours, the reaction mixture was removed. Afterward, the resin was washed by adding 50 mL each of dichloromethane and dimethylformamide three times, thereby preparing Compound 8.

### Preparation of Compound 9

40 mL of a 20% piperidine-containing dimethylformamide (volume ratio) solution was added to a resin and stirred for 10 minutes at room temperature, and then the reaction solution was removed (repeated twice). Afterward, the remaining resin was washed three times by adding 20 mL each of dichloromethane and dimethylformamide. N-alpha-fluorenylmethoxycarbony l (Fmoc)-N-epsilon-[1-(4,4-dimethyl-2,6-dioxycyclohex-1-ylidine)ethyl]-L-lysine (5.6 mmol, 2.0 equiv.), hydroxybenzotriazole (11.2 mmol, 4.0 equiv.), and N,N'-diisopropylcarbodiimide (5.6 mmol, 2 equiv.), which would be used in a subsequent reaction, were added to the resin and stirred in 50 mL of dimethylformamide as a solvent for 2 hours at room temperature, followed by removing the reaction solution. Afterward, the above process was repeatedly performed in the same manner as described above by sequentially adding N-alpha-Fmoc-N-epsilon-[1(4,4-dimethyl-2,6-dioxycyclohex-1-ylidin)ethyl]-L-lysine and 2-azidoacetic acid. After the reaction with 2-azidoacetic acid, the reaction was washed by adding 50 mL each of dichloromethane and dimethylformamide three times, thereby preparing Compound 9.

### Preparation of Compound 10

50 mL of 5% hydrazine-containing dimethylformamide (volume ratio) was added to a resin and stirred at room temperature for 10 minutes, and then the reaction solution was removed. Afterward, the resin was washed by adding 50 mL each of dichloromethane and dimethylformamide three times, thereby preparing Compound 10.

### Preparation of Compound 11

N-alpha-Fmoc-N-epsilon-fluorenylmethoxycarbonyl-L-lysine (N-alpha-Fmoc-N-epsilon-Fmoc-L-lysine) (11.2 mmol, 4.0 equiv.), hydroxybenzotriazole (22.4 mmol, 8.0 equiv.), and diisopropylcarbodiimide (11.2 mmol, 4.0 equiv.) were added to a resin and stirred in 50 mL of dimethylformamide as a solvent for 2 hours at room temperature, followed by removing the reaction solution. After the reaction, 40 mL of a 20% piperidine-containing dimethylformamide (volume ratio) solution was added to the resin and stirred for 10 minutes at room temperature, and then the solution was removed (repeated twice). Afterward, the resin was washed by adding 50 mL each of dichloromethane and dimethylformamide three times. In a subsequent process, after adding 4,7,10,13,16,19,22,25-octaoxahexacosanoic acid (22.4 mmol, 8.0 equiv.), hydroxybenzotriazole (44.8 mmol, 16.0 equiv.) and diisopropylcarbodiimide (22.4 mmol, 8.0 equiv.) were added to the resin in the same manner as described above, and then 50 mL of dimethylformamide was added as a solvent and stirred at room temperature for 2 hours, followed by removing the reaction solution. After the reaction, the resin was washed by adding 50 mL each of dichloromethane and dimethylformamide three times and then dried, thereby preparing Compound 11.

### Preparation of Compound 12

50 mL of trifluoroacetic acid (TFA), triisopropylsilane (TIS), and deionized water (DW) were mixed in a volume ratio of 95:2.5:2.5 (TFA:TIS:DW), and the resulting mixture was added to a resin which that had been prepared up to Compound 14 and then stirred at room temperature for 2 hours. After stirring, the resulting solution and the resin were isolated. 500 mL of diethyl ether at 0 °C was added to the isolated solution and stirred, and then left at 0 °C for 2 hours or more. After filtering the precipitated product, 50 mL of diethyl ether at 0 °C was added and filtered again (repeated twice). Subsequently, the product was dried, thereby preparing Compound 12 (2.13 mmol, 76%).

### Preparation of Compound 13

Compound 12 (1 g, 0.433 mmol) and tetramethyl-O-(N-succinimidyl)uranium tetrafluoroborate (1 mmol, 2.0 equiv.) were mixed in 5 mL of dimethylformamide, diisopropylethylamine (1.3 mmol, 3.0 equiv.) was added, and then the resulting mixture was stirred at room temperature for 1 hour. After the reaction, trifluoroacetic acid was added to adjust the pH of the resulting solution to 4.0. The reaction solution was purified by Prep-HPLC (C18), thereby obtaining Compound 13 (0.303 mmol, 70%).

### Preparation of Compound 14

Compound 13 (0.303 mmol) and tert-butyl 2-mercaptoacetate (0.606 mmol, 2.0 equiv.) were mixed in 5 mL of dimethylformamide, and then diisopropylethylamine (0.909 mmol, 3.0 equiv.) was added, and the resulting mixture was stirred at room temperature for 1 hour. After the reaction, trifluoroacetic acid was added to adjust the pH of the resulting solution to 4.0. The reaction solution was purified by Prep-HPLC (C18) and lyophilized, thereby obtaining Compound 14 (0.242 mmol, 80%).

### Preparation of Compound 15

10 mL each of trifluoroacetic acid and dichloromethane (volume ratio of 1:1) was added to Compound 14 (0.242 mmol) and then stirred at room temperature for 1 hour. After the reaction, the solution was removed using a vacuum evaporator and then 10 mL of dichloromethane was added to perform concentration under reduced pressure (repeated three times), thereby preparing Compound 18. Compound 15 was dried and stored at -80 °C.

### Preparation of Compound 16

10 mL of a dimethylformamide solution was added to Compound 15 (0.242 mmol), and diisopropylethylamine was added to adjust the pH to 8 to 9. After stirring at room temperature for 10 minutes, tetramethyl-O-(N-succinimidyl)uranium tetrafluoroborate (0.7 mmol, 2.0 equiv.) was added together with diisopropylethylamine (0.375 mmol, 1.0 equiv.), and then stirred at room temperature for 2 hours. After the reaction, trifluoroacetic acid was added to allow the pH of the solution to be 4.0. The resulting solution was purified by Prep-HPLC (C18) and lyophilized, thereby obtaining Compound 16 (0.124 mmol, 51%).

### Preparation of Compound 17

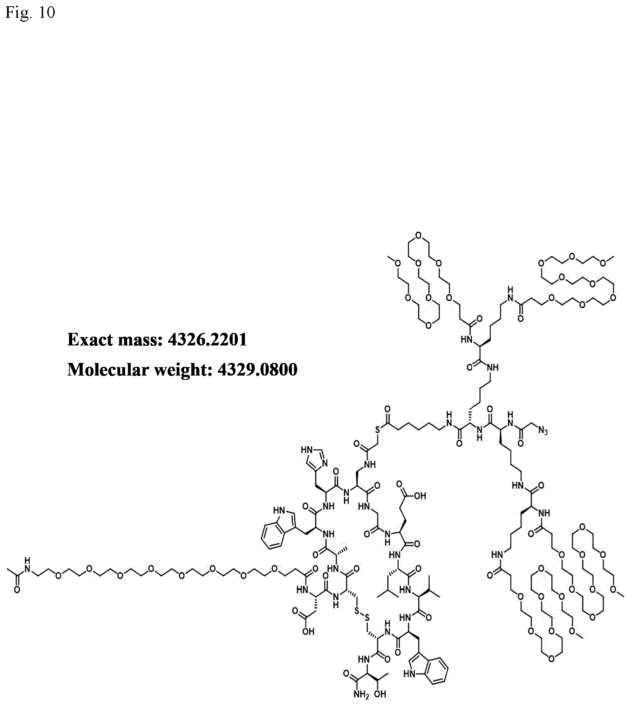

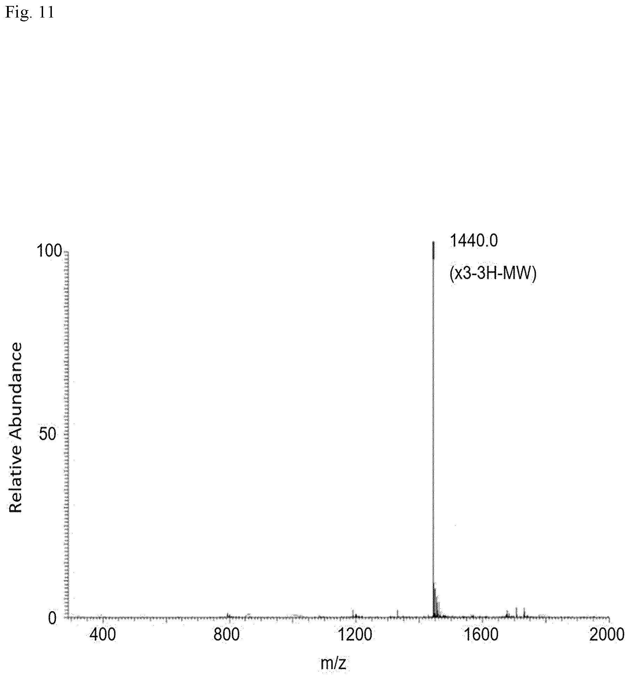

5 mL of a dimethylformamide solution was added to Compound 16 (0.173 mmol), FcBP (L6Dap FcBP) (0.225 mmol, 1.2 equiv.) peptide composite that had been synthesized by a solid phase peptide synthesis (SPPS) method was added, and then diisopropylethylamine (0.26 mmol, 1.5 equiv.) was added to allow a reaction at room temperature for 1 hour. After the reaction, Prep-HPLC (C18) was purified and lyophilized to obtain Compound 17 (283 mg, 0.066 mmol, 53%). The detailed structure of Compound 17 (FcBP-C6PEG4) is shown in FIG. 10. The mass of the obtained Compound 17 was analyzed using a mass spectrometer (using analysis instrument combined with Waters, Quatro Premier XE and Acquity Waters LC system), and the mass spectrometry data of the synthesized Compound 17 (FcBP-C6PEG4) is shown in FIG. 11 ([M+3H]³⁺: 1440.0).

### 3.3. Preparation of compound for transferring functional group to antibody (3): FcBP-C6PEG3 (Compound 2 7)

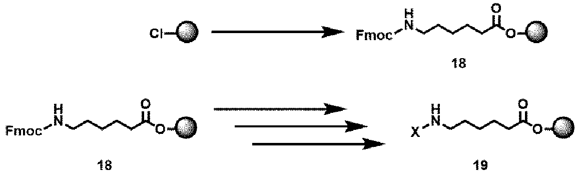

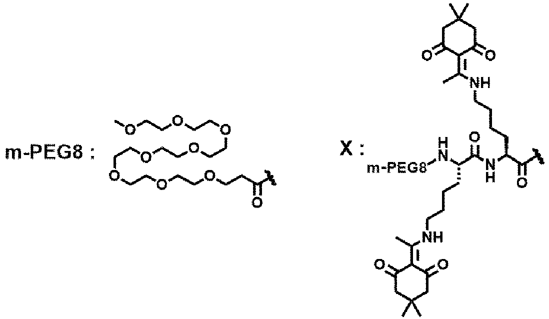

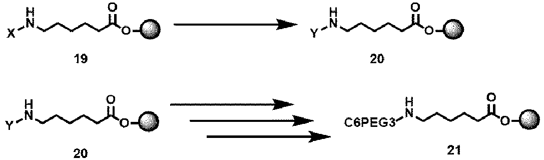

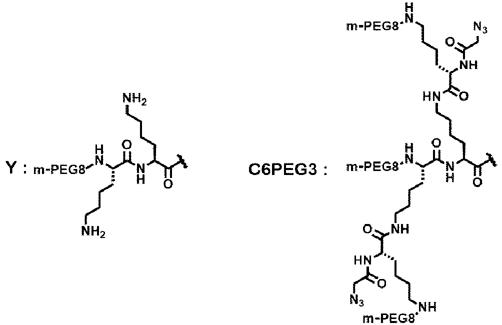

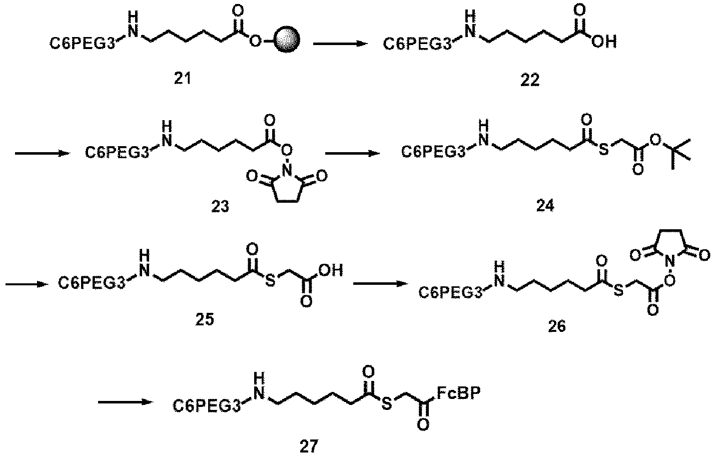

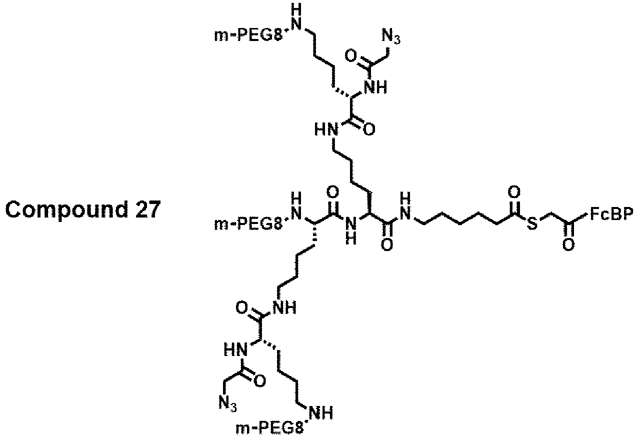

### Preparation of Compound 18

50 mL of dichloromethane was added to 5 g of 2-chlorotrityl chloride resin (1.4 mmol/g) and stirred for 30 minutes. After stirring, a solvent was removed, and for a subsequent reaction, Fmoc-L-6-aminohexanoic acid (14 mmol, 2.0 equiv.) and N,N'-diisopropylethylamine (28 mmol, 4.0 equiv.) were mixed with 40 mL of dichloromethane and added to a resin. Afterward, the resulting mixture was stirred at room temperature for 2 hours, and then the reaction solution was removed. Subsequently, the resin was washed by adding 50 mL each of dichloromethane and dimethylformamide three times, thereby preparing Compound 18.

### Preparation of Compound 19

40 mL of a 20% piperidine-containing dimethylformamide (volume ratio) solution was added to a resin and stirred for 10 minutes at room temperature, and then the reaction solution was removed (repeated twice). Afterward, the remaining resin was washed by adding 20 mL each of dichloromethane and dimethylformamide three times. N-alpha-Fmoc-N-epsilon-[1-(4,4-dimethyl-2,6-dioxycyclohex-1-ylidine)ethyl]-L-lysine (14 mmol, 2.0 equiv.), hydroxybenzotriazole (28 mmol, 4.0 equiv.), and N,N'-diisopropylcarbodiimide (14 mmol, 2.0 equiv.), which were used in a subsequent reaction, were added to the resin, and then stirred in 50 mL of dimethylformamide as a solvent for 2 hours at room temperature, followed by removing the reaction solution. Afterward, the process was repeatedly performed in the same manner by sequentially adding amino acid N-alpha-Fmoc-N-epsilon-[1(4,4-dimethyl-2,6-dioxycyclohex-1-ylidine)ethyl]-L-lysine and 4,7,10,13,16,19,22,25-octaoxahexacosanoic acid. After the reaction of 4,7,10,13,16,19,22,25-octaoxahexacosanoic acid, the resin was washed by adding 50 mL each of dichloromethane and dimethylformamide three times, thereby preparing Compound 19.

### Preparation of Compound 20

50 mL of 5% hydrazine-containing dimethylformamide (volume ratio) was added to a resin and stirred at room temperature for 10 minutes, and then the reaction solution was removed. Subsequently, the resin was washed by adding 50 mL each of dichloromethane and dimethylformamide three times, thereby preparing Compound 20.

### Preparation of Compound 21

N-alpha-Fmoc-N-epsilon-[1-(4,4-dimethyl-2,6-dioxycyclohex-1-ylidine)ethyl]-L-lysine (28 mmol, 4.0 equiv.), hydroxybenzotriazole (56 mmol, 8.0 equiv.), and diisopropylcarbodiimide (28 mmol, 4.0 eq) were added to a resin and stirred in 50 mL of dimethylformamide as a solvent for 2 hours at room temperature, followed by removing the reaction solution. After the reaction, 40 mL of a 20% piperidine-containing dimethylformamide (volume ratio) solution was added to the resin and stirred for 10 minutes at room temperature, followed by removing the reaction solution (repeated twice). Subsequently, the resin was washed by adding 50 mL each of dichloromethane and dimethylformamide three times. In a subsequent reaction, 2-azidoacetic acid (28 mmol, 2.0 equiv.) was added, and then hydroxybenzotriazole (56 mmol, 4.0 equiv.) and diisopropylcarbodiimide (28 mmol, 2.0 equiv.) were added to a resin in the same manner as above. The resulting mixture was then stirred in 50 mL of dimethylformamide as a solvent for 2 hours at room temperature, and then the solution was removed. After the reaction, the resin was washed by adding 50 mL each of dichloromethane and dimethylformamide three times. In a subsequent process, the process of preparing Compound 3 was performed in the same manner as above. 2,5,8,11,14,17,20,23-Octaoxahexacosan-26-oic acid (28 mmol, 2.0 equiv.), hydroxybenzotriazole (56 mmol, 4.0 equiv.), and diisopropylcarbodiimide (28 mmol, 2.0 equiv.) were added sequentially and stirred in 50 mL of dimethylformamide as a solvent for 2 hours at room temperature, followed by removing the solution. After the reaction, the resin was washed by adding 50 mL each of dichloromethane and dimethylformamide three times and then dried, thereby preparing Compound 21.

### Preparation of Compound 22

50 mL of trifluoroacetic acid (TFA), triisopropylsilane (TIS), and deionized water (DW) were mixed in a volume ratio of 95:2.5:2.5 (TFA:TIS:DW) and then added to the resin subjected to the preparation of Compound 4, followed by stirring it at room temperature for 2 hours. After stirring, the solution and the resin were separated. 500 mL of diethyl ether at 0 °C was added to the separated solution, and then left at 0 °C for 2 hours or more. After filtering the precipitated product, 50 mL of diethyl ether at 0 °C was added and filtered again (repeated twice). Subsequently, the product was dried, thereby preparing Compound 22 (5.11 mmol, 73%).

### Preparation of Compound 23

Compound 22 (0.996 g, 0.5 mmol) and tetramethyl-O-(N-succinimidyl)uranium tetrafluoroborate (1 mmol, 2.0 equiv.) were mixed in 5 mL of dimethylformamide, diisopropylethylamine (1.5 mmol, 3.0 equiv.) was added, and then the resulting mixture was stirred at room temperature for 1 hour. After the reaction, trifluoroacetic acid was added to adjust the pH of the resulting solution to 4.0. The reaction solution was purified by Prep-HPLC (C18), thereby obtaining Compound 23 (0.375 mmol, 75%).

### Preparation of Compound 24

Compound 23 (0.375 mmol) and tert-butyl 2-mercaptoacetate (0.7 mmol, 2.0 equiv.) were mixed in 5 mL of dimethylformamide, diisopropylethylamine (1.1 mmol, 3.0 equiv.) was added, and then the resulting mixture was stirred at room temperature for 1 hour. After the reaction, trifluoroacetic acid was added to adjust the pH of the resulting solution to 4.0. The reaction solution was purified by Prep-HPLC (C18), thereby obtaining Compound 24 (0.3 mmol, 80%).

### Preparation of Compound 25

10 mL each of trifluoroacetic acid and dichloromethane (volume ratio of 1:1) was added to Compound 24 (0.375 mmol) and then stirred at room temperature for 1 hour. After the reaction, the solution was removed using a vacuum evaporator, and 10 mL of dichloromethane was added to perform concentration under reduced pressure (repeated three times), thereby obtaining Compound 8. Compound 25 was dried and stored at -80 °C (0.297 mmol, 99%).

### Preparation of Compound 26

10 mL of a dimethylformamide solution was added to Compound 25 (0.375 mmol), and diisopropylethylamine was added to adjust the pH to 8 to 9. After stirring at room temperature for 10 minutes, tetramethyl-O-(N-succinimidyl)uranium tetrafluoroborate (0.7 mmol, 2.0 equiv.) was added together with diisopropylethylamine (0.375 mmol, 1.0 equiv.), and then stirred at room temperature for 2 hours. After the reaction, trifluoroacetic acid was added so that the pH of the solution was 4.0. The resulting solution was purified by Prep-HPLC (C18) and lyophilized, thereby obtaining Compound 26 (0.173 mmol, 46%).

### Preparation of Compound 27

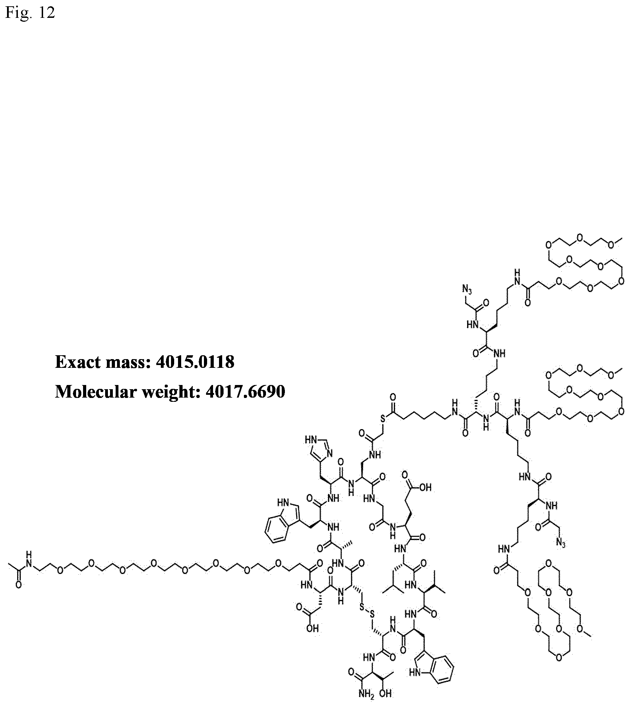

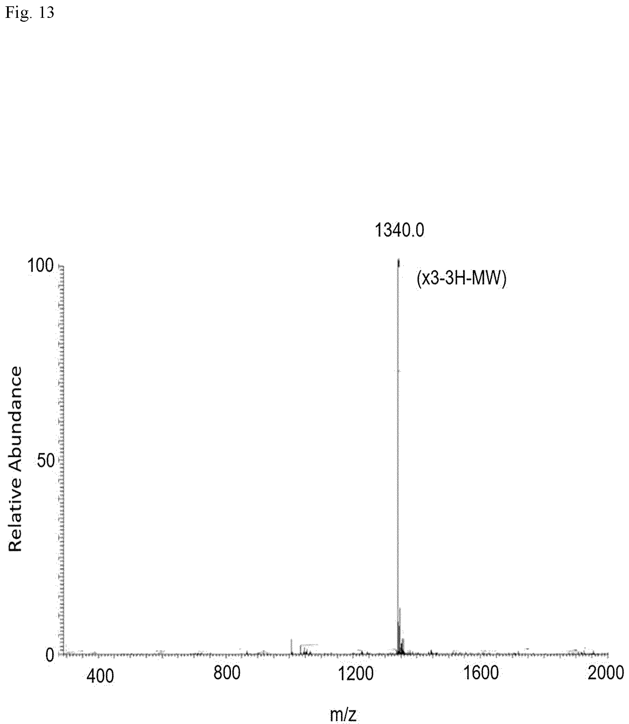

5 mL of a dimethylformamide solution was added to Compound 26 (0.173 mmol), FcBP (L6Dap FcBP) (0.225 mmol, 1.2 equiv.) peptide composite that had been synthesized by a solid phase peptide synthesis (SPPS) method was added, and then diisopropylethylamine (0.26 mmol, 1.5 equiv.) was added to allow a reaction at room temperature for 1 hour. After the reaction, Prep-HPLC (C18) was purified and lyophilized to obtain Compound 27 (348 mg, 0.087 mmol, 50%). The detailed structure of Compound 27 (FcBP-C6PEG3) is shown in FIG. 12. The mass of the obtained Compound 27 was analyzed using a mass spectrometer (using an analysis instrument combined with Waters, Quatro Premier XE and Acquity Waters LC system), and the mass spectrometry data of the synthesized Compound 27 is shown in FIG. 13 ([M+3H]³⁺: 1340.0).

### 3.4. Preparation of compound for transferring functional group to antibody (4): FcBP-azidobenzoic acid (Compound 31)

After dissolving dithiodiglycolic acid in dichloromethane, glycine t-butyl ester (2.2 equiv.), N,N-diiso-propylamine (4.8 molar equiv.), and 1-ethyl-3-(3-dimethylaminopropyl) carbodiimide hydrochloride (2.4 molar equiv.) were added. In addition, the mixture was stirred for one hour. The reactants were separated and washed with water, and an organic layer was obtained and concentrated under reduced pressure. The obtained residue was dissolved in N,N-dimethylformamide/water (1:1), tris(2-carboxyethyl) phosphine hydrochloride (1.3 molar equiv.) was added and stirred at room temperature for 1 hour. The solution was extracted with ethyl acetate, concentrated under reduced pressure, and treated with ethyl acetate/hexane in a silica gel column, thereby obtaining Compound 28.

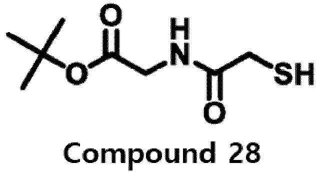

Compound 28 was dissolved in dichloromethane, 4-azidobenzoic acid (1 molar equiv.), 1H-benzotriazol-1-yloxytripyrrolidinophosphonium hexafluorophosphate (1.2 molar equiv.), and N,N-diisopropylethylamine (1.5 molar equiv.) were added, and the resulting mixture was stirred at room temperature for 30 minutes. The reactant was purified using a silica gel column. Afterward, the obtained white crystals were dissolved in dichloromethane/trifluoroacetic acid (1:1), and stirred at room temperature for one hour. The reactant was lyophilized, thereby obtaining a first linker (Compound 29) comprising thioglycolate as a releasing group.

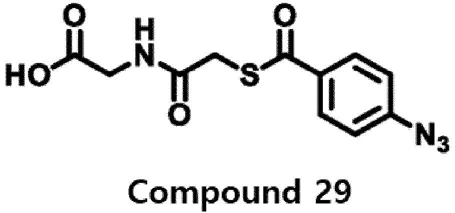

FcBP-azidobenzoic acid (first linker-FcBP, Compound 31) was prepared by reacting Compound 29 with Compound 30. A specific method is as follows.

FcBP (Compound 30), Compound 29 (40 molar equiv.), 1-ethyl-3-(3-dimethylaminopropyl) carbodiimide hydrochloride (32 molar equiv.), and 1-hydroxybenzotriazole (32 molar equiv.) were mixed, and the mixture was stirred for 2 hours. After the reaction, the resulting mixture was purified by HPLC, thereby obtaining FcBP-azidobenzoic acid (first linker-FcBP, Compound 31).

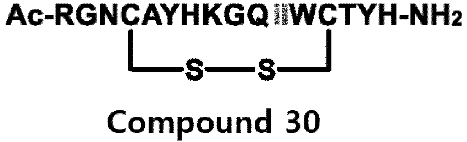

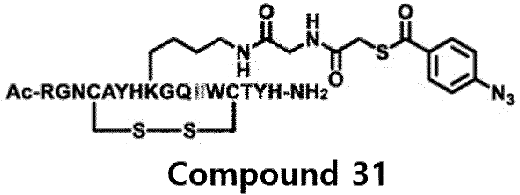

### 4. Confirmation of effect of thiol-reactive additive in preparation of antibody comprising functional group 1

Referring to the following Experimental Example, it was confirmed that the yield of an antibody comprising a functional group or antibody-drug conjugate increases when a thiol-reactive additive is used in the process of preparing an antibody comprising a functional group or an antibody-drug conjugate.

### 4. 1. Confirmation of effect of thiol-reactive additive in preparation of antibody-drug conjugate using FcBP-N₃ (Compound 7)

### (1) Preparation of antibody-drug conjugate

### 1) Preparation of antibody comprising bio-orthogonal functional group (azide)

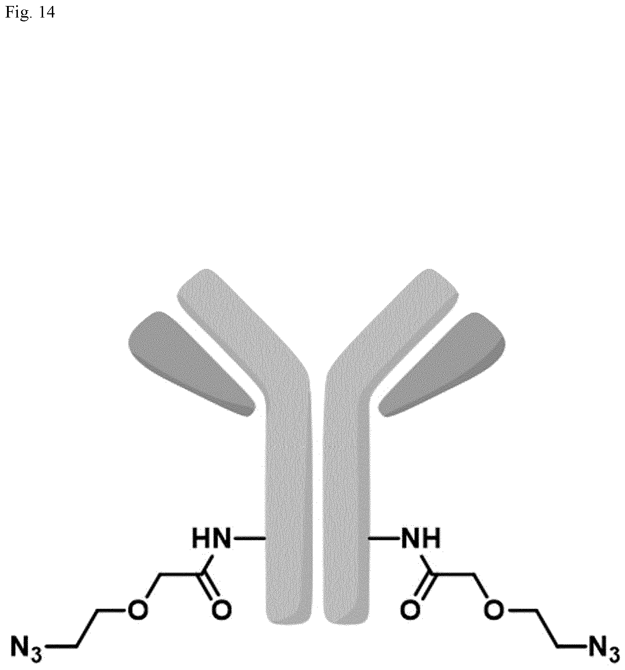

An antibody comprising a functional group was prepared by the following method. FcBP-N₃ (Compound 7) was prepared at a concentration of 10 mM in DMSO solvent. A thiol-reactive additive (N-tert-butylmaleimide) was prepared at a concentration of 100 to 1000 mM in DMSO solvent. 1 mg of an antibody (trastuzumab) and 0, 10, 100, 1000, and 6910 equiv. of a thiol-reactive additive (N-tert-butylmaleimide) based on the antibody were mixed in 1 x PBS (pH 7.4). Based on the target site of the antibody (since there were two K248 in the antibody), each of 0, 5, 50, 500, and 3455 equiv. of the thiol-reactive additives was used. After mixing at room temperature for about 10 minutes, based on the antibody, 10 equiv. of the compound (Compound 7, FcBP-N₃) for transferring a functional group was added and mixed for 6 to 7 hours. Based on a target site, 5 equiv. of the compound for transferring a functional group was used. After the reaction, excess FcBP-N₃ and thiol-reactive additive (N-tert-butylmaleimide) were removed using size exclusion chromatography, spin desalting, and dialysis, and thereby an antibody comprising a bio-orthogonal functional group (azied) was obtained. The schematic diagram of the antibody comprising a functional group (azide) is shown in FIG. 14.

### 2) Payload synthesis

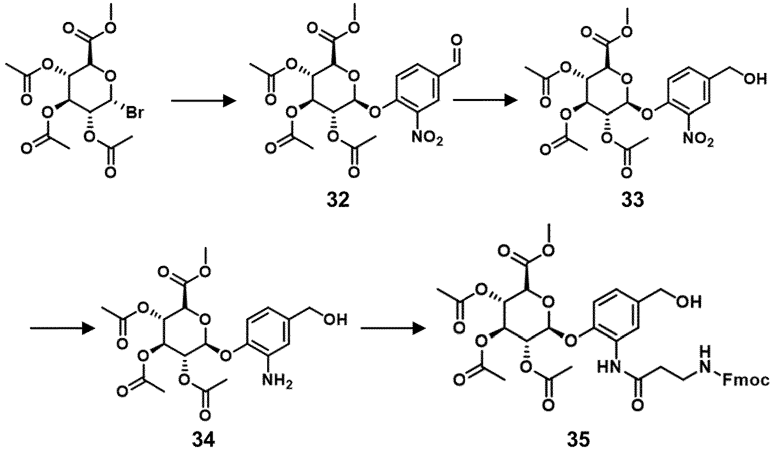

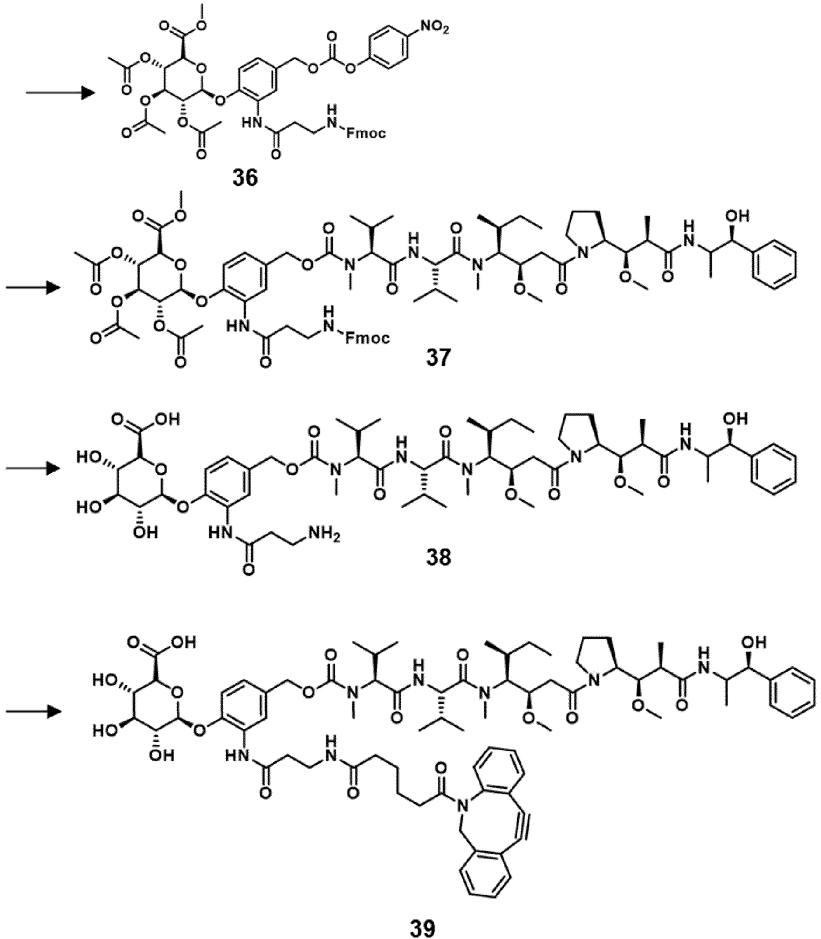

### Preparation of Compound 32

4-Hydroxy-3-nitrobenzaldehyde (4 g, 29.6 mmol) was dissolved in 120 mL acetonitrile, and then a molecular sieve (10 g) was added. In addition, acetobromo-α-D- glucuronic acid methyl ether (10.3 g, 32.6 mmol) and silver oxide (I) (7 g, 30.2 mmol) were added and stirred in an argon atmosphere for 18 hours. The reaction solution was filtered through Celite, concentrated under reduced pressure, and an organic layer was extracted with ethyl acetate and distilled water. The collected organic layer was dried with anhydrous magnesium sulfate. The resulting product was filtered, concentrated, and then purified by column chromatography, thereby obtaining Compound 32 (12.6 g, 88%).

### Preparation of Compound 33

Compound 32 (12.6 g, 26.0 mmol) was dissolved in isopropyl alcohol (50.6 mL) and chloroform (185 mL), sodium borohydride (1.67 g, 44.1 mmol) was slowly added at 0 °C in a nitrogen atmosphere, and then the mixture was stirred for 2.5 hours. Distilled water was added to the reaction solution and stirred for 15 minutes. Ethyl acetate and distilled water were added to extract an organic layer. The collected organic layer was dried with anhydrous magnesium sulfate. The resulting product was filtered, concentrated, and then purified by column chromatography, thereby obtaining Compound 33 (7.7 g, 61.1%).

### Preparation of Compound 34

Compound 33 (6.7 g, 14.2 mmol) was dissolved in ethyl acetate (90 mL), ethanol (190 mL), and methanol (44 mL), palladium (0.33 g) was added, and then the resulting mixture was stirred in a hydrogen atmosphere for 3 hours. The reaction solution was filtered with Celite, concentrated under reduced pressure, and purified by column chromatography, thereby obtaining Compound 34 (5.3 g, 76.9%).

### Preparation of Compound 35

After dissolving Fmoc-beta-alanine (2.46 g, 7.9 mmol) in dichloromethane (13 mL) and N,N-dimethylformamide (0.5 mL), oxalyl chloride (1.36 mL, 15.8 mmol) was slowly added in a nitrogen atmosphere and stirred for 2 hours. After concentrating the reaction solution under reduced pressure, and resulting concentrate was slowly added to Compound 34 dissolved in dichloromethane (33 mL) dropwise at 0 °C. After the concentration of the reaction solution, it was purified by column chromatography, thereby obtaining Compound 35 (3.9 g, 79.1%).

### Preparation of Compound 36

Compound 35 (2.5 g, 3.34 mmol) was dissolved in dichloromethane (30 mL), bis(4-nitrophenyl)carbonate (1.73 g, 5.7 mmol), and N,N-diisopropylethylamine (0.99 mL, 5.68 mmol) were added and then the resulting mixture was stirred for 2 hours. The reaction solution was concentrated under reduced pressure and purified by column chromatography, thereby obtaining Compound 36 (2.3 g, 75.4%).

### Preparation of Compound 37

Compound 36 (1 g, 1.09 mmol) was dissolved inN,N-diisopropylethylamine (10.8 mL), monomethyl auristatin E (MMAE, 0.864 g, 1.2 mmol), N-hydroxybenzotriazole hydrate (HOBt·H2O, 0.44 g, 3.27 mmol), and N,N-diisopropylethylamine (0.28 mL, 1.64 mmol) were added and then the resulting mixture was stirred at room temperature for 18 hours. The reaction solution was concentrated under reduced pressure and purified by column chromatography, thereby obtaining Compound 37 (1.45 g, 89%).

### Preparation of Compound 38

Compound 37 (1.4 g, 9.37 mmol) was dissolved in methanol (15.2 mL), a 0.4N lithiumhydroxide solution (1.5 mL) was added, and then the resulting mixture was stirred for 6 hours. The reaction solution was purified by column chromatography, thereby obtaining Compound 38 (0.62 g, 59%).

### Preparation of Compound 39 (payload)

Compound 38 (0.6 g, 0.53 mmol) was dissolved in N,N-dimethylformamide (5 mL), DBCO-C6-NHS (0.27 g, 0.64 mmol), and N,N-diisopropylethylamine (185 µL, 1.06 mmol) were added, and then the resulting solution was stirred at room temperature for 2 hours. The reaction solution was purified by column chromatography, thereby obtaining Compound 39 (0.5 g, 65%).

### 3) Preparation of antibody-drug conjugate

A DBCO functional group that undergoes click chemistry reaction with an azide and the payload (Compound 39) containing a drug were prepared at a concentration of 10 mM in DMSO solvent. 1 mg (1 mg/ml) of the antibody linked to the bio-orthogonal functional group (azide), which had been obtained after dialysis and the payload (Compound 39) were mixed in 1 x PBS buffer (pH 7.4), and the reaction was performed for 1 day. Here, 3 equiv. of the payload per reaction site was used. The reaction was observed using hydrophobic interaction chromatography. After completion of the reaction, an excess payload was removed using size exclusion chromatography, spin desalting, and dialysis.

### (2) Confirmation of effect of using thiol-reactive additive

### 1) Analysis method

As described above, an antibody-drug conjugate was prepared, and analyzed using hydrophobic-interaction chromatography (HIC)-HPLC. For analysis, an HIC-butyl column with specifications of 4.6 × 100 mm and 5 µm (MAbPac^{™}, Thermo Fisher Scientific) was used.

### 2) Analysis result

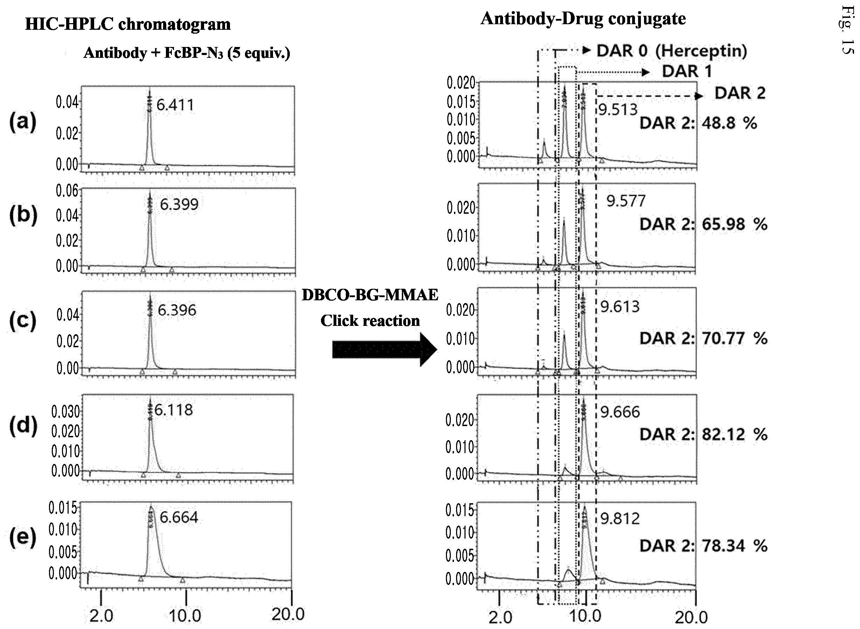

The yield of the antibody-drug conjugate was analyzed according to whether the thiol-reactive additive was used and the amount of the thiol-reactive additive added. The analysis result is shown in FIG. 15. Specifically, FIG. 15A is the result of the case in which the thiol-reactive additive was not used. FIG. 15B is the result for 5 equiv. of the thiol-reactive additive sample. FIG. 15C is the result for 50 equiv. of the thiol-reactive additive sample. FIG. 15D is the result for 500 equiv. of the thiol-reactive additive sample. FIG. 15E is the result for 3455 equiv. of the thiol-reactive additive sample.

In the process of preparing an antibody comprising a functional group or an antibody-drug conjugate, when the thiol-reactive additive was used, the yield of the antibody-drug conjugate was observed to be higher than when no thiol-reactive additive was used.

In addition, as the amount of the thiol-reactive additive used increased, the yield of the antibody-drug conjugate increased. When 3455 equiv. of the thiol-reactive additive was used, the yield of the observed antibody-drug conjugate was lower than that observed when 500 equiv. of the thiol-reactive additive was used.

### 4.2. Confirmation of effect of thiol-reactive additive in preparation of antibody-drug conjugate using FcBP-C6PEG4 (Compound 17)

### (1) Preparation of antibody-drug conjugate

### 1) Preparation of antibody comprising functional group (azide)

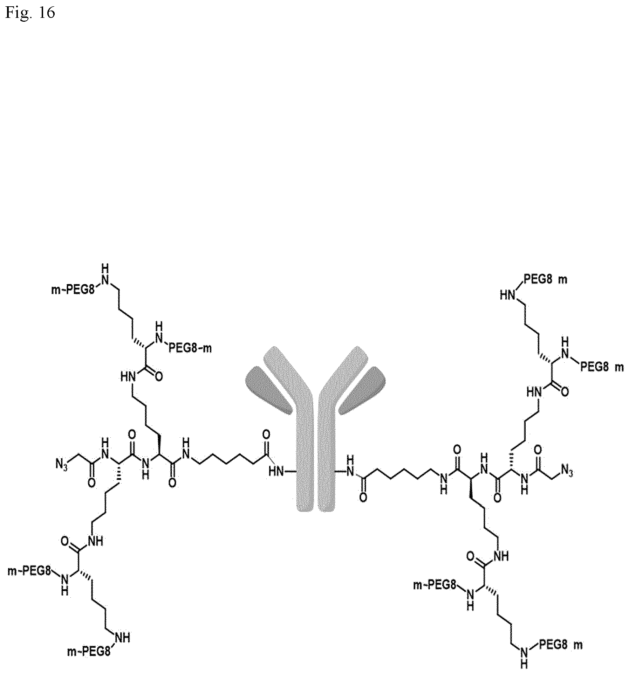

FcBP-C6PEG4 (Compound 17) was prepared at a concentration of 10 mM in DMSO. A thiol-reactive additive (N-tert-butylmaleimide) was prepared at concentrations of 100 mM and 1000 mM in DMSO. 1 mg (1 mg/mL) of an antibody (trastuzumab) and each of 0, 10, 100, 1000, and 6910 equiv. of thiol-reactive additives based on the antibody were mixed in 1 × PBS (pH 7.4). Based on the target site of the antibody, each of 0, 5, 50, 500, and 3455 equiv. of the thiol-reactive additives was used. After mixing at room temperature for about 10 minutes, based on the antibody, 10 equiv. of the compound (Compound 17, FcBP-C6PEG4) for transferring a functional group was added and mixed for 6 to 7 hours. Based on a target site, 5 equiv. of the compound for transferring a functional group was used. After the reaction, excess FcBP-C6PEG4 and thiol-reactive additive (N-tert-butylmaleimide) were removed using size exclusion chromatography, spin desalting, and dialysis. The schematic diagram of the antibody comprising a functional group (azide) is shown in FIG. 16.

### 2) Preparation of antibody-drug conjugate

A DBCO functional group that undergoes click chemistry reaction with an azide and the payload (Compound 39) containing a drug were prepared at a concentration of 10 mM in DMSO solvent. 1 mg (1 mg/ml) of the antibody linked to the bio-orthogonal functional group (azide), which had been obtained after dialysis, and the payload (Compound 39) were mixed in 1 × PBS buffer (pH 7.4), and the reaction was performed for 1 day. Here, 3 equiv. of the payload per reaction site was used. The reaction was observed using hydrophobic interaction chromatography. After completion of the reaction, an excess payload was removed using size exclusion chromatography, spin desalting, and dialysis.

### (2) Confirmation of effect of using thiol-reactive additive

### 1) Analysis method

As described above, an antibody-drug conjugate was prepared, and analyzed using hydrophobic-interaction chromatography (HIC)-HPLC. For analysis, an HIC-butyl column with specifications of 4.6 × 100 mm and 5 µm (MAbPac^{™}, Thermo Fisher Scientific) was used.

### 2) Analysis method

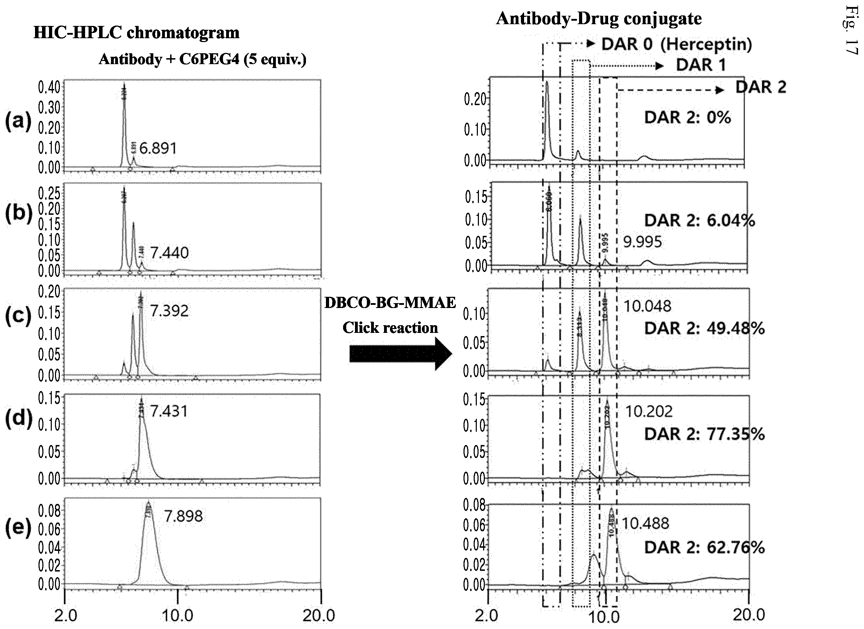

The yield of the antibody-drug conjugate was analyzed according to whether the thiol-reactive additive was used and the amount of the thiol-reactive additive added. The analysis method is shown in FIG. 17. FIG. 17A is the result of the case in which the thiol-reactive additive was not used. FIG. 17B is the result for 5 equiv. of the thiol-reactive additive sample. FIG. 17C is the result for 50 equiv. of the thiol-reactive additive sample. FIG. 17D is the result for 500 equiv. of the thiol-reactive additive sample. FIG. 17E is the result for 3455 equiv. of the thiol-reactive additive sample.

In the process of preparing an antibody comprising a functional group or an antibody-drug conjugate, when the thiol-reactive additive was used, the yield of the antibody-drug conjugate was observed to be higher than when no thiol-reactive additive was used.

In addition, as the amount of the thiol-reactive additive used increased, the yield of the antibody-drug conjugate increased. When 3455 equiv. of the thiol-reactive additive was used, the yield of the observed antibody-drug conjugate was lower than that observed when 500 equiv. of the thiol-reactive additive was used.

### 4.3. Confirmation of effect of thiol-reactive additive in preparation of antibody-drug conjugate using FcBP-C6PEG3 (Compound 27)

### (1) Preparation of antibody-drug conjugate

### 1) Preparation of antibody comprising functional group (azide)

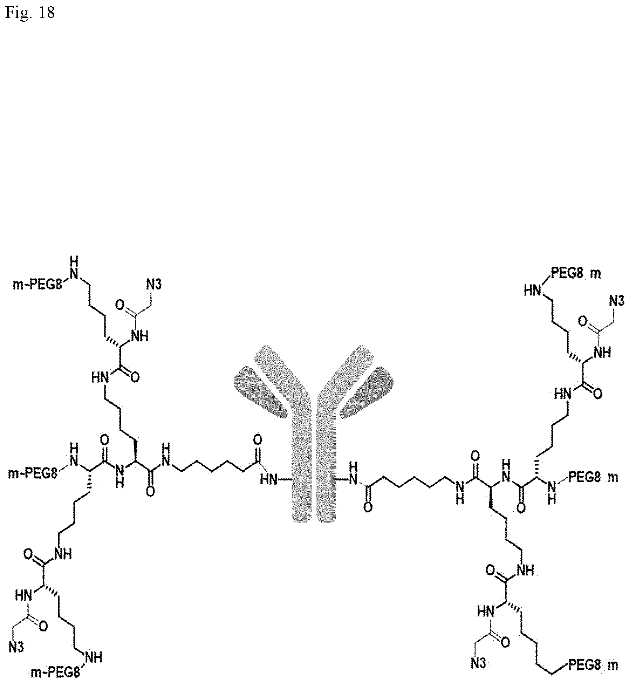

FcBP-C6PEG3 (Compound 27) was prepared at a concentration of 10 mM in DMSO. A thiol-reactive additive (N-tert-butylmaleimide) was prepared at concentrations of 100 mM and 1000 mM in DMSO. 1 mg (1mg/mL) of an antibody (trastuzumab) and each of 0, 10, 100, 1000, and 6910 equiv. of thiol-reactive additives based on the antibody were mixed in 1 × PBS (pH 7.4). Based on the target site of the antibody, each of 0, 5, 50, 500, and 3455 equiv. of the thiol-reactive additives was used. After mixing at room temperature for about 10 minutes, based on the antibody, 10 equiv. of the compound (Compound 27, FcBP-C6PEG3) was added and mixed for 6 to 7 hours. Based on a target site, 5 equiv. of the compound for transferring a functional group was used. After the reaction, excess FcBP-C6PEG4 and thiol-reactive additive (N-tert-butylmaleimide) were removed using size exclusion chromatography, spin desalting, and dialysis. The schematic diagram of the antibody comprising a functional group (azide) is shown in FIG. 18.

### 2) Preparation of antibody-drug conjugate

A DBCO functional group that undergoes click chemistry with an azide and the payload (Compound 39) containing a drug were prepared at a concentration of 10 mM in DMSO solvent. 1 mg (1 mg/ml) of the antibody linked to the bio-orthogonal functional group (azide), which had been obtained after dialysis, and the payload (Compound 39) were mixed in 1 x PBS buffer (pH 7.4), and the reaction was performed for 1 day. Here, 3 equiv. of the payload per reaction site was used. The reaction was observed using hydrophobic interaction chromatography. After completion of the reaction, an excess payload was removed using size exclusion chromatography, spin desalting, and dialysis.

### (2) Confirmation of effect of using thiol-reactive additive

### 1) Analysis method

As described above, an antibody-drug conjugate was prepared, and analyzed using hydrophobic-interaction chromatography (HIC)-HPLC. For analysis, an HIC-butyl column with specifications of 4.6 × 100 mm and 5 µm (MAbPac^{™}, Thermo Fisher Scientific) was used.

### 2) Analysis result

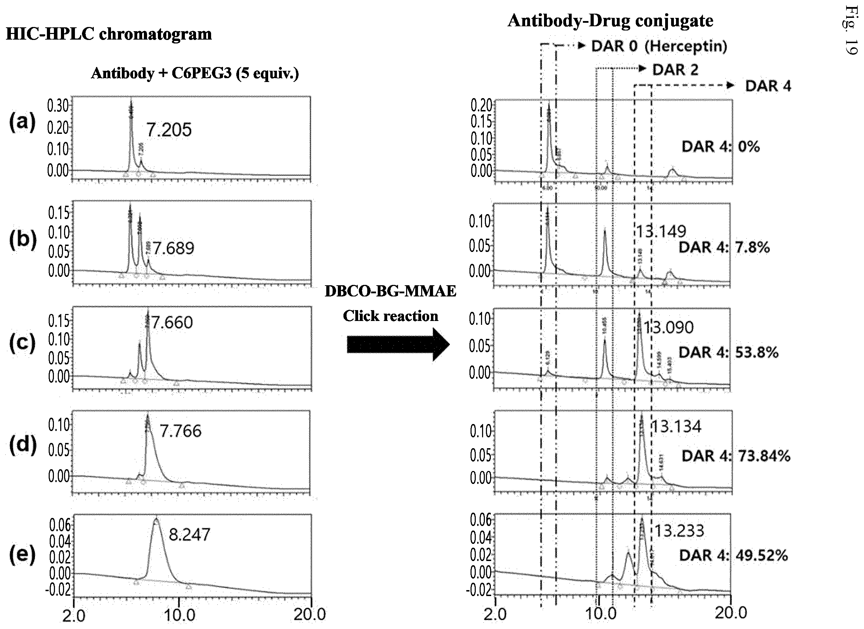

The yield of the antibody-drug conjugate was analyzed according to whether the thiol-reactive additive was used and the amount of the thiol-reactive additive added. The analysis method is shown in FIG. 19. Specifically, FIG. 19A is the result of the case in which the thiol-reactive additive was not used. FIG. 19B is the result for 5 equiv. of the thiol-reactive additive sample. FIG. 19C is the result for 50 equiv. of the thiol-reactive additive sample. FIG. 19D is the result for 500 equiv. of the thiol-reactive additive sample. FIG. 19E is the result for 3455 equiv. of the thiol-reactive additive sample.

In the process of preparing an antibody comprising a functional group or an antibody-drug conjugate (DAR 4, four drugs were conjugated), when the thiol-reactive additive was used, the yield of the antibody-drug conjugate was observed to be higher than when no thiol-reactive additive was used.

In addition, as the amount of the thiol-reactive additive used increased, the yield of the antibody-drug conjugate increased. When 3455 equiv. of the thiol-reactive additive was used, the yield of the observed antibody-drug conjugate was lower than that observed when 500 equiv. of the thiol-reactive additive was used.

### 4.4. Confirmation of effect of thiol-reactive additive in preparation of antibody-drug conjugate using FcBP-azidobenzoic acid (Compound 31)

### (1) Preparation of antibody-drug conjugate

### 1) Preparation of antibody comprising functional group (azide)

An antibody comprising a bio-orthogonal functional group (azide) was prepared using a thiol-reactive additive (N-tert-butylmaleimide), FcBP-azidobenzoic acid, and an antibody (trastuzumab) in a similar manner to that described in 4.1(1) Preparation of antibody-drug conjugate.

### 2) Preparation of antibody-drug conjugate

An antibody-drug conjugate was prepared using an antibody comprising a functional group and a payload containing DBCO and a drug in a similar manner to that described in 4.1(1) Preparation of antibody-drug conjugate.

### (2) Confirmation of effect of using thiol-reactive additive

### 1) Analysis method

An antibody-drug conjugate prepared using HPLC was analyzed in a similar manner to the method described in 4.1. (2) Confirmation of effect of using thiol-reactive additive.

### 2) Analysis result

The yield of the antibody-drug conjugate was analyzed according to whether the thiol-reactive additive was used and the amount of the thiol-reactive additive added in a similar method to the method described in 4.1. (2) Confirmation of effect of using thiol-reactive additive.

### 5. Confirmation of effect of thiol-reactive additive in preparation of antibody comprising functional group 2

The inventors of the present application confirmed an effect of a thiol-reactive additive in the preparation of an antibody comprising a functional group (an antibody comprising norbornene) synthesized in a previous study. The method of preparing an antibody comprising a functional group (i.e., an antibody comprising norbornene) synthesized in the previous study and data related to the antibody comprising a functional group and an antibody-drug conjugate are described in detail in the documents incorporated herein by reference in their entirety, such as Experimental Example of International Application No. PCT/KR2020/003282 (Publication No. WO2020/184944 A1); and the document [Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759]. The antibody comprising a functional group that is disclosed in the document International Application No. PCT/KR2020/003282 (Publication No. WO2020/184944 A1) was prepared by the reaction between a compound for transferring a functional group to an antibody and the antibody in a similar manner to the above description. As an antibody, trastuzumab was used.

### 5.1. Preparation of compound for transferring functional group to antibody used in this experiment

### (1) Preparation of tert-butyl (5-exo-norbornene carbonylthio)acetate (Compound 40)

5-Exo-norbornene carboxylic acid (200 mg, 1.45 mmol, 1.0 equiv.) was dissolved in 5 mL DMF. Hexafluorophosphate azabenzotriazole tetramethyl uranium (HATU) (660 mg, 1.74 mmol, 1.2 equiv.) and DIPEA (0.66 mL, 3.48 mmol, 2.4 equiv.) were added, the resulting mixture was stirred in an argon atmosphere. Tert-butyl α-thioacetate (0.25 mL, 1.74 mmol, 1.2 equiv.) was slowly added to the reaction mixture. Sixteen hours later, DMF was removed under reduced pressure, and tert-butyl (5-exo-norbornene carbonylthio)acetate (Compound 32) (350 mg, 90%, white solid) was purified through flash column chromatography using 2% ethyl acetate in hexane. R_{F} = 0.5 (10% ethyl acetate in hexane); MS (ESI): m/z 269.34 [M+H]⁺ HRMS: [M+H]⁺ calcd. for C₁₄H₂₁O₃S 269.1206 Found 269.1214

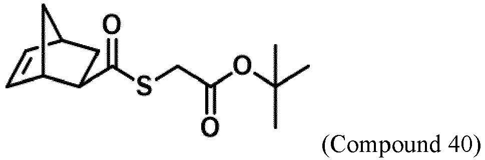

### (2) Preparation of 5-exo-norbornene carbonylthio)acetic acid NHS ester (Compound 41)

In an ice water bath, 5 mL of TFA was slowly added to a solution of Compound 40 (310 mg, 1.16 mmol, 1.0 equiv.) in 5 mL DCM. Two hours later, TFA was removed under reduced pressure, the residue was redissolved in DCM (5 mL), and a volatile material was removed three times under reduced pressure. The residue was dried in high vacuum and redissolved in 10 mL DCM. In an ice water bath, DIPEA (0.6 mL, 3.48 mmol, and 3.0 equiv.) was slowly added to adjust the pH of the solution to 9.0, and *N,N,N',N'*-tetramethyl-*O*-(*N* succinimidyl)uranium tetrafluoroborate (1.05 g, 3.48 mmol, 3.0 equiv.) was added. Three hours later, the reaction mixture was washed with 10% citric acid and brine, and dried over Na₂SO₄. Subsequently, the solvent was removed under reduced pressure. A white solid, Compound 41 (310 mg, 87%), was purified through flash column chromatography using 10% ethyl acetate in hexane. R_{F} = 0.3 (33% ethyl acetate in hexane); MS (ESI): m/z 310.26 [M+H]⁺ HRMS: [M+H]⁺ calcd. for C₁₄H₁₆NO₅S 310.0744, found 310.0756

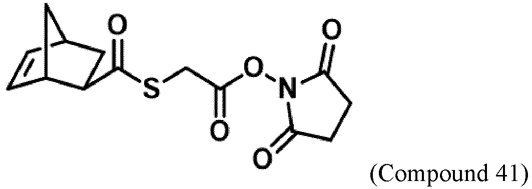

### (3) Preparation of compound for transferring functional group to antibody

Using each of synthesized peptides, such as L6Dap FcBP, L6Dab FcBP, L6Orn FcBP, and L6Lys FcBP (refer to Section "2. Preparation of Fc-binding peptide" of Experimental Example) and Compound 41, FcBP-norbornene was prepared. 5-Exo-norbornene thioester was introduced into the amine functional group (the 6^{th} site of the peptide) of each peptide using 1.5 equiv. of Compound 41 in DMF and 6 equiv. of N,N-diisopropylethylamine (DIPEA) (where FcBP is 1 equiv.). The final FcBP-norbornene was purified by preparative HPLC, and its purity was verified by analytical HPLC.

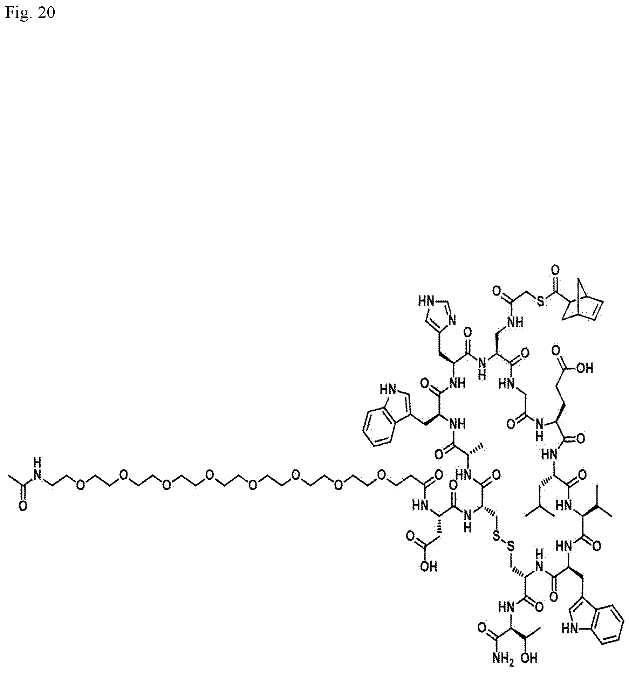

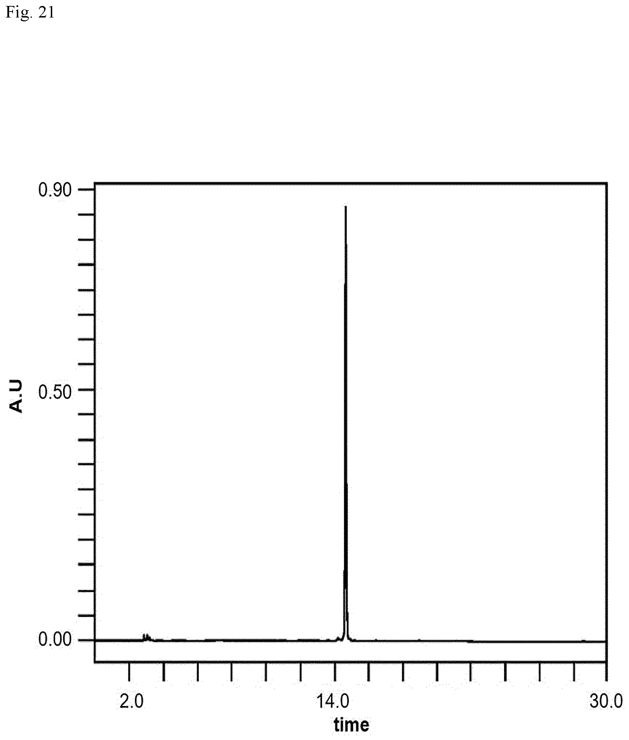

Synthesized FcBP (L6Dap)-norbornene was purified by preparative HPLC and lyophilized, thereby obtaining a white solid. Its purity was confirmed by analytical HPLC. MS (ESI): m/z 1082.34 [M+2H]²⁺. The structure of the FcBP (L6Dap)-norbornene is shown in FIG. 20. The HPLC chromatogram data (RT=14.7 min) of FcBP (L6Dap)-norbornene is shown in FIG. 21.

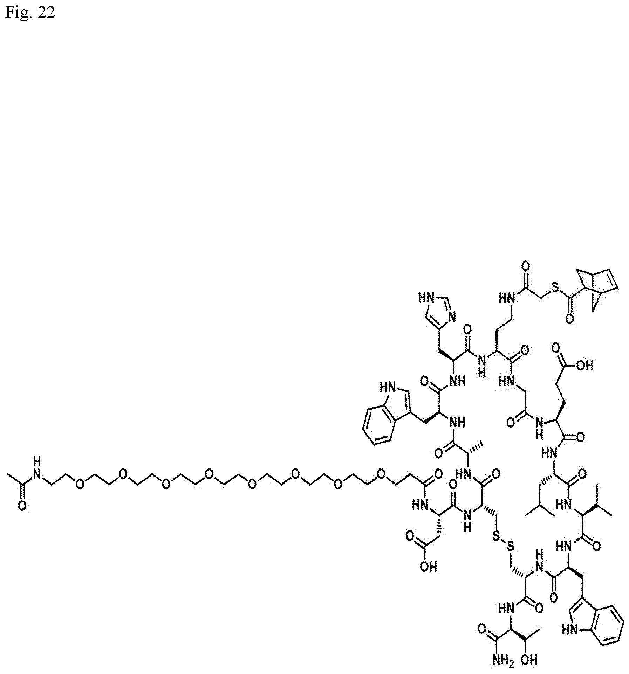

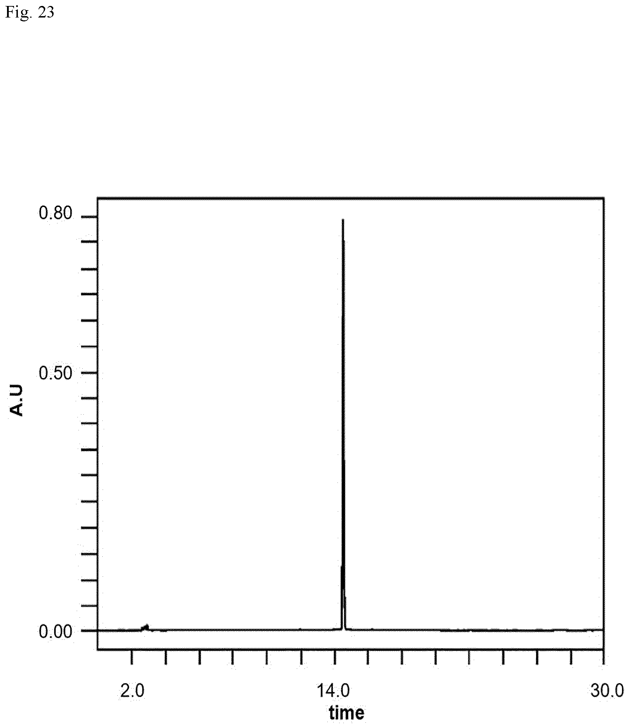

Synthesized FcBP (L6Dab)-norbornene was purified by preparative HPLC and lyophilized, thereby obtaining a white solid. Its purity was confirmed by analytical HPLC. MS (ESI): m/z 1089.94 [M+2H]²⁺. The structure of the FcBP (L6Dab)-norbornene is shown in FIG. 22. The HPLC chromatogram data (RT=14.6 min) of FcBP (L6Dab)-norbornene is shown in FIG. 23.

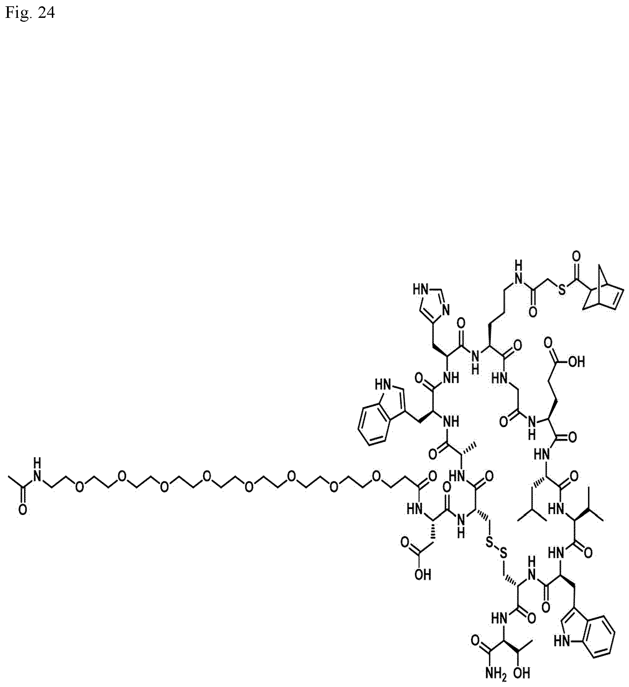

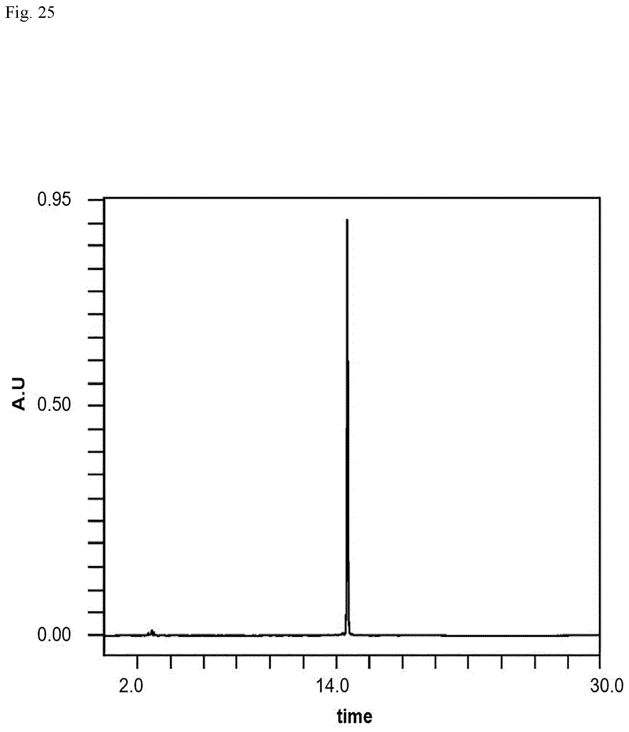

Synthesized FcBP (L6Orn)-norbornene was purified by preparative HPLC and lyophilized, thereby obtaining a white solid. Its purity was confirmed by analytical HPLC. MS (ESI): m/z 1089.94 [M+2H]²⁺. The structure of the FcBP (L6Orn)-norbornene is shown in FIG. 24. The HPLC chromatogram data (RT=14.7min) of FcBP (L6Orn)-norbornene is shown in FIG. 25.

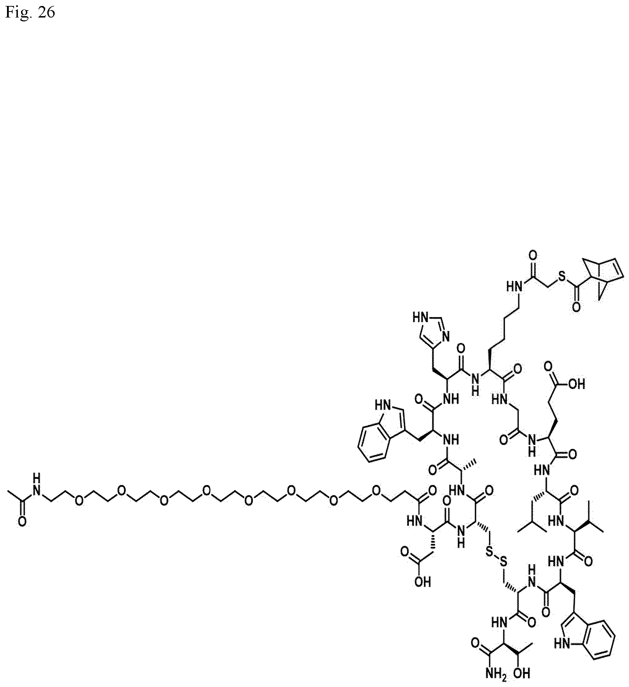

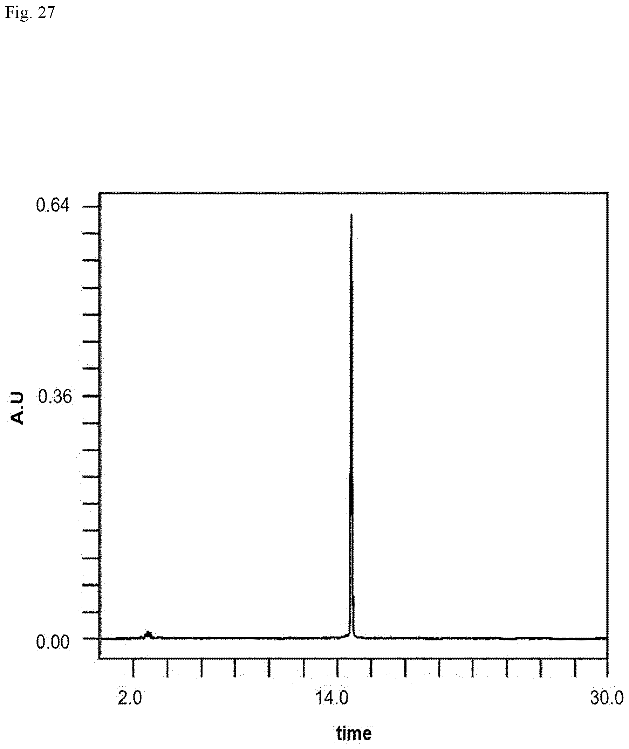

Synthesized FcBP(L6Lys)-norbornene was purified by preparative HPLC and lyophilized, thereby obtaining a white solid. Its purity was confirmed by analytical HPLC. MS (ESI): m/z 1089.94 [M+2H]²⁺. The structure of the FcBP (L6Lys)-norbornene is shown in FIG. 26. The HPLC chromatogram data (RT=14.9 min) of FcBP (L6Lys)-norbornene is shown in FIG. 27.

### 5.2. Acceleration of reaction between antibody and compound for transferring functional group using thiol-reactive additive

### (1) Reaction between antibody and compound for transferring functional group without thiol-reactive additive

To prepare an antibody comprising a functional group, 3 equiv. of each FcBP-norbornene was mixed with trastuzumab (1 mg/mL, 1 mL). The resulting mixture was incubated in 1X PBS (pH 7.4) for 18 hours at 25 °C. The reaction was monitored by HIC-HPLC. Referring to the documents [PCT International Application No. PCT/KR2020/003282; and Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759], it is confirmed that, by using the FcBP-click chemistry functional group (e.g., azide or norbornene), the click chemistry functional group was transferred to the K248 site of the Fc domain of the antibody.

### (2) Reaction between antibody and compound for transferring functional group in the presence of thiol-reactive additive

A mixture of trastuzumab (1 mg/mL, 1 mL) and 50 equiv. of N-tert-butylmaleimide was incubated in 1xPBS with pH 7.4 at 25 °C for 10 minutes. 3 equiv. of each FcBP-norbornene was added to the mixture and incubated for 18 hours. Excess peptides and scavengers were removed by spin desalting. Crosslinking of FcBP-norbornene and trastuzumab was monitored and HIC-HPLC analysis was performed.

### (3) Comparison of results

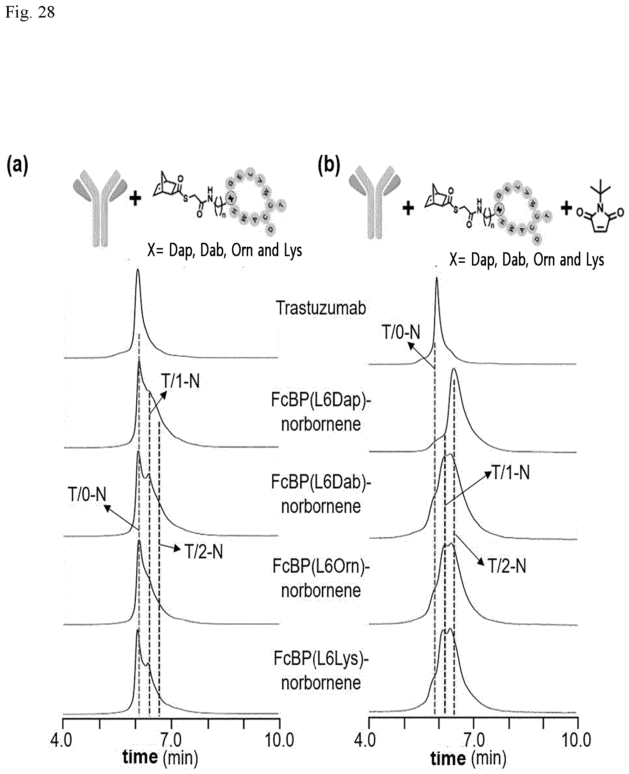

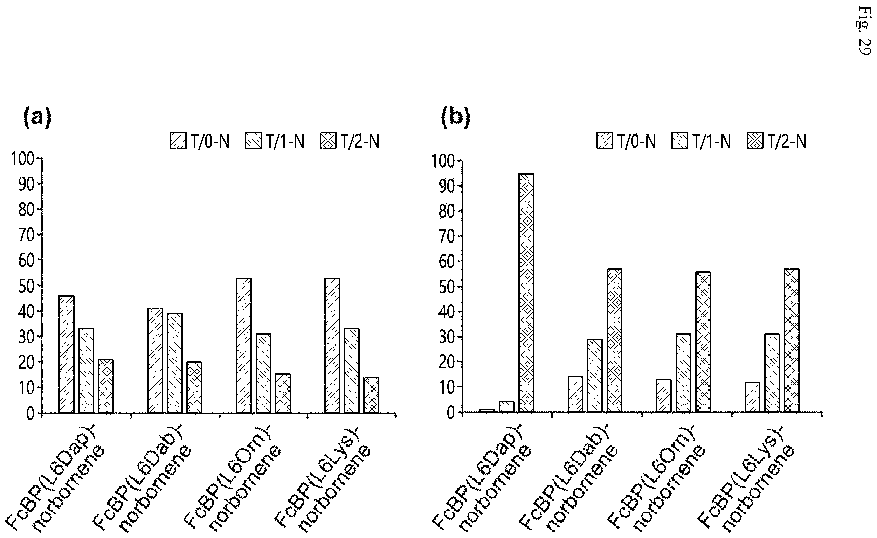

The crosslinking ratio depending on the presence or absence of the thiol-reactive additive (i.e., the preparation ratio of antibodies comprising functional group) was investigated. The results deduced from the HIC-HPLC analysis are shown in FIGS. 28 and 29. Tables 2 and 3 below are the summary of the results of the graphs shown in FIG. 29. FIGS. 28 and 29A represent the crosslinking monitoring result of each FcPB-norbornene in the absence of the thiol-reactive additive. FIGS. 28 and 29B represent the crosslinking monitoring result of each FcBP-norbornene in the presence of the thiol-reactive additive. In FIGS. 28 and 29 and Tables 2 and 3, T/1-N represents a trastuzumab/1-norbomene conjugate, i.e., a compound in which one norbornene is crosslinked to trastuzumab. T/2-N represents a trastuzumab/2-norbornene conjugate, i.e., a compound in which two norbornenes are crosslinked to trastuzumab (wherein norbornene is linked to K248 in the Fc region of each heavy chain) (refer to the documents [PCT International Application No. PCT/KR2020/003282; and Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759]).

**Table 2. Crosslinking ratio (HIC-HPLC analysis result) in absence of thiol-reactive additive**

| | T/0-N (5.9 min) | T/1-N (6.2 min) | T/2-N (6.5 min) |
|---|---|---|---|
| FcBP(L6Dap)-norbomene | 46% | 33% | 21% |
| FcBP(L6Dab)-norbornene | 41% | 39% | 20% |
| FcBP(L6Orn)-norbornene | 53% | 31% | 15% |
| FcBP(L6Lys)-norbomene | 53% | 33% | 14% |

**Table 3. Crosslinking ratio (HIC-HPLC analysis result) in presence of thiol-reactive additive**

| | T/0-N (5.9 min) | T/1-N (6.2 min) | T/2-N (6.5 min) |
|---|---|---|---|
| FcBP(L6Dap)-norbornene | 1% | 4% | 95% |
| FcBP(L6Dab)-norbornene | 14% | 29% | 57% |
| FcBP(L6Orn)-norbornene | 13% | 31% | 56% |
| FcBP(L6Lys)-norbomene | 12% | 31% | 57% |

### (4) Additional experiment for FcBP(L6Dap)-norbornene: Confirmation of degree of reaction over reaction time

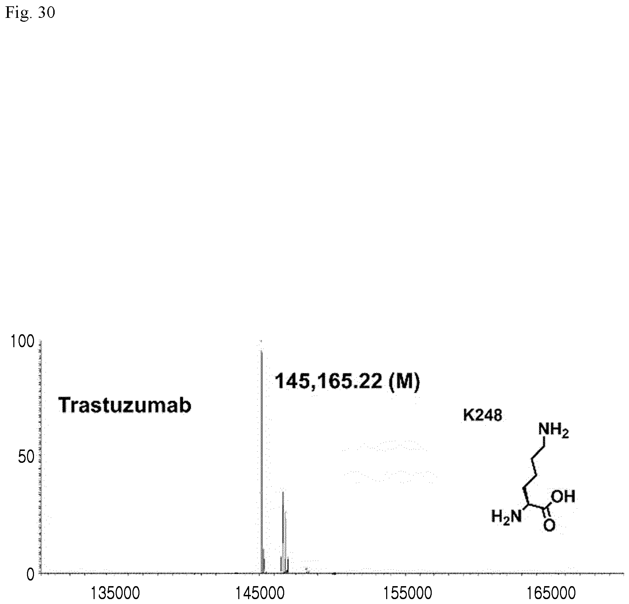

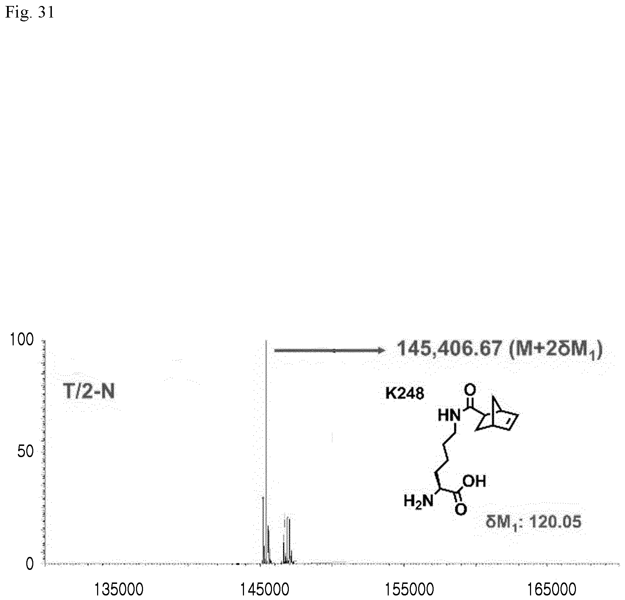

Trastuzumab (5.4 mg/mL, 10 mL) and 50 equiv. of N-tert-butylmaleimide were incubated in 1xPBS with pH 7.4 at 25 °C for 10 minutes (in a control, N-tert-butylmaleimide was not used). Afterward, 5 equiv. of FcBP (L6Dap)-norbornene was added and then the resulting mixture was incubated for 5 hours. To remove excess FcBP (L6Dap)-norbornene and N-tert-butylmaleimide, the reaction mixture was subjected to spin desalting and then further investigated. For biotinylation of unreacted trastuzumab, 1 equiv. of FcBP-biotin (bFcBP) 10 of trastuzumab was incubated together with the mixture in 1xPBS (pH 7.4) (6 hrs, 25 °C). To remove the biotinylated antibody, the antibody mixture was treated with streptavidin beads. After biotin-streptavidin pulldown of unreacted or partially reacted trastuzumab, highly pure T/2-N was obtained. The removal of an unreacted conjugate was monitored by HIC-HPLC, and the intact mass of the purified T/2-N was analyzed by LC-MS (refer to FIGS. 30 and 31) (refer to the document [Lee, TaeJin, et al., "Site-Selective Antibody-Drug Conjugation by a Proximity-Driven S to N Acyl Transfer Reaction on a Therapeutic Antibody," Journal of Medicinal Chemistry 65.7 (2022): 5751-5759]). In FIG. 31, T/2-N represents a trastuzumab/2-norbornene conjugate, i.e., a compound in which two norbornenes are crosslinked to trastuzumab.

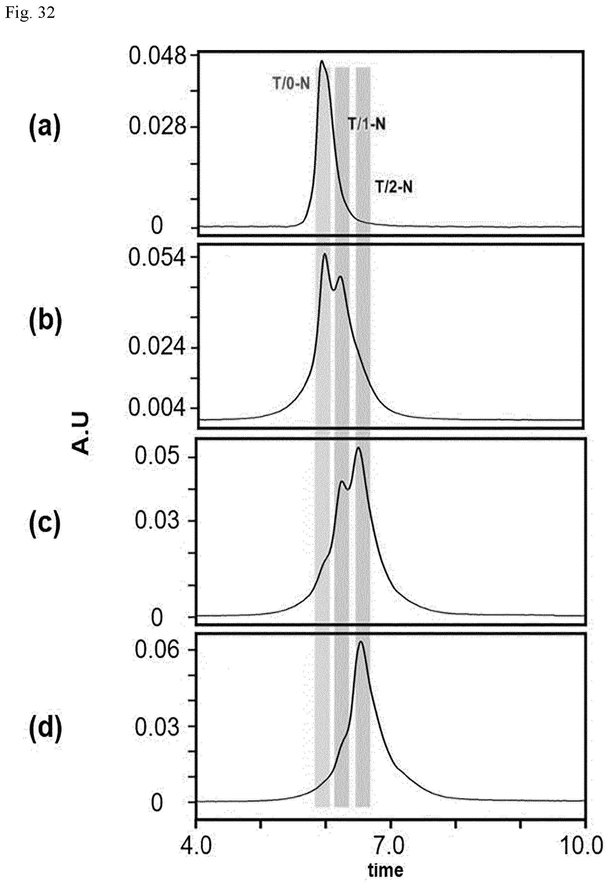

The reaction was monitored by HIC-HPLC. The HIC-HPLC monitoring result is shown in FIG. 31. Specifically, FIG. 32 shows the HIC-HPLC monitoring result over incubation time. FIG. 32A shows the peak of trastuzumab (5.9 min), FIG. 32B shows 2 h incubation, FIG. 32C shows 4 h incubation, and FIG. 32D shows 6 h incubation.

### 6. Confirmation of effect of thiol-reactive additive in preparation of antibody comprising functional group 3

### 6.1. Thiol-reactive additive used in this experiment

In "6. Confirmation of effect of thiol-reactive additive in preparation of antibody comprising functional group 3," an experiment was performed using the following thiol-reactive additives 1 to 12. The thiol-reactive additives 1 to 12 used in this experiment are shown in Table 4 below.

**Table 4. Thiol-reactive additives used in this experiment**

| | **Chemical name** | **Chemical structure** |
|---|---|---|
| Thiol reactive additive 1 | 5,5'-Dithiobis-(2-nitrobenzoic acid) (DTNB) | 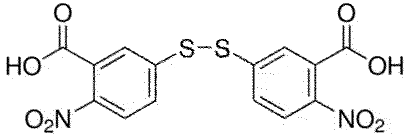 |
| Thiol reactive additive 2 | Diphenyl disulfide (phenyl disulfide) | 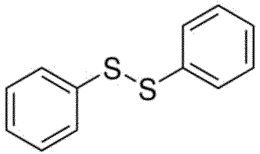 |
| Thiol reactive additive 3 | 4-Aminophenyl disulfide | 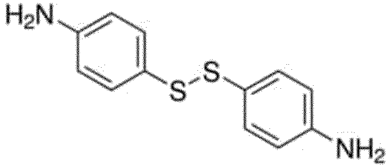 |
| Thiol reactive additive 4 | 2,2'-Dipyridyldisulfide (Aldrithiol^{™}-2) | 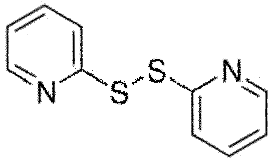 |
| Thiol reactive additive 5 | 4,4'-Dipyridyl disulfide (Aldrithiol^{™}-4) | 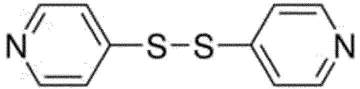 |
| Thiol reactive additive 6 | 2,2'-Dithiodibenzoic acid | 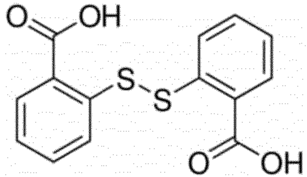 |
| Thiol reactive additive 7 | N-Tert-butylmaleimide | 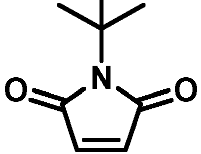 |
| Thiol reactive additive 8 | 2-Aminophenyl disulfide (2,2'-dithiodianiline) | 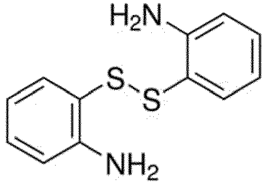 |
| Thiol reactive additive 9 | 4,4'-Dichlorodiphenyl disulfide | 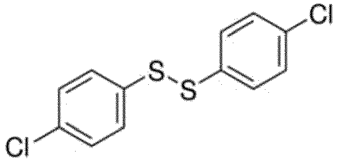 |
| Thiol reactive additive 10 | Bis(2-nitrophenyl) disulfide | 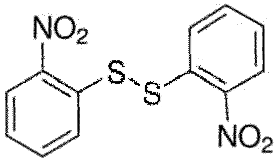 |
| Thiol reactive additive 11 | p-Tolyl disulfide (di-p-tolyl disulfide) | 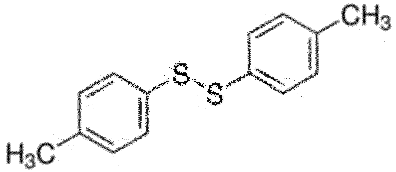 |
| Thiol reactive additive 12 | Bis(4-nitrophenyl) disulfide | 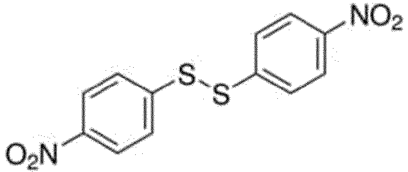 |

The following compounds were purchased from Sigma-Aldrich (St. Louis, MO, USA): 5,5'-dithiobis-(2-nitrobenzoic acid) (DTNB) (Cas No. 69-78-3) (Cat No. D218200); phenyl disulfide (Cas No. 882-33-7) (Cat No. 169021); 4-aminophenyl disulfide (Cas No. 722-27-0) (Cat No. 369462); Aldrithiol^{™}-2 (Cas No. 2127-03-9) (Cat No. 143049); Aldrithiol^{™}-4 (Cas No. 2645-22-9) (Cat No. 143057); and 2,2'-dithiodibenzoic acid (Cas No. 119-80-2) (Cat No. 43761).

The following compounds were purchased from Tokyo Chemical Industry (Tokyo, Japan): N-tert-butylmaleimide (Cas No. 4144-22-3) (Cat No. B3638); 2,2'-dithiodianiline (Cas No. 1141-88-4) (Cat No. D1246); 4,4'-dichlorodiphenyl disulfide (Cas No. 1142-19-4) (Cat No. D0360); bis(2-nitrophenyl) disulfide (Cas No. 1155-00-6) (Cat No. B0503); di-p-tolyl disulfide (Cas No. 103-19-5) (Cat No. T1074); and bis(4-nitrophenyl) disulfide (Cas No. 100-32-3) (Cat No. B0504).

### 6.2. Confirmation of effect of thiol-reactive additive in preparation of antibody-drug conjugate using FcBP-N₃ (Compound 7)

### (1) Preparation of control

### 1) Preparation of antibody comprising functional group (azide)

1 mg (1 mg/mL) of an antibody and 10 equiv. of FcBP-N₃ (Compound 7) based on the antibody were mixed in 1 x PBS (pH 7.4). After the reaction at room temperature for 6 to 7 hours, excess FcBP-N₃ (Compound 7) was removed using size-exclusion chromatography, spin desalting, and dialysis, thereby obtaining an antibody comprising a bio-orthogonal functional group (azide).

### 2) Preparation of antibody-drug (MMAE) conjugate

The payload (Compound 39) was prepared at a concentration of 10 mM in DMSO. 1 mg (1 mg/mL) of the bio-orthogonal functional group (azide)-linked antibody, which had been obtained by the dialysis technique, and the payload (Compound 39) were mixed in 1 x PBS (pH 7.4), and reacted for 1 hour. Here, 3 equiv. of the payload per reaction site was used. The reaction was observed using hydrophobic interaction chromatography. After the reaction, an excess payload was removed using size-exclusion chromatography, spin desalting, and a dialysis technique, thereby obtaining an antibody-drug (MMAE) conjugate.

### (2) Preparation of antibody-drug conjugate using thiol-reactive additive

### 1) Preparation of antibody comprising functional group (azide)

FcBP-N₃ (Compound 7) was prepared at a concentration of 10 mM in DMSO. Thiol-reactive additives 1 to 12 were prepared at a concentration of 100 mM in DMSO. 1 mg (1 mg/mL) of an antibody (anti-CLDN18.2 antibody, refer to the document [Korean Patent Application No. 10-2021-7023724, Publication No. 10-2021-0110339]) and 100 equiv. of each thiol-reactive additive (thiol-reactive additives 1 to 12) based on the antibody were mixed in 1 x PBS (pH 7.4). Based on a target site, 50 equiv. of the thiol-reactive additive was used. After mixing at room temperature for about 10 minutes, 10 equiv. of the compound for transferring a functional group (Compound 7, FcBP-N₃) based on the antibody was added and mixed for 6 to 7 hours. Based on a target site, 5 equiv. of FcBP-N₃ was used. After the reaction, excess FcBP-N₃ and thiol-reactive additive were removed using size-exclusion chromatography, spin desalting, and dialysis. The anti-CLDN18.2 antibody used in this experiment is an antibody that includes two light chains having the sequence of SEQ ID NO: 81 and two heavy chains having the sequence of SEQ ID NO: 82. The schematic diagram of the antibody comprising a functional group (azide) is shown in FIG. 14.

DIVMTQSPLSLPVTPGEPASISCKSSQTLLNSGNQKNYLTWYLQKPGQSPQLLIYWA
STGESGVPDRFSGSGSGTDFTLKISRVEAEDVGVYYCQNAYFYPFTFGGGTKVEIK
RTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC (SEQ
ID NO: 81)

QVQLVESGGGVVQPGRSLRLSCAASGFSLTTYGVHWVRQAPGKGLEWVAVIWAGG

STNYNSALMSRFTISRDNSKNTLYLQMNSLRAEDTAVYYCARAAYYGNGLDYWGQ
GTMVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKRVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK (SEQ
ID NO: 82)

### 2) Preparation of antibody-drug conjugate

The payload (Compound 39) was prepared at a concentration of 10 mM in DMSO. 1 mg (1 mg/mL) of the antibody comprising a bio-orthogonal functional group (azide), which had been obtained by dialysis, and the payload (Compound 39) were mixed in 1 x PBS (pH 7.4) and reacted for 1 day. Here, 3 equiv. of the payload was prepared based on the reaction site. The reaction was observed using hydrophobic interaction chromatography. After the reaction, an excess payload was removed using size-exclusion chromatography, spin desalting, and dialysis, thereby obtaining an antibody-drug (MMAE) conjugate.

### (3) Confirmation of effect in using thiol-reactive additive

### 1) Analysis method

An antibody-drug conjugate was prepared by the above-described method, and then analyzed using hydrophobic-interaction chromatography (HIC)-HPLC. For analysis, an HIC-butyl column with specifications of 4.6 x 100 mm and 5 µm (MAbPacTM, Thermo Fisher Scientific) was used.

### 2) Analysis result

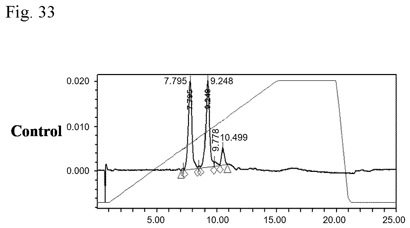

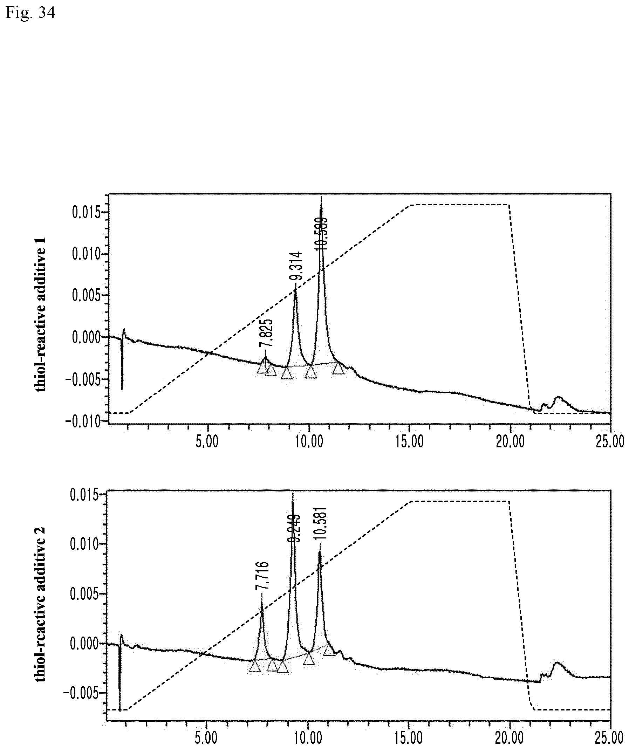

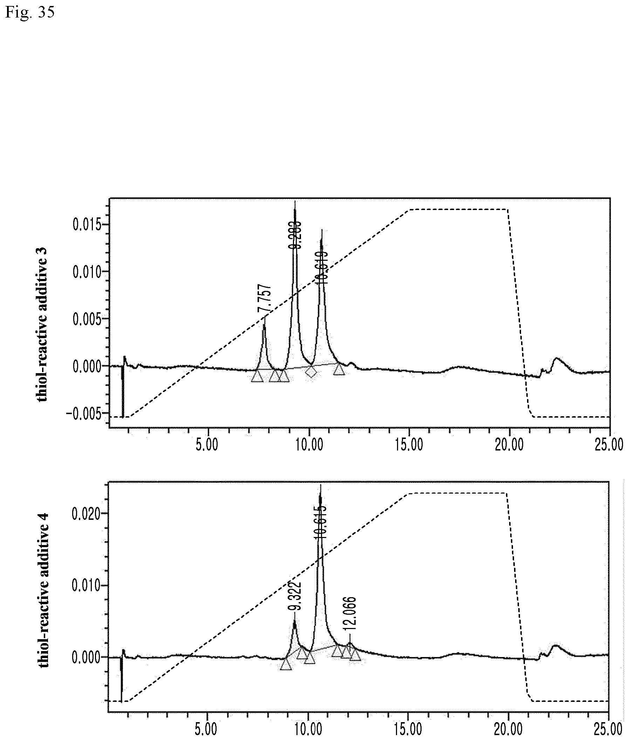

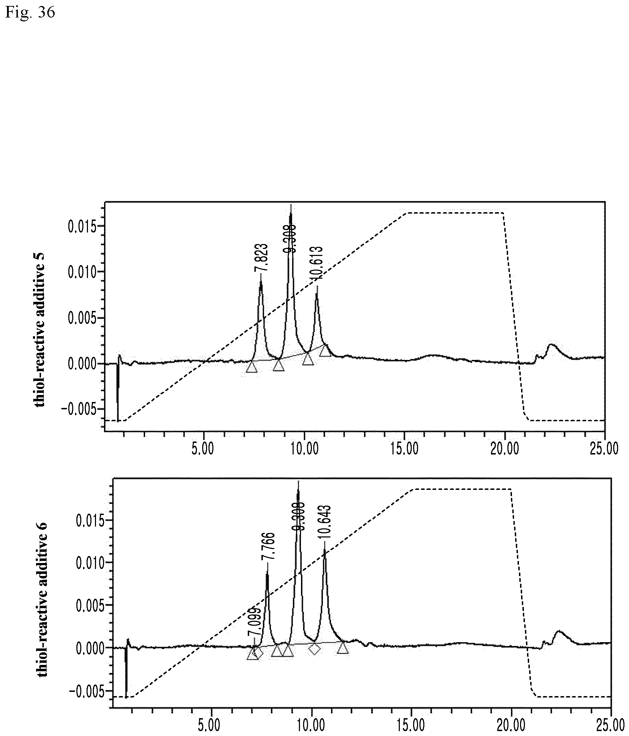

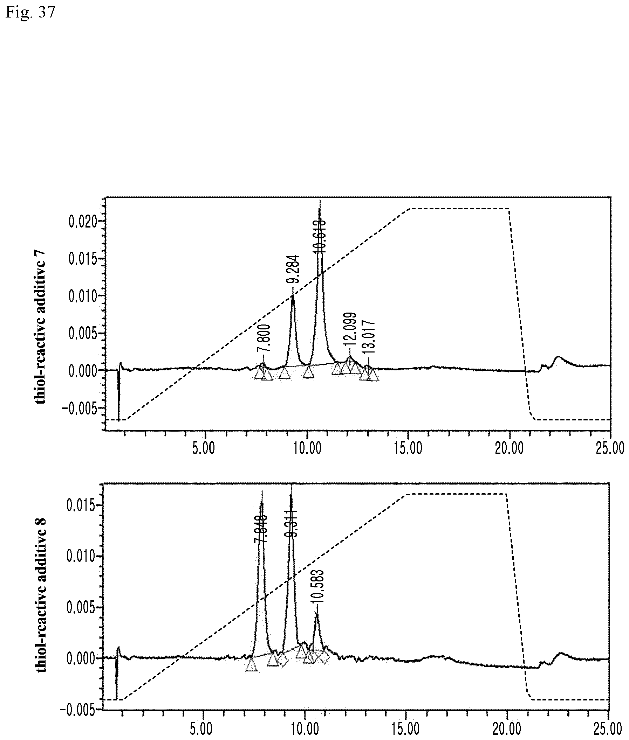

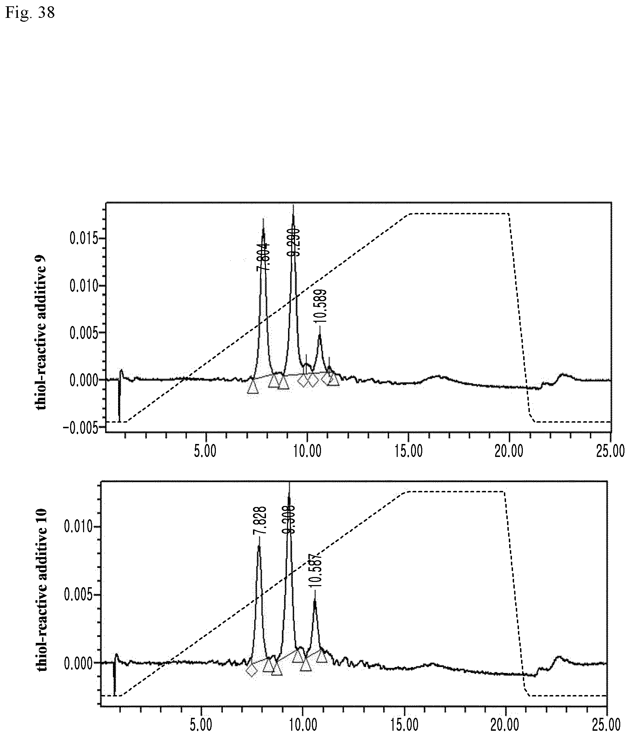

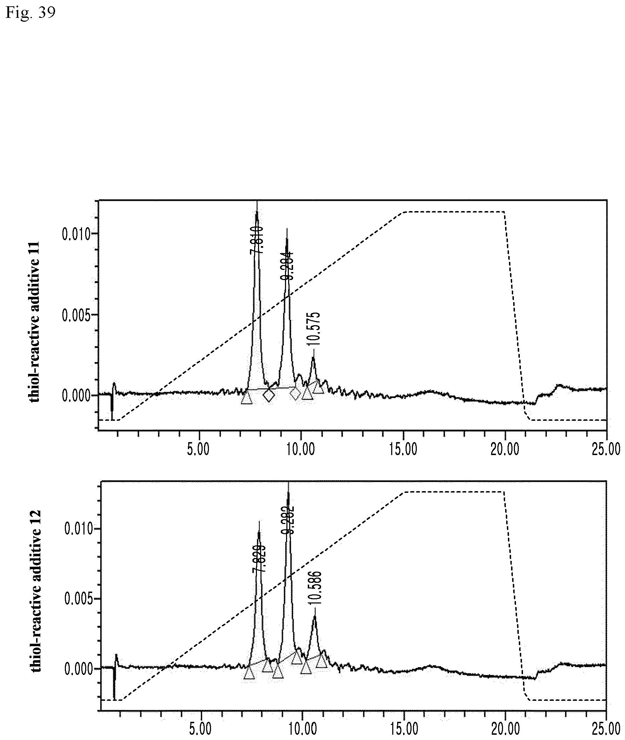

The yield of the antibody-drug conjugate depending on whether the thiol-reactive additive was used was analyzed. The HIC-HPLC analysis results using the use of thiol-reactive additives 1 to 12 are shown in FIGS. 33 to 39. The following table is a summary of the results of FIGS. 33 to 39. A control is the result obtained when a thiol-reactive additive is absent. The ratio of the antibody or the prepared antibody-drug conjugate was deduced from the peak area of the HIC-HPLC analysis graph.

**Table 5. Production yield of antibody-drug conjugate depending on whether thiol-reactive additive was used (FcBP-Ns)**

| | **DAR 0** | **DAR 1** | **DAR 2** |
|---|---|---|---|
| Control | 45.6% (7.795 min) | 44.0% (9.248 min) | 7.2% (10.499 min) |
| Thiol reactive additive 1 | 1.2% (7.825 min) | 30.4% (9.314 min) | 68.4% (10.589 min) |
| Thiol reactive additive 2 | 16.5% (7.716 min) | 52.9% (9.249 min) | 30.6% (10.581 min) |
| Thiol reactive additive 3 | 10.9% (7.757 min) | 46.1% (9.283 min) | 43.0% (10.619 min) |
| Thiol reactive additive 4 | 0% | 14.0% (9.322 min) | 84.3% (10.615 min) |
| Thiol reactive additive 5 | 29.9% (7.823 min) | 53.6% (9.308 min) | 16.5% (10.613 min) |
| Thiol reactive additive 6 | 17.8% (7.766 min) | 49.3% (9.308 min) | 32.6% (10.643 min) |
| Thiol reactive additive 7 | 0.9% (7.800 min) | 27.1% (9.284 min) | 69.6% (10.613 min) |
| Thiol reactive additive 8 | 47.3% (7.848 min) | 42.9% (9.311 min) | 9.0% (10.583 min) |
| Thiol reactive additive 9 | 42.2% (7.804 min) | 44.2% (9.290 min) | 9.8% (10.589 min) |
| Thiol reactive additive 10 | 36.3% (7.828 min) | 49.6% (9.308 min) | 14.1% (10.587 min) |
| Thiol reactive additive 11 | 52.7% (7.810 min) | 42.4% (9.284 min) | 4.8% (10.575 min) |
| Thiol reactive additive 12 | 39.6% (7.829 min) | 48.4% (9.282 min) | 12.0% (10.586 min) |

### 6.3. Confirmation of effect of thiol-reactive additive in preparation of antibody-drug conjugate using FcBP-C6PEG4 (Compound 17)

### (1) Preparation of control

### 1) Preparation of antibody comprising functional group (azide)

1 mg (1 mg/mL) of an antibody and 10 equiv. of FcBP-C6PEG4 (Compound 17) based on the antibody were mixed in 1xPBS (pH 7.4). After the reaction at room temperature for 6 to 7 hours, excess FcBP-C6PEG4 was removed by size-exclusion chromatography, spin desalting, and dialysis, thereby obtaining an antibody comprising a bio-orthogonal functional group (azide).

### 2) Preparation of antibody-drug conjugate

The payload (Compound 39) was prepared at a concentration of 10 mM in DMSO. 1 mg (1 mg/mL) of the antibody comprising a bio-orthogonal functional group (azide), which had been obtained by dialysis, and the payload (Compound 39) were mixed in 1 x PBS (pH 7.4) and reacted for 1 day. Here, 3.5 equiv. of the payload per reaction site was used. The reaction was observed using hydrophobic interaction chromatography. After the reaction, excess payload was removed using size-exclusion chromatography, spin desalting, and dialysis, thereby obtaining an antibody-drug (MMAE) conjugate.

### (2) Preparation of antibody-drug conjugate using thiol-reactive additive

### 1) Preparation of antibody comprising functional group (azide)

FcBP-C6PEG4 (Compound 17) was prepared at a concentration of 10 mM in DMSO. Thiol-reactive additives 1 to 12 were prepared at a concentration of 100 mM in DMSO. 1 mg (1 mg/mL) of an antibody (anti-CLDN18.2 antibody, refer to the document [Korean Patent Application No. 10-2021-7023724, Publication No. 10-2021-0110339]) and 100 equiv. of each thiol-reactive additive (thiol-reactive additives 1 to 12) based on the antibody were mixed in 1 x PBS (pH 7.4). Based on a target site, 50 equiv. of the thiol-reactive additive was used. After mixing at room temperature for about 10 minutes, 10 equiv. of the compound for transferring a functional group (Compound 7, FcBP-N₃) based on the antibody was added and mixed for 6 to 7 hours. Based on a target site, 5 equiv. of FcBP-N₃ was used. After the reaction, excess FcBP-N₃ and thiol-reactive additive were removed using size-exclusion chromatography, spin desalting, and dialysis. The anti-CLDN18.2 antibody used in this experiment is an antibody that includes two light chains comprising the sequence of SEQ ID NO: 81 and two heavy chains comprising the sequence of SEQ ID NO: 82. The schematic diagram of the antibody comprising a functional group (azide) is shown in FIG. 16.

### 2) Preparation of antibody-drug conjugate

The payload (Compound 39) was prepared at a concentration of 10 mM in DMSO. 1 mg (1 mg/mL) of the antibody comprising a bio-orthogonal functional group (azide), which had been obtained by dialysis, and the payload (Compound 39) were mixed in 1 x PBS (pH 7.4) and reacted for 1 day. Here, 3.5 equiv. of the payload was used based on a reaction site. The reaction was observed using hydrophobic interaction chromatography. After the reaction, excess payload was removed using size-exclusion chromatography, spin desalting, and dialysis, thereby obtaining an antibody-drug (MMAE) conjugate.

### (3) Confirmation of effect in using thiol-reactive additive

### 1) Analysis method

An antibody-drug conjugate was prepared by the above-described method, and then analyzed using hydrophobic-interaction chromatography (HIC)-HPLC. For analysis, an HIC-butyl column with specifications of 4.6 x 100 mm and 5 µm (MAbPacTM, Thermo Fisher Scientific) was used.

### 2) Analysis result

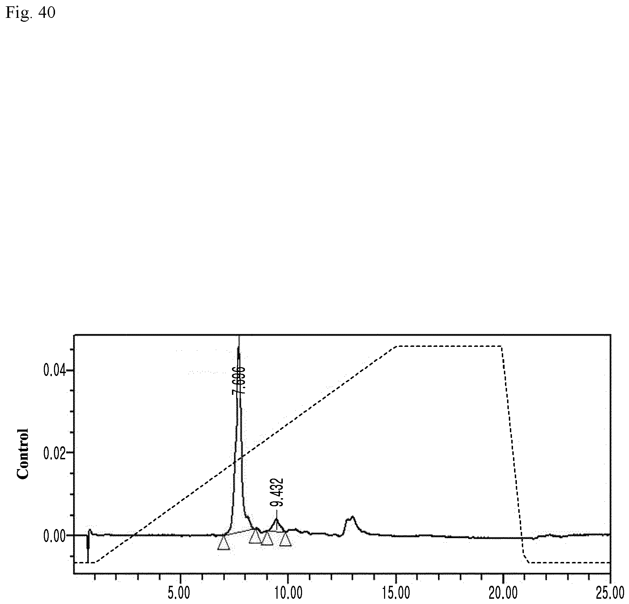

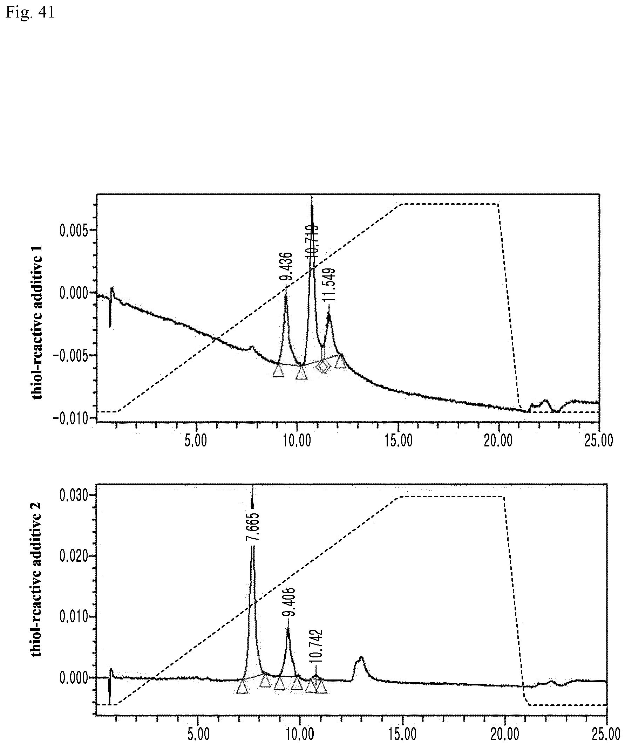

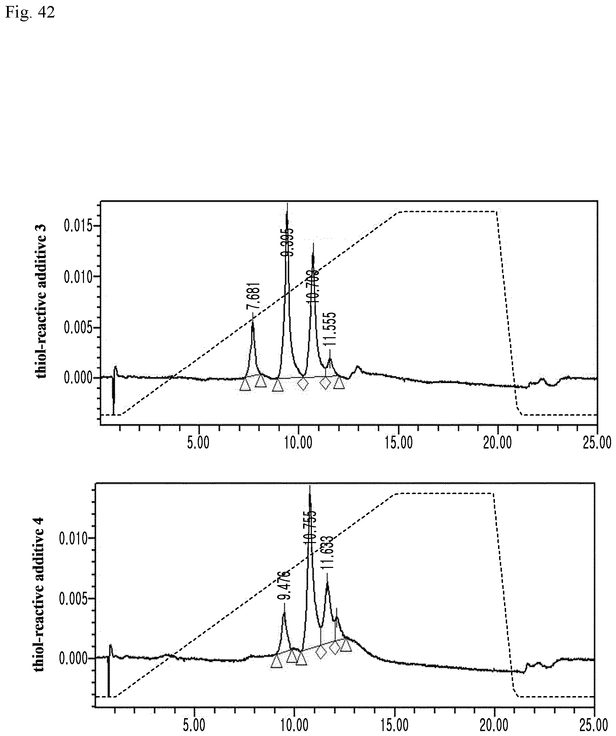

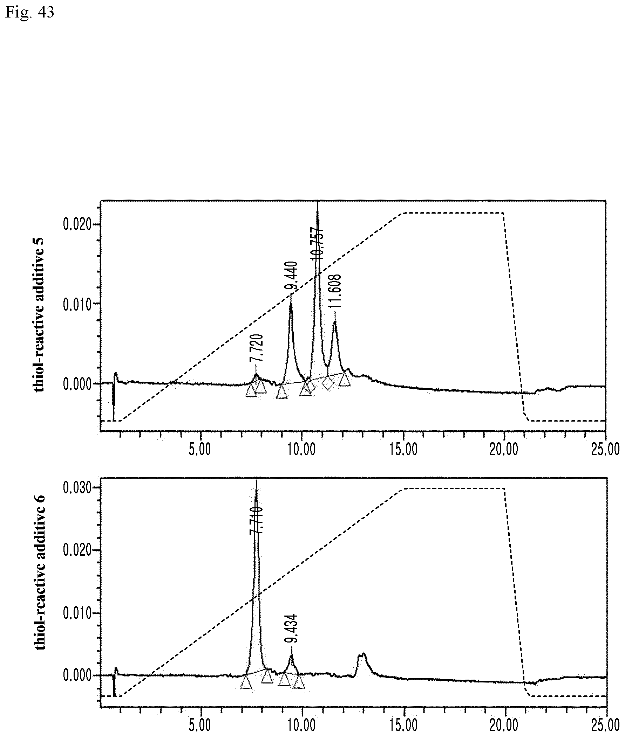

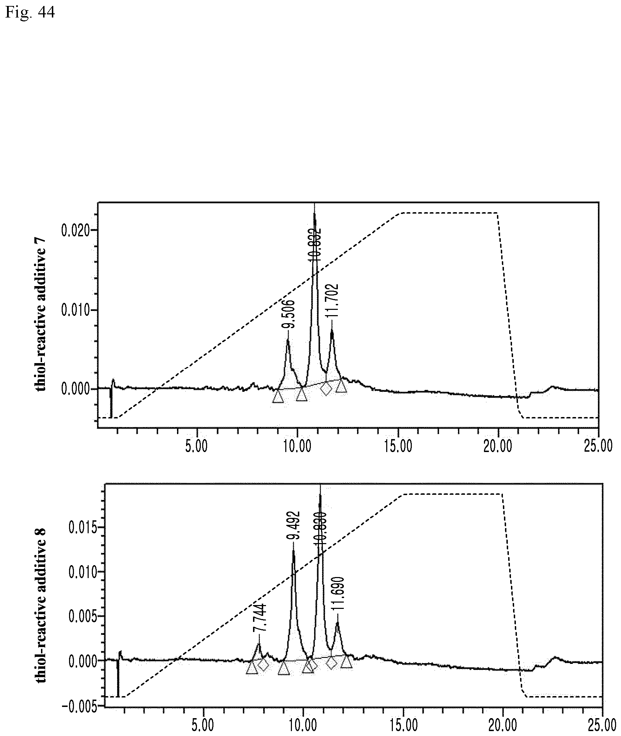

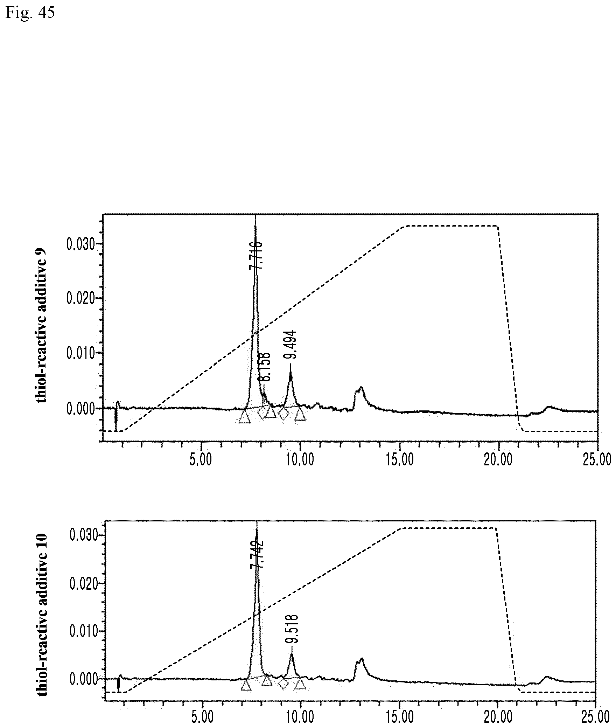

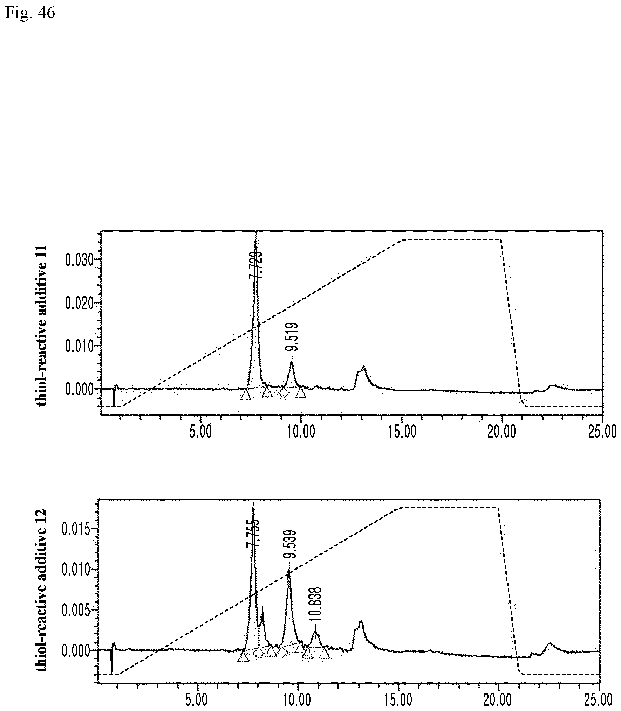

The yield of the antibody-drug conjugate depending on whether the thiol-reactive additive was used was analyzed. The HIC-HPLC analysis results using the use of thiol-reactive additives 1 to 12 are shown in FIGS. 40 to 46. The following table is a summary of the results of FIGS. 40 to 46. The control of Table 6 is the result obtained when a thiol-reactive additive is absent. The ratio of the antibody or the prepared antibody-drug conjugate was deduced from the peak area of the HIC-HPLC analysis graph.

**Table 6. Production yield of antibody-drug conjugate depending on whether thiol-reactive additive was used (FcBP-C6PEG4)**

| | **DAR 0** | **DAR 1** | **DAR 2** |
|---|---|---|---|
| Control | 92.2% (7.696 min) | 7.8% (9.432 min) | 0% |
| Thiol reactive additive 1 | 0% | 25.6% (9.436 min) | 53.7% (10.719 min) |
| Thiol reactive additive 2 | 75.3% (7.665 min) | 23.1% (9.408 min) | 1.6% (10.742 min) |
| Thiol reactive additive 3 | 12.6% (7.691 min) | 44.6% (9.395 min) | 37.5% (10.703 min) |
| Thiol reactive additive 4 | 0% | 12.1% (9.476 min) | 55.4% (10.755 min) |
| Thiol reactive additive 5 | 1.5% (7.720 min) | 27.4% (9.440 min) | 51.3% (10.757 min) |
| Thiol reactive additive 6 | 90.6% (7.710 min) | 9.4% (9.434 min) | 0% |
| Thiol reactive additive 7 | 0% | 20.7% (9.506 min) | 61.8% (10.832 min) |
| Thiol reactive additive 8 | 4.1% (7.744 min) | 36.1% (9.492 min) | 49.1% (10.830 min) |
| Thiol reactive additive 9 | 79.1% (7.716 min) | 17.1% (9.494 min) | 0% |
| Thiol reactive additive 10 | 83.5% (7.742 min) | 16.5% (9.518 min) | 0% |
| Thiol reactive additive 11 | 83.6% (7.729 min) | 16.4% (9.519 min) | 0% |
| Thiol reactive additive 12 | 48.7% (7.755 min) | 31.5% (9.539 min) | 8.0% (10.838 min) |

### 7. Confirmation of reactivity of maleimide-based compound among thiol-reactive additives with antibody

### 7.1. Experimental method

The inventors of the present application confirmed the reactivity of a maleimide-based compound with an antibody.

1 mg of the antibody (trastuzumab), and 50 equiv. of a thiol-reactive additive were mixed in 1xPBS (pH 7.4) and reacted. The reaction was performed at room temperature for 18 hours. After the reaction, the thiol-reactive additive that had not reacted with the antibody was removed using size-exclusion chromatography. The reaction between the antibody and the thiol-reactive additive was analyzed using hydrophobic interaction chromatography (HIC-HPLC) and MALDI-TOF mass spectrometry.

### 7.2. Results

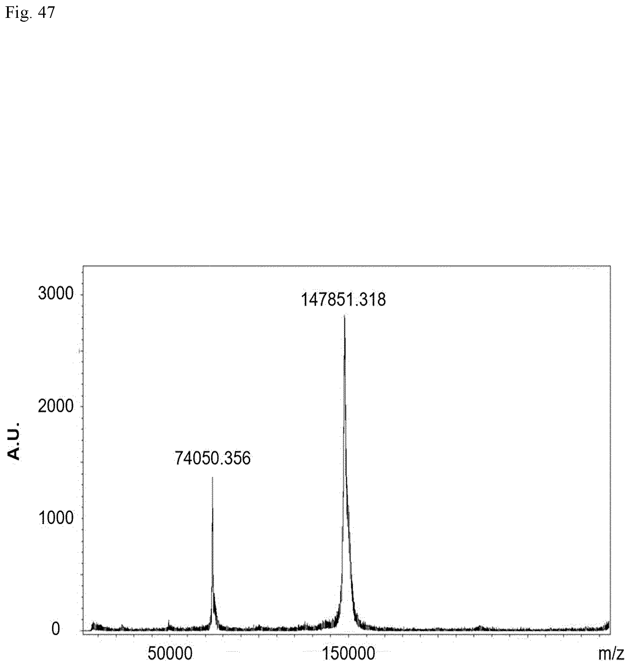

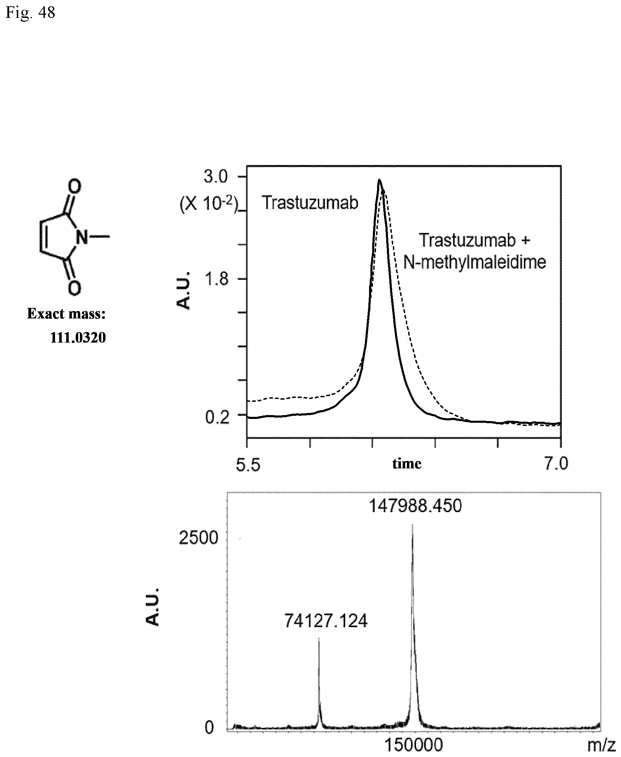

The experimental results are disclosed in FIGS. 48 to 54 and Table 7 below. As a result of mass spectrometry, when the molecular weight of the mixed sample increased after reacting an antibody with the molecular weight of a compound (thiol-reactive additive), it can be seen that the thiol-reactive additive reacted with the antibody. FIG. 47 is the mass spectrometry result of trastuzumab.

**Table 7. Summary of result of reaction of compound that can be used as thiol-reactive additive with antibody**

| **Thiol reactive additive** | **Chemical structure** | **Molecular weight of thiol reactive additive** | **Observed mass of mixed sample** | **Increased mass** | **Number of reactions with Lys** |
|---|---|---|---|---|---|
| N-Methylmaleimide | 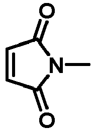 | 111 Da | 147,988 Da | + 137 Da | 1 |
| N-Ethylmaleimide | 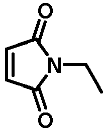 | 125 Da | 147,981 Da | + 130 Da | 1 |
| N-Phenylmaleimide | 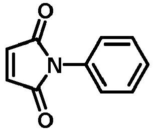 | 173 Da | 148,031 Da | + 180 Da | 1 |
| N-Benzylmaleimide | 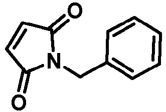 | 187 Da | 148,034 Da | + 183 Da | 1 |
| N-Cyclohexylmaleimide | 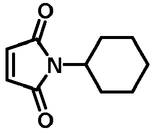 | 179 Da | 148,010 Da | + 159 Da | 1 |
| 3 -Maleimidopropionic acid | 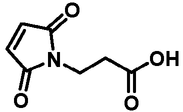 | 169 Da | 148,043 Da | + 192 Da | 1 |
| N-tert-butylmaleimide | 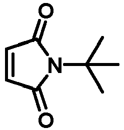 | 153 Da | 147,856 Da | + 5 Da | 0 |

As a result of reacting N-methylmaleimide with an antibody, it can be seen that one molecule of N-methylmaleimide reacted with the antibody. As shown in FIG. 48, when the antibody and N-methylmaleimide were reacted, it can be confirmed that the HIC-HPLC peak of the mixed sample of N-methylmaleimide and the antibody shifted. As shown in FIG. 48, when the antibody and N-methylmaleimide were reacted, it was observed by mass spectrometry that the mass of the mixed sample of N-methylmaleimide and the antibody increased.

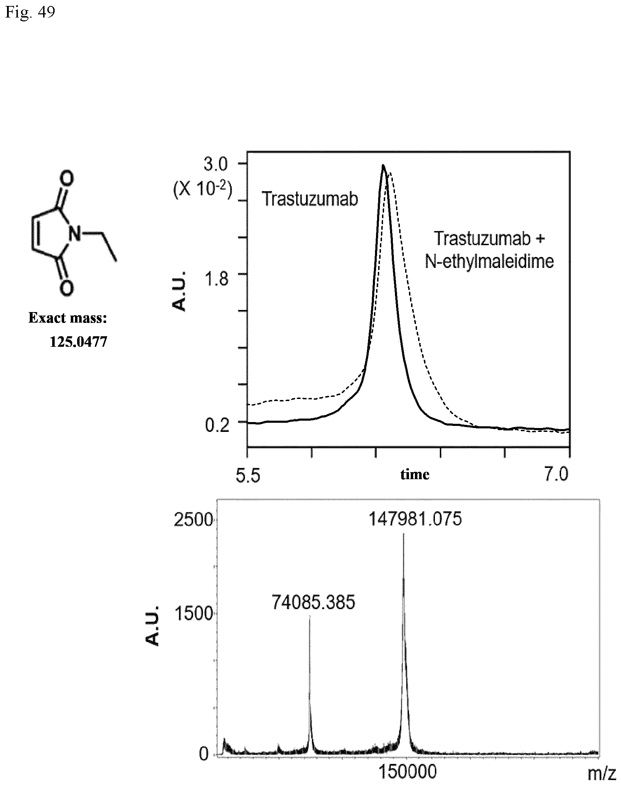

As a result of reacting N-ethylmaleimide with the antibody, it can be seen that one molecule of N-ethylmaleimide reacted with the antibody. As shown in FIG. 49, when the antibody reacted with N-ethylmaleimide, it can be confirmed that the HIC-HPLC peak of the mixed sample of N-ethylmaleimide and the antibody shifted. As shown in FIG. 49, when the antibody reacted with N-ethylmaleimide, it was observed by mass spectrometry that the mass of the mixed sample of N-ethylmaleimide and the antibody increased.

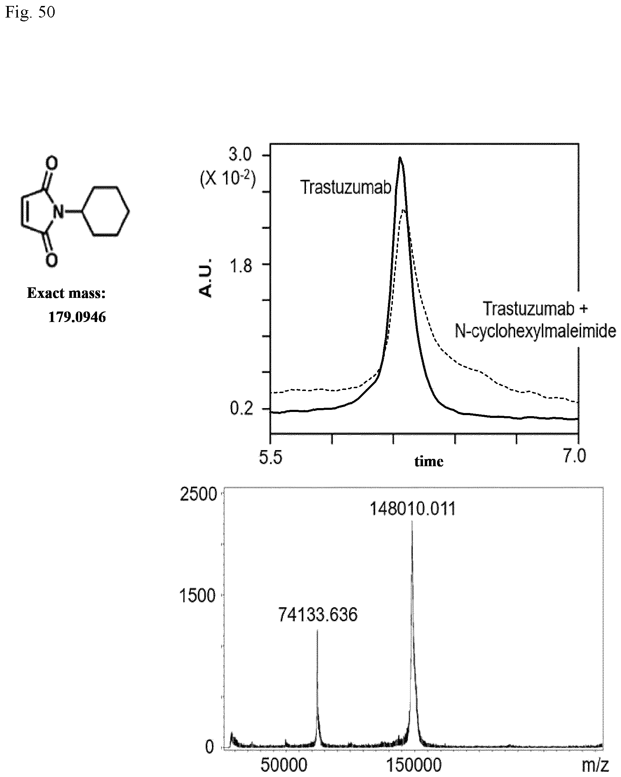

As a result of reacting N-cyclohexylmaleimide with the antibody, it can be seen that one molecule of N-cyclohexylmaleimide reacted with the antibody. As shown in FIG. 50, when the antibody reacted with N-cyclohexylmaleimide, it can be confirmed that the HIC-HPLC peak of the mixed sample of N-methylmaleimide and the antibody shifted. As shown in FIG. 50, when the antibody reacted with N-cyclohexylmaleimide, it was observed by mass spectrometry that the mass of the mixed sample of N-cyclohexylmaleimide and the antibody increased.

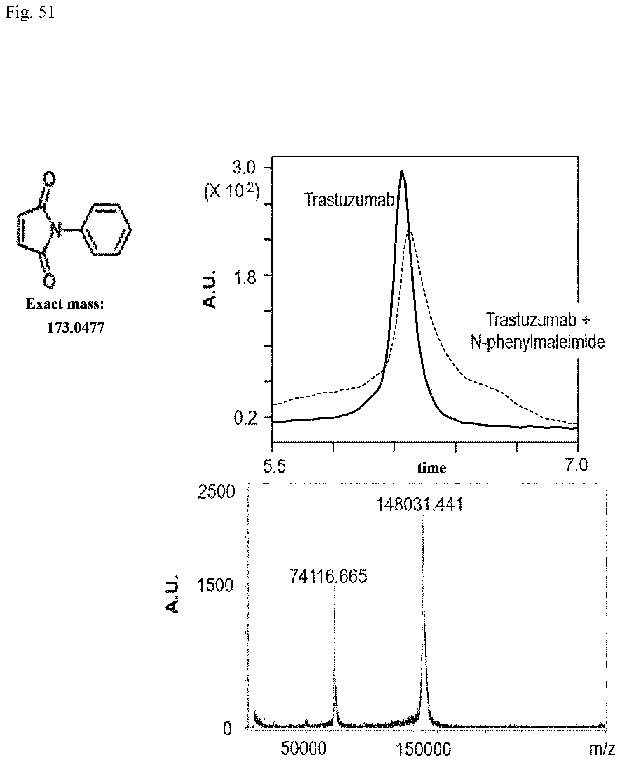

As a result of reacting N-phenylmaleimide with the antibody, it can be seen that one molecule of N-phenylmaleimide reacted with the antibody. As shown in FIG. 51, when the antibody and N-phenylmaleimide were reacted, it can be confirmed that the HIC-HPLC peak of the mixed sample of N-phenylmaleimide and the antibody shifted. As shown in FIG. 51, when the antibody and N-phenylmaleimide were reacted, it was observed by mass spectrometry that the mass of the mixed sample of N-phenylmaleimide and the antibody increased.

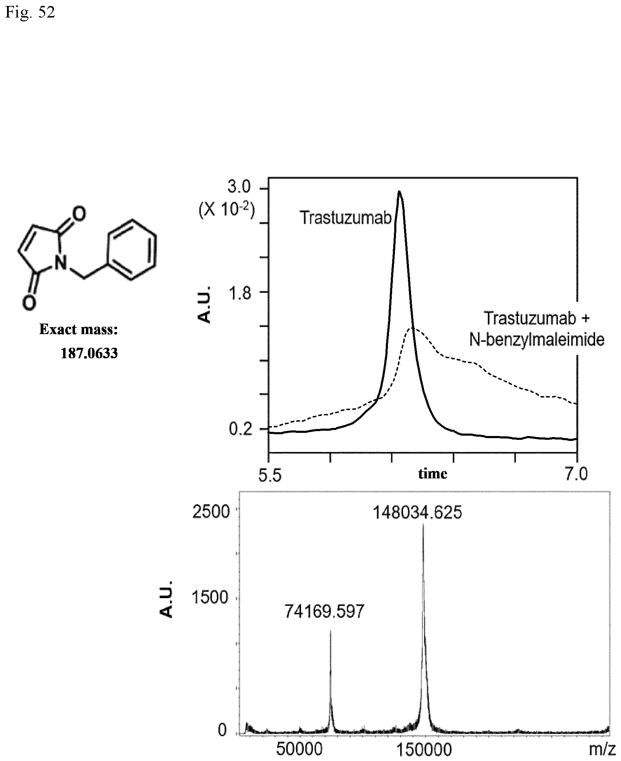

As a result of reacting N-benzylmaleimide with the antibody, it can be seen that one molecule of N-benzylmaleimide reacted with the antibody. As shown in FIG. 52, when the antibody and N-benzylmaleimide were reacted, it can be confirmed that the HIC-HPLC peak of the mixed sample of N-benzylmaleimide and the antibody shifted. As shown in FIG. 52, when the antibody and N-benzylmaleimide were reacted, it was observed by mass spectrometry that the mass of the mixed sample of N-benzylmaleimide and the antibody increased.

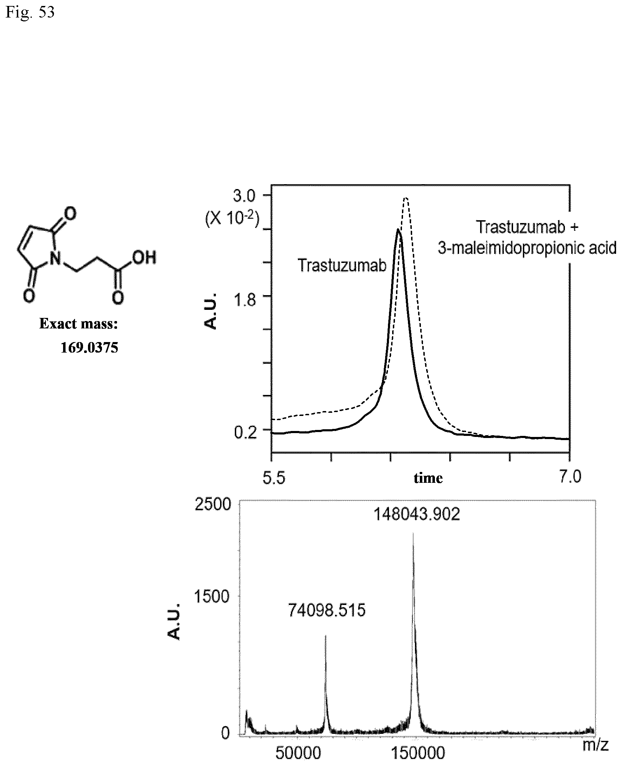

As a result of reacting 3-maleimidopropionic acid with the antibody, it can be seen that one molecule of 3-maleimidopropionic acid reacted with the antibody. As shown in FIG. 53, when the antibody and 3-maleimidopropionic acid were reacted, it can be confirmed that the HIC-HPLC peak of the mixed sample of 3-maleimidopropionic acid and the antibody shifted. As shown in FIG. 53, when the antibody and 3-maleimidopropionic acid were reacted, it was observed by mass spectrometry that the mass of the mixed sample of 3-maleimidopropionic acid and the antibody increased.

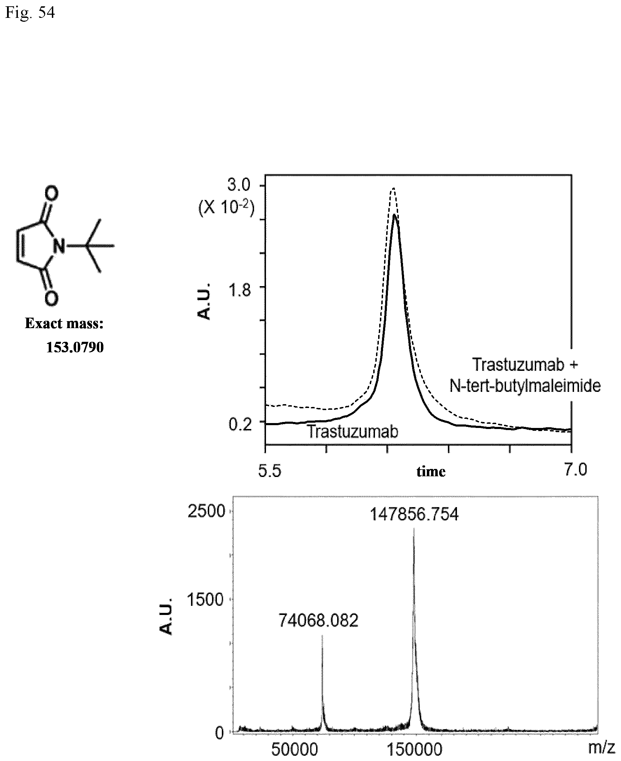

As a result of reacting N-tert-butyl-maleimide with the antibody, it can be seen that N-tert-butylmaleimide did not react with the antibody. As shown in FIG. 54, when the antibody and N-tert-butylmaleimide were reacted, it can be confirmed that the HIC-HPLC peak of the mixed sample of N-tert-butylmaleimide and the antibody did not shift. As shown in FIG. 54, when the antibody and N-tert-butylmaleimide were reacted, it was observed by mass spectrometry that the mass of the mixed sample did not increase.

## Claims

1. A method for increasing yield of an antibody comprising a functional group, which is produced by reaction between an antibody and a compound for transferring the functional group, by using a thiol reactive additive, the method comprising:
(a) contacting the antibody and the compound for transferring the functional group in reaction composition,
wherein the compound for transferring the functional group comprises a thioester group, a functional group (FG), and a Fc-binding peptide (FcBP),
wherein the thioester group of the compound for transferring the functional group reacts with a nucleophile of the antibody,
as a result of the reaction between the thioester and the nucleophile of the antibody, the antibody comprising the functional group, and a by-product comprising a thiol group and Fc-binding peptide derived from the compound for transferring the functional group are produced, wherein the thiol group of the by-product is produced by reaction between the thioester group of the compound for transferring the functional group and the nucleophile of the antibody,
wherein the by-product has a reaction inhibition effect, which inhibits a reaction between antibody that has not yet reacted in the reaction composition and compound for transferring the functional group that has not yet reacted in the reaction composition; and
(b) contacting the by-product with the thiol reactive additive, for removing the reaction inhibition effect of the by-product,
wherein the thiol reactive additive has reactivity with a thiol group,
wherein the thiol reactive additive reacts with the thiol group of the by-product,
as a result of the reaction between the thiol reactive additive and the thiol group, the thiol group of the by-product is capped.

2. The method of claim 1,
the reactive inhibition effect of the by-product is removed by capping the thiol group of the by-product.

3. The method of claim 1,
the thiol reactive additive is a compound that has little or no reactivity with antibody.

4. The method of claim 1,
the thiol reactive additive is a small molecule compound.

5. The method of claim 1,
molecular weight of the thiol reactive additive is 50 g/mol or more and 1000 g/mol or less.

6. The method of claim 1,
the thiol reactive additive is a compound that undergoes a thiol-disulfide exchange reaction.

7. The method of claim 1,
the thiol reactive additive comprises a disulfide group.

8. The method of claim 1,
the thiol reactive additive is a diphenyl disulfide or its derivative.

9. The method of claim 1,
the thiol reactive additive is a diphenyl disulfide, dipyridyldisulfide, 5,5'-dithiobis-(2-nitrobenzoic acid) (DTNB), 2,2'-Dithiodibenzoic acid, 4-Aminophenyl disulfide, 2-Aminophenyl disulfide, Bis(4-chlorophenyl) disulfide, Bis(2-nitrophenyl) disulfide), p-tolyl disulfide, Bis(4-nitrophenyl) disulfide, Bis(2-nitrophenyl) disulfide, Bis(4-methoxyphenyl) disulfide, or Bis(2-methoxyphenyl) disulfide, or one of derivatives thereof.

10. The method of claim 1,
the thiol reactive additive is a 5,5'-dithiobis-(2-nitrobenzoic acid) (DTNB) or 2,2'-Dipyridyldisulfide.

11. The method of claim 1,
the thiol reactive additive is a compound having structure of Formula 06: 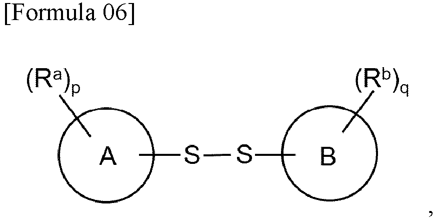 wherein,
A is 5-membered or 6-memberd aromatic ring, wherein A optionally comprises one or more heteroatom in the ring, wherein each heteroatom is independently selected from N, O, and S,
p is an integer of 0 to 4,
each Ra is independently selected from -R, -NO2, -CR3, -NR2, -OR, -SR, -SO2R, - OSO2R, -PO2R2, -OPOR2, -C(=O)R, -C(=O)CR3, -C(=O)OR, and -C(=O)NR2,
B is 5-membered or 6-memberd aromatic ring, wherein B optionally comprises one or more heteroatom in the ring, wherein each heteroatom is independently selected from N, O, and S,
q is an integer of 0 to 4,
each Rb is independently selected from -R, -NO2, -CR3, -NR2, -OR, -SR, -SO2R, - OSO2R, -PO2R2, -OPOR2, -C(=O)R, -C(=O)CR3, -C(=O)OR, and -C(=O)NR2,
each R is independently selected from H, halogen, C1-6 alkyl, C3-10 cycloalkyl, C3-10 heterocycloalkyl, arly, heteroaryl, -OH, =O, =S, and -NH2.

12. The method of claim 11,
at least one of Ra is an electron withdrawing group or hydrophilic group.

13. The method of claim 11,
at least one of Rb is an electron withdrawing group or hydrophilic group.

14. The method of claim 1,
the thiol reactive additive is a maleimide or derivative thereof.

15. The method of claim 1,
the thiol reactive additive is maleimide, N-Methylmaleimide, N-Ethylmaleimide, N-Phenylmaleimide, N-Benzylmaleimide, 3-Maleimidopropionic acid, N-tert-butylmaleimide, or N-Cyclohexylmaleimide, or derivatives thereof.

16. The method of claim 1,
the thiol reactive additive is tert-butylmaleimide.

17. The method of claim 1,
the thiol reactive additive is a compound having structure of Formula 09: 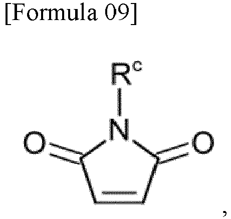
wherein,Rc is selected from -R, =O, =S, -NO2, -CR3, -CH2CR3, -NR2, -OR, -SR, - SO2R, -OSO2R, -PO2R2, -OPOR2, -C(=O)R, -C(=O)CR3, -C(=O)OR, and - C(=O)NR2,wherein each R is independently selected from H, halogen, C1-6 alkyl, C3-10 cycloalkyl, C3-10 heterocycloalkyl, arly, heteroaryl, -OH, =O, =S, and -NH2.

18. The method of claim 1,
the thiol reactive additive is a compound having structure of any one of Formulas 10 to 31.

19. The method of claim 1,
the compound for transferring the functional group has structure of Formula 01:
[Formula 01] FU-RU-PU,
wherein,
FU is a functional unit comprising the functional group (FG),
RU is a reactive unit comprising the thioester group,
PU is a peptide unit comprising the Fc-binding peptide (FcBP).

20. The method of claim 1,
the Fc-binding peptide has length of 8 to 20 amino acid residues.

21. The method of claim 1,
the Fc-binding peptide comprises any one amino acid sequence selected from SEQ ID NOs:2 to 80, or amino acid sequences having at least 80% sequence identity thereto.

22. The method of claim 1,
the functional group comprises one or more bio-orthogonal functional groups.

23. The method of claim 22,
each bio-orthogonal functional group is independently a group that participates in any one of the bio-orthogonal reaction selected from Staudinger Ligation, Copper-Catalyzed Azide-Alkyne Cycloaddition (CuAAC), Copper-Free Azide-Alkyne Cycloaddition, Tetrazine Ligation, Tetrazole Ligation, Oxime Ligation, and Isocyanide Click Reaction.

24. The method of claim 22,
each bio-orthogonal functional group is independently any one selected from azide, terminal alkyne, cycloalkyne, tetrazine, norbornene, cycloalkene, tetrazole, oxime, and isocyanide groups.

25. The method of claim 1,
the functional group comprises one or more active moieties.

26. The method of claim 25,
each active moiety is independently selected from drug, imaging moiety, ligand for radioactive isotope, affinity substance, stabilizing substance, vitamin, and hydrophilic moiety.

27. The method of claim 1,
the reaction inhibition effect of the by-product occurs because the Fc-binding peptide comprised in the by-product guides the by-product to a reactive site on the antibody, where the reaction between the compound for transferring the functional group and the antibody is occurred, such that the thiol group of the by-product is interacted with the antibody at the reactive site.

28. The method of claim 2,
the yield of the antibody comprising the functional group in the method is 1.5 times higher or more compared to when no thiol reactive additive is used.

29. The method of claim 28,
the yield of the antibody comprising the functional group is measured based on antibody comprising two functional groups.

30. The method of claim 1,
wherein, in the method, (a) contact between the antibody and the compound for transferring the functional group, and (b) contact between the by-product and the thiol reactive additive are carried out by mixing the thiol reactive additive, the antibody, and the compound for transferring the functional group.

31. The method of claim 30,
wherein, for the mixing the thiol reactive additive, the antibody, and the compound for transferring the functional group,
firstly, the thiol reactive additive is mixed with the antibody,
and then the compound for transferring the functional group is mixed with them.

32. The method of claim 30,
wherein, for the mixing the thiol reactive additive, the antibody, and the compound for transferring the functional group,
firstly, the thiol reactive additive is mixed with the compound for transferring the functional group,
and then the antibody is mixed with them.

33. A kit for increasing yield of an antibody comprising a functional group, wherein the kit comprises thiol reactive additive, antibody, and compound for transferring the functional group.

34. A composition for increasing yield of an antibody comprising a functional group, wherein the composition comprises thiol reactive additive, antibody, and compound for transferring the functional group.
